(19) Europäisches Patentamt
European Patent Office
Office européen des brevets

(11) EP 4 520 829 A1

(12) EUROPEAN PATENT APPLICATION
published in accordance with Art. 153(4) EPC

(43) Date of publication:
**12.03.2025 Bulletin 2025/11**

(21) Application number: **23803630.5**

(22) Date of filing: **12.05.2023**

(51) International Patent Classification (IPC):
***C12N 15/53*** (2006.01) ***C07C 227/28*** (2006.01)
***C07C 229/12*** (2006.01) ***C07K 1/00*** (2006.01)
***C12N 9/06*** (2006.01) ***C12N 15/31*** (2006.01)
***C12P 13/04*** (2006.01)

(52) Cooperative Patent Classification (CPC):
**C07C 227/28; C07C 229/12; C07K 1/00; C07K 14/195; C12N 9/0004; C12P 13/04**

(86) International application number:
**PCT/JP2023/017861**

(87) International publication number:
**WO 2023/219156 (16.11.2023 Gazette 2023/46)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority:
**13.05.2022 JP 2022079711**
**13.05.2022 JP 2022079712**
**23.12.2022 PCT/JP2022/047741**
**23.12.2022 EP 22216504**
**23.12.2022 TW 111149813**

(71) Applicant: **CHUGAI SEIYAKU KABUSHIKI KAISHA**
**Tokyo 115-8543 (JP)**

(72) Inventors:
- **ISHIYAMA Shiori**
  **Yokohama City, Kanagawa 244-8602 (JP)**
- **NAKANO Kazuhiko**
  **Singapore, 138623 (SG)**
- **SHINODA Kiyomichi**
  **Yokohama City, Kanagawa 244-8602 (JP)**

(74) Representative: **Vossius & Partner Patentanwälte Rechtsanwälte mbB**
**Siebertstrasse 3**
**81675 München (DE)**

Remarks:
The complete document including Reference Table(s) and the Sequence Listing(s) can be downloaded from the EPO website

# (54) METHOD FOR PRODUCING AMINO ACID AND PEPTIDE COMPOUND, USING ENZYME AND ADDITIVE

(57) One aspect of the present invention relates to a method for producing an amino acid, comprising a step of performing the following reaction (i) or (ii) in the presence of a polypeptide comprising an amino acid sequence having 90% or higher identity to an amino acid sequence represented by SEQ ID NO: 1, a reducing agent, and compound D represented by the following formula (1):
(i) an intermolecular reductive amination reaction between compound A selected from the group consisting of a compound having an amino group and a salt thereof and compound B selected from the group consisting of a compound having a carbonyl group and a salt thereof;
(ii) an intramolecular reductive amination reaction of compound C selected from the group consisting of a compound having an amino group and a carbonyl group and a salt thereof.

[Formula 1]

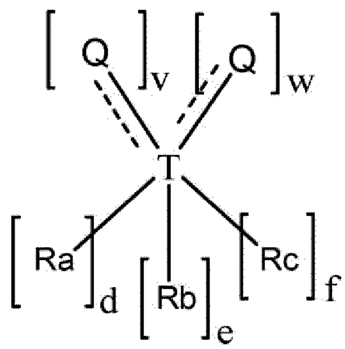

(1)

EP 4 520 829 A1

## Description

[Technical Field]

The present invention relates to a method for producing an amino acid and a peptide compound using an enzyme and an additive.

[Background Art]

Until now, methods for synthesizing amino acids from amines and ketoacids using an enzyme that advances the reductive amination reaction (hereinafter, the enzyme is referred to as a reductive amination enzyme) have been known. For example, Patent Literatures 1 to 4 disclose techniques for synthesizing various amino acids with reductive amination enzymes.

[Citation List]

[Patent Literature]

[Patent Literature 1] Japanese Patent Laid-Open No. 2003-319788
[Patent Literature 2] Japanese Patent Laid-Open No. 2005-095167
[Patent Literature 3] Japanese Patent Laid-Open No. 2012-080878
[Patent Literature 4] Japanese Patent Laid-Open No. 2012-080879

[Summary of Invention]

[Problems to be Solved by the Invention]

However, the reaction efficiency of the reaction to obtain amino acids with the reductive amination enzyme has been not always sufficient, and the improvement of the efficiency has been desired.
An object of the present invention is to provide a method for efficiently producing an amino acid.

[Means for Solving the Problems]

The present invention relates to, for example, each of the following aspects.

[1] A method for producing an amino acid, comprising a step of performing the following reaction (i) or (ii) in the presence of a polypeptide comprising an amino acid sequence having 90% or higher identity to an amino acid sequence represented by SEQ ID NO: 1, a reducing agent, and compound D represented by the following formula (1):

(i) an intermolecular reductive amination reaction between compound A selected from the group consisting of a compound having an amino group and a salt thereof and compound B selected from the group consisting of a compound having a carbonyl group and a salt thereof;
(ii) an intramolecular reductive amination reaction of compound C selected from the group consisting of a compound having an amino group and a carbonyl group and a salt thereof:

[Formula 1]

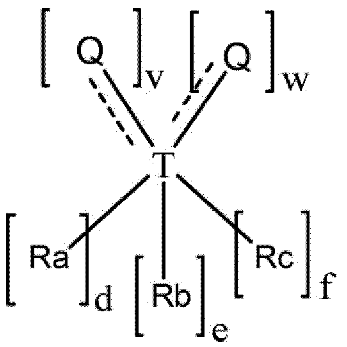

(1)

wherein in the formula (1),

v and w each independently represent 0 or 1,
at least one of v and w represents 1,
T represents a carbon atom, a phosphorus atom, or a sulfur atom,
a functional group represented by the following formula (1a):

[Formula 2]

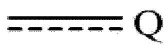

(1 a)

represents =O, -ORd, or a hydroxy group,
when each of v and w is 1, two functional groups represented by a plurality of formulas (1a) are the same or different,
Ra, Rb, and Rc each independently represent a hydrogen atom, a $C_1$ to $C_3$ alkyl group, an alkylamino group, or -$CH_2$-ORd,
at least any two of Ra, Rb, and Rc are optionally linked to each other to form a ring structure together with T,
Rd represents a $C_1$ to $C_3$ alkyl group,
d, e, and f each independently represent 0 or 1,
at least one of d, e, and f represents 1,
when each of v and w is 1, at least one of Ra, Rb, and Rc is a methyl group, and none of Ra, Rb, and Rc are linked to each other to form a ring structure together with T,
when at least one of Ra, Rb, and Rc is a methylamino group, none of Ra, Rb, and Rc are linked to each other to form a ring structure together with T, and
when the functional group represented by the formula (1a) is a hydroxy group and T is a carbon atom, v is 1, w is 0, each of d, e, and f is 1, and each of Ra, Rb, and Rc is a hydrogen atom.

[2] The production method according to [1], wherein the polypeptide comprises an amino acid sequence having 95% or higher identity to an amino acid sequence represented by SEQ ID NO: 1.
[3] The production method according to [1] or [2], wherein the polypeptide comprises an amino acid sequence having 98% or higher identity to an amino acid sequence represented by SEQ ID NO: 1.
[4] The production method according to any one of [1] to [3], wherein in the formula (1), T is a phosphorus atom or a sulfur atom, the functional group represented by the formula (1a) is =O, and each of Ra, Rb, and Rc is a methyl group.
[5] The production method according to any one of [1] to [3], wherein the compound D is at least one compound selected from the group consisting of dimethyl sulfoxide, dimethylsulfone, dimethoxyethane, trimethylphosphine oxide, N,N-dimethylformamide, N,N-dimethylacetamide, tetramethylene sulfoxide, diethyl sulfoxide, methanol, and methylformamide.
[6] The production method according to any one of [1] to [5], wherein the compound D is at least one compound selected from the group consisting of dimethyl sulfoxide, dimethylsulfone and trimethylphosphine oxide.
[7] The production method according to any one of [1] to [6], wherein the compound D is dimethyl sulfoxide.
[8] A method for producing an amino acid, comprising a step of performing an intermolecular reductive amination reaction between compound A selected from the group consisting of a compound having an amino group and a salt thereof and compound B selected from the group consisting of a compound having a carbonyl group and a salt thereof,

or an intramolecular reductive amination reaction of compound C selected from the group consisting of a compound having an amino group and a carbonyl group and a salt thereof, in the presence of a polypeptide having catalytic activity for an intermolecular reductive amination reaction between the compound A and the compound B, or an intramolecular reductive amination reaction of the compound C under at least one reaction condition, a reducing agent, and compound D',

wherein the compound D' is at least one compound selected from the group consisting of dimethyl sulfoxide, dimethylsulfone, trimethylphosphine oxide, dimethoxyethane, N,N-dimethylformamide, N,N-dimethylacetamide, tetramethylene sulfoxide, diethyl sulfoxide, methanol, and methylformamide.

[9] The production method according to [8], wherein the compound D' is at least one compound selected from the group consisting of dimethyl sulfoxide, dimethylsulfone and trimethylphosphine oxide.

[10] The production method according to [8] or [9], wherein the compound D' is dimethyl sulfoxide.

[11] The production method according to any one of [1] to [10], wherein the reducing agent is at least one compound selected from the group consisting of reduced nicotinamide adenine dinucleotide phosphate (NADPH), oxidized nicotinamide adenine dinucleotide phosphate (NADP+), reduced nicotinamide adenine dinucleotide (NADH) and oxidized nicotinamide adenine dinucleotide (NAD+).

[12] The production method according to any one of [1] to [11], wherein the reducing agent is reduced nicotinamide adenine dinucleotide phosphate.

[13] The production method according to any one of [1] to [12], wherein the polypeptide has catalytic activity for an intermolecular reductive amination reaction between the compound A and the compound B or an intramolecular reductive amination reaction of the compound C under at least one reaction condition.

[14] The production method according to any one of [1] to [13], wherein the polypeptide has an amino acid sequence represented by SEQ ID NO: 1 with one or more amino acid residues thereof engineered.

[15] The production method according to any one of [1] to [14], wherein the polypeptide comprises an amino acid sequence represented by SEQ ID NO: 8 wherein the amino acid residue represented by X is a histidine residue.

[16] The production method according to any one of [1] to [15], wherein the polypeptide comprises an amino acid sequence other than the sequence having 90% or higher sequence identity to an amino acid sequence represented by SEQ ID NO: 1 with one amino acid residue thereof engineered at any one or both of the N terminus and the C terminus.

[17] The production method according to any one of [1] to [16], wherein the polypeptide comprises a tag sequence at any one or both of the N terminus and the C terminus.

[18] The production method according to any one of [1] to [17], wherein the polypeptide comprises one or more selected from the group consisting of a streptavidin-bound peptide tag sequence and a His tag sequence at any one or both of the N terminus and the C terminus.

[19] The production method according to any one of [1] to [18], wherein the number of amino acid residues of the polypeptide is 300 or more and 400 or less.

[20] The production method according to any one of [1] to [19], wherein the step is a step of performing an intermolecular reductive amination reaction between the compound A and the compound B.

[21] The production method according to [20], wherein the compound A is at least one compound selected from the group consisting of a compound represented by the following formula (2) and a salt thereof:

[Formula 3]

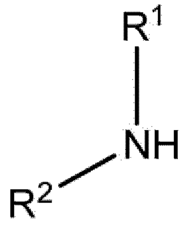

(2)

wherein in the formula (2),

$R^1$ and $R^2$ each independently represent a hydrogen atom, an alkyl group, an alkenyl group, an alkynyl group, a cycloalkyl group, an aryl group, a heterocyclyl group, or a heteroaryl group, these groups are optionally substituted, and at least one of $R^1$ and $R^2$ is a hydrogen atom.

[22] The production method according to [21], wherein in the formula (2), $R^1$ is a hydrogen atom, and $R^2$ is a hydrogen atom or an alkyl group.

[23] The production method according to [21] or [22], wherein in the formula (2), $R^1$ is a hydrogen atom, and $R^2$ is a hydrogen atom, a methyl group, or an ethyl group.

[24] The production method according to any one of [20] to [23], wherein the compound B is at least one compound

selected from the group consisting of a compound represented by the following formula (3) and a salt thereof:

[Formula 4]

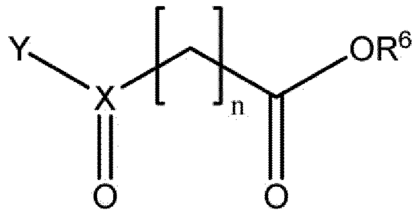

(3)

[Formula 5]

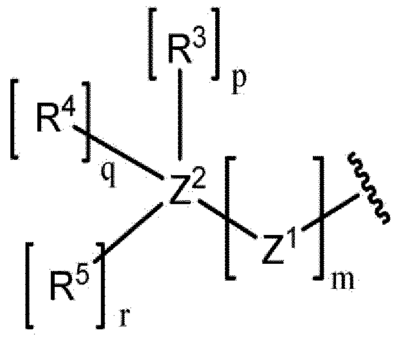

(4)

wherein in the formula (3),

X represents a carbon atom,
Y represents a hydrogen atom, or a group represented by the formula (4),
n represents an integer of 0 or more and 2 or less, and
$R^6$ represents a hydrogen atom, an optionally substituted $C_1$ to $C_6$ alkyl group, an optionally substituted $C_1$ to $C_6$ aryl group, an optionally substituted heteroaryl group having 5 or more and 12 or less ring-constituting atoms, a group containing a nitrogen atom, or a group containing an oxygen atom,
in the formula (4),

[Formula 6]

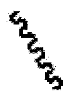

represents a binding point to X,

m represents an integer of 0 or more and 6 or less,
p is 0 or 1,
q is 0 or 1,
r is 0 or 1,
$Z^1$ represents an optionally substituted alkylene group, or an ether bond-containing group having 1 or more and 6 or less carbon atoms, and when m is an integer of 2 or more, a plurality of $Z^1$ are the same or different,
$Z^2$ represents a carbon atom,
$R^3$, $R^4$, and $R^5$ each independently represent a hydrogen atom, an optionally substituted $C_1$ to $C_6$ alkyl group, an optionally substituted $C_5$ to $C_{12}$ aryl group, an optionally substituted heteroaryl group having 5 or more and 12 or less ring-constituting atoms, a group containing a nitrogen atom, or a group containing an oxygen atom, any two or more of $R^3$, $R^4$, and $R^5$ are optionally linked to each other to form a ring structure together with $Z^2$, the ring structure is optionally a cycloalkyl group, an aryl group, a heterocyclyl group, or a heteroaryl group, and these groups are optionally substituted,

$R^3$, $R^4$, and $R^5$ each optionally form a double bond or a triple bond with $Z^2$, and when any one of $R^3$, $R^4$, and $R^5$ is bonded to $Z^2$ through a double bond or a triple bond, at least one of p, q and r is 0.

[25] The production method according to [24], wherein in the formula (3), n is 0, $R^6$ is a hydrogen atom, Y is a $C_3$ to $C_8$ cycloalkyl group, or a $C_6$ to $C_9$ aralkyl group, and the aralkyl group is optionally substituted by a $C_1$ to $C_3$ alkyl group or halogen.
[26] The production method according to [24] or [25], wherein in the formula (3), n is 0, $R^6$ is a hydrogen atom, and Y is a (2-chlorophenyl)ethyl group, a phenylmethyl group, or a cyclopentyl group.
[27] The production method according to any one of [24] to [26], wherein in the formula (2), $R^1$ is a hydrogen atom, and $R^2$ is a hydrogen atom or a methyl group; and in the formula (3), each of n and m is 0, Y is a group represented by formula (4), $R^3$ is a (2-chlorophenyl)methyl group, each of $R^4$, $R^5$ and $R^6$ is a hydrogen atom, and each of p, q and r is 1.
[28] The production method according to any one of [24] to [26], wherein in the formula (2), $R^1$ is a hydrogen atom, and $R^2$ is an ethyl group; and in the formula (3), each of n and m is 0, Y is a group represented by formula (4), $R^3$ is a phenyl group, each of $R^4$, $R^5$ and $R^6$ is a hydrogen atom, and each of p, q and r is 1.
[29] The production method according to any one of [24] to [26], wherein in the formula (2), $R^1$ is a hydrogen atom, and $R^2$ is a methyl group; and in the formula (3), each of n and m is 0, Y is a group represented by formula (4), $R^3$ and $R^4$ are linked to each other to form a cyclopentane ring with $Z^2$, each of $R^5$ and $R^6$ is a hydrogen atom, and each of p, q and r is 1.
[30] The production method according to any one of [24] to [26], wherein in the formula (4), at least one of $R^3$, $R^4$ and $R^5$ is not methyl.
[31] The production method according to any one of [1] to [20], wherein the compound A is at least one compound selected from the group consisting of ammonia, methylamine, ethylamine and a salt thereof.
[32] The production method according to any one of [1] to [20] or [31], wherein the compound B is at least one compound selected from the group consisting of 4-(2-chlorophenyl)-2-oxobutanoic acid, phenylpyruvic acid, 2-cyclopentyl-2-oxo-acetic acid and a salt thereof.
[33] The production method according to any one of [1] to [20], or [31] or [32], wherein the compound A is ammonia or a salt thereof, or methylamine or a salt thereof, and the compound B is 4-(2-chlorophenyl)-2-oxobutanoic acid or a salt thereof, or phenylpyruvic acid or a salt thereof.
[34] The production method according to any one of [1] to [20], or [31] or [32], wherein the compound A is ethylamine or a salt thereof, and the compound B is phenylpyruvic acid or a salt thereof.
[35] The production method according to any one of [1] to [20], or [31] or [32], wherein the compound A is methylamine or a salt thereof, and the compound B is 2-cyclopentyl-2-oxoacetic acid or a salt thereof.
[36] The production method according to any one of [1] to [35], wherein in the step, a concentration of the compound A at the start of reaction is 100 mM or higher and 3000 mM or lower in the total amount of a reaction solution.
[37] The production method according to any one of [1] to [36], wherein in the step, a concentration of the compound B at the start of reaction is 0.001 mM or higher and 1000 mM or lower in the total amount of a reaction solution.
[38] The production method according to any one of [1] to [37], wherein the step is performed under conditions in which a ratio of a concentration of the compound A to a concentration of the compound B in a reaction solution is 1 or more.
[39] The production method according to any one of [1] to [19], wherein the step is a step of performing an intramolecular reductive amination reaction of the compound C.
[40] The production method according to [39], wherein the compound C is a compound represented by the following formula (5):

[Formula 7]

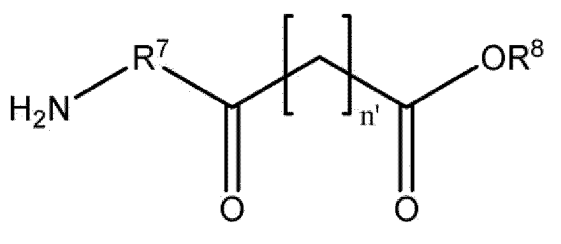

(5)

wherein in the formula (5), n' represents an integer of 0 or more and 2 or less, and $R^7$ represent an alkylene group, an alkenylene group, an alkynylene group, a cycloalkylene group, an arylene group, a heterocyclylene group, or a heteroarylene group, and these groups are optionally substituted, $R^8$ represents a hydrogen atom, an optionally substituted $C_1$ to $C_6$ alkyl group, an optionally substituted $C_1$ to $C_6$ aryl group, an optionally substituted heteroaryl group having 5 or more and 12 or less ring-constituting atoms, a group containing a nitrogen atom, or a group

containing an oxygen atom.

[41] The production method according to any one of [1] to [40], wherein in the step, a concentration of the compound C at the start of reaction is 0.001 mM or higher and 1000 mM or lower in the total amount of a reaction solution.

[42] The production method according to any one of [1] to [41], wherein in the step, glucose and glucose dehydrogenase are present in a reaction solution.

[43] The production method according to any one of [1] to [42], wherein the step is performed under appropriate reaction conditions.

[44] The production method according to any one of [1] to [43], wherein the step is performed under temperature conditions of 0°C or higher and 50°C or lower.

[45] The production method according to any one of [1] to [44], wherein the step is performed under pH conditions of 7 or higher and 11 or lower.

[46] The production method according to any one of [1] to [45], wherein the step is performed under conditions in which a concentration of compound D or compound D' in a reaction solution is 1 v/v% or more and 60 v/v% or less if the compound D or compound D' is a liquid under conditions of 25°C and 1 atm, and a concentration of compound D or compound D' in a reaction solution is 1 w/v% or more and 60 v/v% or less if the compound D or compound D' is a solid under conditions of 25°C and 1 atm.

[47] The production method according to any one of [1] to [46], wherein the step is performed under conditions in which a concentration of the polypeptide in a reaction solution is 0.1 μM or more and 10 μM or less.

[48] The production method according to any one of [1] to [47], wherein the step is performed under conditions in which a concentration of the reducing agent in a reaction solution is 0.1 mM or more and 100 mM or less.

[49] The production method according to any one of [1] to [48], wherein a yield of an amino acid formed through a reaction in the presence of the compound D or the compound D' is 1.2 or more times a yield of an amino acid formed through a reaction under conditions of the absence of the compound D or the compound D'.

[50] The production method according to any one of [1] to [49], wherein a yield of an amino acid formed in the step is 50% or more.

[51] The production method according to any one of [1] to [50], wherein a yield of an amino acid formed in the step is measured by the following conditions: the reaction is started with 50 mM phenylpyruvic acid, 2-oxo-3-(p-tolyl) propanoic acid, or 2-cyclopentyl-2-oxo-acetic acid, 100 mM D(+)-glucose, 500 mM ammonia, methylamine or ethylamine, a 100 mM phosphate buffer solution, 1 mM NADPH, a 0.002 unit/μL GDH solution, the polypeptide at a concentration of 2.5 μM, and when the compound D or compound D' is used, the compound D or compound D' at 20 v/v% if the compound D or compound D' is a liquid under conditions of 25°C and 1 atm, or the compound D or compound D' at 20 w/v% if the compound D or compound D' is a solid under conditions of 25°C and 1 atm, in a reaction solution under conditions of 37°C and pH 8 to 9, and a yield of an amino acid formed through reductive amination reaction is determined after a lapse of 19 hours.

[52] The production method according to any one of [1] to [51], wherein catalytic activity of the polypeptide is evaluated under the following conditions: the reaction is started with 50 mM sodium 4-(2-chlorophenyl)-2-oxobutanoate, sodium phenylpyruvate, or sodium 2-cyclopentyl-2-oxoacetate, 100 mM D(+)-glucose, 500 mM methylamine or ethylamine, a 100 mM phosphate buffer solution, 1 mM NADPH, a 0.002 unit/μL GDH solution, the polypeptide to be evaluated at 2.5 μM, and the compound D or compound D' at 20 v/v% or 20 w/v% in a reaction solution under conditions of 25°C or 37°C and pH 8 to 9, and a yield of an amino acid formed through reductive amination reaction is determined after a lapse of 3 or 23 hours.

[53] A method for producing a peptide compound, comprising the steps of:

(1) producing an amino acid by the production method according to any one of [1] to [51]; and
(2) linking the amino acid to one or more selected from the group consisting of other amino acids and a peptide to produce a peptide compound.

[54] A reductive amination reaction accelerator represented by the following formula (1), which accelerates the following reaction (i) or (ii):

(i) an intermolecular reductive amination reaction between compound A selected from the group consisting of a compound having an amino group and a salt thereof and compound B selected from the group consisting of a compound having a carbonyl group and a salt thereof;
(ii) an intramolecular reductive amination reaction of compound C selected from the group consisting of a compound having an amino group and a carbonyl group and a salt thereof:

[Formula 8]

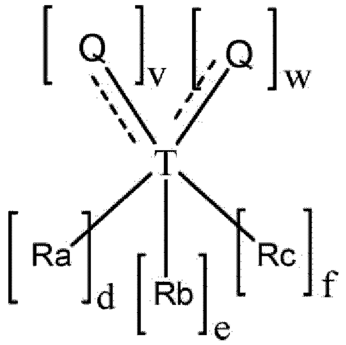

(1)

wherein in the formula (1),

v and w each independently represent 0 or 1,
at least one of v and w represents 1,
T represents a carbon atom, a phosphorus atom, or a sulfur atom,
a functional group represented by the following formula (1a):

[Formula 9]

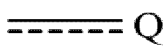

(1 a)

represents =O, -ORd, or a hydroxy group,
when each of v and w is 1, two functional groups represented by a plurality of formulas (1a) are the same or different,
Ra, Rb, and Rc each independently represent a hydrogen atom, a $C_1$ to $C_3$ alkyl group, an alkylamino group, or $-CH_2-ORd$,
at least any two of Ra, Rb, and Rc are optionally linked to each other to form a ring structure together with T,
Rd represents a $C_1$ to $C_3$ alkyl group,
d, e, and f each independently represent 0 or 1,
at least one of d, e, and f represents 1,
when each of v and w is 1, at least one of Ra, Rb, and Rc is a methyl group, and none of Ra, Rb, and Rc are linked to each other to form a ring structure together with T,
when at least one of Ra, Rb, and Rc is a methylamino group, none of Ra, Rb, and Rc are linked to each other to form a ring structure together with T, and
when the functional group represented by the formula (1a) is a hydroxy group and T is a carbon atom, each of d, e, and f is 1, and each of Ra, Rb, and Rc is a hydrogen atom.

[55] The reductive amination reaction accelerator according to [54], wherein in the formula (1), T represents a phosphorus atom or a sulfur atom, the functional group represented by the formula (1a) is =O, and each of Ra, Rb, and Rc is a methyl group.
[56] The reductive amination reaction accelerator according to [54] or [55], which is at least one compound selected from the group consisting of dimethyl sulfoxide, dimethylsulfone, dimethoxyethane, trimethylphosphine oxide, N,N-dimethylformamide, N,N-dimethylacetamide, tetramethylene sulfoxide, diethyl sulfoxide, methanol, and methylformamide.
[57] The reductive amination reaction accelerator according to any one of [54] to [56], which is at least one compound selected from the group consisting of dimethyl sulfoxide, dimethylsulfone and trimethylphosphine oxide.
[58] The reductive amination reaction accelerator according to any one of [54] to [57], which is dimethyl sulfoxide.

[Advantageous Effects of Invention]

According to the present invention, a method for efficiently producing an amino acid can be provided.

[BRIEF DESCRIPTION OF THE DRAWINGS]

FIG. 1 is $^1$H-NMR measurement spectra of (2S, 3S) - 2-amino-3-phenyl-butanoic acid (I) obtained by the reaction in the example, purchased standard compounds (2S, 3R) - 2-amino-3-phenyl-butanoic acid hydrochloride (II) and (2S, 3S) - 2-amino-3-phenyl-butanoic acid hydrochloride (III).

FIG. 2 is chiral HPLC analysis data of (2S, 3S)-2-amino-3-phenylbutanoic acid (I) obtained by the reaction in the Example and a mixture (V) prepared by mixing the purchased standard compounds (III) and (IV) at a ratio of (III) : (IV) = 7 : 3.

[Description of Embodiments]

Hereinafter, the embodiments for carrying out the present invention will be described in detail. However, the present invention is not limited by embodiments given below.

In the present specification, the term "one or more" means a number of 1 or 2 or larger. When the term "one or more" is used in a context related to a substituent for a certain group, this term means a number from 1 to the maximum number of substituents accepted by the group. Examples of the term "one or more" specifically include 1, 2, 3, 4, 5, 6, 7, 8, 9, 10, and/or larger numbers.

In the present specification, the term "to" that indicates a range includes values of both ends thereof. For example, "A to B" means the range of A or more and B or less.

In the present specification, the term "approximately", when used in combination with a numeric value, means the value range of +10% and -10% of the numeric value.

In the present specification, the term "and/or" is meant to include every combination of the terms "and" and "or" appropriately combined. Specifically, for example, the term "A, B and/or C" includes the following seven engineerings: (i) A, (ii) B, (iii) C, (iv) A and B, (v) A and C, (vi) B and C, and (vii) A, B and C.

In the present specification, the "alkyl group" is a monovalent group induced by the removal of one arbitrary hydrogen atom from aliphatic hydrocarbon, and has a subset of a hydrocarbyl or hydrocarbon group structure containing hydrogen atoms and carbon atoms without containing a heteroatom (which refers to an atom other than carbon atoms and hydrogen atoms) or an unsaturated carbon-carbon bond in the skeleton. The alkyl group includes not only a linear form but a branched form. The alkyl group is preferably an alkyl group having 1 to 20 carbon atoms ($C_1$ to $C_{20}$; hereinafter, "$C_p$ to $C_q$" means that the number of carbon atoms is p to q), preferably a $C_1$ to $C_{10}$ alkyl group, more preferably a $C_1$ to $C_6$ alkyl group, further preferably a $C_1$ to $C_3$ alkyl group. Examples of the alkyl group specifically include a methyl group, an ethyl group, a n-propyl group, an i-propyl group, a n-butyl group, a s-butyl group, a t-butyl group, an isobutyl (2-methylpropyl) group, a n-pentyl group, a s-pentyl (1-methylbutyl) group, a t-pentyl (1,1-dimethylpropyl) group, a neopentyl (2,2-dimethylpropyl) group, an isopentyl (3-methylbutyl) group, a 3-pentyl (1-ethylpropyl) group, a 1,2-dimethylpropyl group, a 2-methylbutyl group, a n-hexyl group, a 1,1,2-trimethylpropyl group, a 1,2,2-trimethylpropyl group, a 1,1,2,2-tetramethylpropyl group, a 1,1-dimethylbutyl group, a 1,2-dimethylbutyl group, a 1,3-dimethylbutyl group, a 2,2-dimethylbutyl group, a 2,3-dimethylbutyl group, a 3,3-dimethylbutyl group, a 1-ethylbutyl group, and a 2-ethylbutyl group.

In the present specification, the "alkenyl group" is a monovalent group having at least one double bond (two adjacent $SP^2$ carbon atoms). Depending on the conformation of the double bond and a substituent (if present), the geometric morphology of the double bond can assume entgegen (E) or zusammen (Z) and cis or trans conformations. The alkenyl group includes not only a linear form but a branched form. The alkenyl group is preferably a $C_2$ to $C_{10}$ alkenyl group, more preferably a $C_2$ to $C_6$ alkenyl group. Examples thereof specifically include a vinyl group, an allyl group, a 1-propenyl group, a 2-propenyl group, a 1-butenyl group, a 2-butenyl group (which includes cis and trans), a 3-butenyl group, a pentenyl group, a 3-methyl-2-butenyl group, and a hexenyl group.

In the present specification, the "alkynyl group" is a monovalent group having at least one triple bond (two adjacent SP carbon atoms). The alkynyl group includes not only a linear form but a branched form. The alkynyl group is preferably a $C_2$ to $C_{10}$ alkynyl group, more preferably a $C_2$ to $C_6$ alkynyl group. Examples thereof specifically include an ethynyl group, a 1-propynyl group, a propargyl group, a 3-butynyl group, a pentynyl group, a hexynyl group, a 3-phenyl-2-propynyl group, a 3-(2'-fluorophenyl)-2-propynyl group, a 2-hydroxy-2-propynyl group, a 3-(3-fluorophenyl)-2-propynyl group, and a 3-methyl-(5-phenyl)-4-pentynyl group.

In the present specification, the "cycloalkyl group" means a saturated or partially saturated cyclic monovalent aliphatic hydrocarbon group. The cycloalkyl group includes a monocyclic ring, a bicyclo ring, or a spiro ring. Preferred examples of the cycloalkyl group include a $C_3$ to $C_8$ cycloalkyl group. Examples thereof specifically include a cyclopropyl group, a cyclobutyl group, a cyclopentyl group, a cyclohexyl group, a cycloheptyl group, a cyclooctyl group, a bicyclo[2.2.1]heptyl group, and a spiro[3.3]heptyl group.

In the present specification, the "aryl group" means a monovalent aromatic hydrocarbon ring. Preferred

examples of the aryl group include a $C_6$ to $C_{10}$ aryl group. Examples of the aryl group specifically include a phenyl group and a naphthyl (e.g., 1-naphthyl and 2-naphthyl) group.

In the present specification, the "heterocyclyl group" means a nonaromatic cyclic monovalent group containing a carbon atom as well as 1 to 5 heteroatoms. The heterocyclyl group may have a double and/or triple bond in the ring. A carbon atom in the ring may form carbonyl through oxidation, and the ring may be a monocyclic ring or a condensed ring. In the case of a condensed ring, an aromatic ring such as a benzene ring, a pyridine ring, or a pyrimidine ring may form a condensed ring with a saturated cycloaliphatic ring such as a cyclopentane ring or a cyclohexane ring, or a saturated heterocyclic ring such as a tetrahydropyran ring, a dioxane ring, or a pyrrolidine ring. The number of atoms constituting the ring is preferably 4 to 10 (4- to 10-membered heterocyclyl group), more preferably 4 to 7 (4-to 7-membered heterocyclyl group). Examples of the heterocyclyl group specifically include an azetidinyl group, an oxiranyl group, an oxetanyl group, an azetidinyl group, a dihydrofuryl group, a tetrahydrofuryl group, a dihydropyranyl group, a tetrahydropyranyl group, a tetrahydropyridyl group, a tetrahydropyrimidyl group, a morpholinyl group, a thiomorpholinyl group, a pyrrolidinyl group, a piperidinyl group, a piperazinyl group, a pyrazolidinyl group, an imidazolinyl group, an imidazolidinyl group, an oxazolidinyl group, an isoxazolidinyl group, a thiazolidinyl group, an isothiazolidinyl group, a 1,2-thiazinane group, a thiadiazolidinyl group, an azetidinyl group, an oxazolidone group, a benzodioxanyl group, a benzoxazolyl group, a dioxolanyl group, a dioxanyl group, a tetrahydropyrrolo[1,2-c]imidazole group, a thietanyl group, a 3,6-diazabicyclo[3.1.1]heptanyl group, a 2,5-diazabicyclo[2.2.1]heptanyl group, a 3-oxa-8-azabicyclo[3.2.1]octanyl group, a sultam group, and a 2-oxaspiro[3.3]heptyl group.

In the present specification, the "protected heterocyclyl group" means a group in which one or more functional groups, for example, an amino group, contained in the "heterocyclyl group" defined above, is protected with an arbitrary protective group. The protected heterocyclyl is preferably a protected 4- to 7-membered heterocyclyl group. Specific examples of the protective group include Boc, Fmoc, Cbz, Troc, and Alloc. Specific examples of the protected heterocyclyl include Boc-protected azetidine.

In the present specification, the "heterocyclidene group" means a divalent group obtained by removing two hydrogen atoms from one carbon atom present in a non-aromatic ring containing a carbon atom as well as 1 to 5 heteroatoms. The free valence in the heterocyclidene group may constitute or may not constitute a portion of a double bond. The non-aromatic ring containing a carbon atom as well as 1 to 5 heteroatoms may be the same as the ring described in the "heterocyclyl group". The heterocyclidene group is preferably a 4- to 7-membered heterocyclidene group. Specific examples thereof include a tetrahydropyran-4-ylidene group and an azetidin-3-ylidene group.

In the present specification, the "protected heterocyclidene group" means a group in which one or more functional groups, for example, an amino group, contained in the "heterocyclidene group" defined above, is protected with an arbitrary protective group. The protected heterocyclidene group is preferably a protected 4- to 7-membered heterocyclidene group. Specific examples of the protective group include Boc, Fmoc, Cbz, Troc, and Alloc. Specific examples of the protected heterocyclidene group include a Boc-protected azetidin-3-ylidene group.

In the present specification, the "heteroaryl group" means an aromatic cyclic monovalent group containing a carbon atom as well as 1 to 5 heteroatoms. The ring may be a monocyclic ring or a condensed ring with another ring and may be partially saturated. The number of atoms constituting the ring may be 5 to 12 (5- to 12-membered heteroaryl group), may be 6 to 10 (6- to 10-membered heteroaryl group), or may be 6 or 7 (6- or 7-membered heteroaryl group). Examples of the heteroaryl group specifically include a furyl group, a thienyl group, a pyrrolyl group, an imidazolyl group, a pyrazolyl group, a thiazolyl group, an isothiazolyl group, an oxazolyl group, an isoxazolyl group, an oxadiazolyl group, a thiadiazolyl group, a triazolyl group, a tetrazolyl group, a pyridyl group, a pyrimidyl group, a pyridazinyl group, a pyrazinyl group, a triazinyl group, a benzofuranyl group, a benzothienyl group, a benzothiadiazolyl group, a benzothiazolyl group, a benzoxazolyl group, a benzoxadiazolyl group, a benzimidazolyl group, an indolyl group, an isoindolyl group, an indazolyl group, a quinolyl group, an isoquinolyl group, a cinnolinyl group, a quinazolinyl group, a quinoxalinyl group, a benzodioxolyl group, an indolizinyl group, and an imidazopyridyl group.

In the present specification, the "alkoxy group" means an oxy group bonded to the "alkyl" defined above. The alkoxy group is preferably a $C_1$ to $C_6$ alkoxy group. Examples of the alkoxy specifically include a methoxy group, an ethoxy group, a 1-propoxy group, a 2-propoxy group, a n-butoxy group, an i-butoxy group, a s-butoxy group, a t-butoxy group, a pentyloxy group, and a 3-methylbutoxy group.

In the present specification, the "alkylthio group" means a thio group bonded to the "alkyl group" defined above. The alkylthio group is preferably a $C_1$ to $C_6$ alkylthio group. Examples of the alkylthio group specifically include a methylthio group, an ethylthio group, a 1-propylthio group, a 2-propylthio group, a n-butylthio group, an i-butylthio group, a s-butylthio group, and a t-butylthio group.

In the present specification, the "alkenyloxy group" means an oxy group bonded to the "alkenyl group" defined above. The alkenyloxy group is preferably a $C_2$ to $C_6$ alkenyloxy group. Examples of the alkenyloxy group specifically include a vinyloxy group, an allyloxy group, a 1-propenyloxy group, a 2-propenyloxy group, a 1-butenyloxy group, a 2-butenyloxy group (which includes cis and trans), a 3-butenyloxy group, a pentenyloxy group, and a hexenyloxy group.

In the present specification, the "cycloalkoxy group" means an oxy group bonded to the "cycloalkyl group" defined

above. The cycloalkoxy group is preferably a $C_3$ to $C_8$ cycloalkoxy group. Examples of the cycloalkoxy group specifically include a cyclopropoxy group, a cyclobutoxy group, and a cyclopentyloxy group.

In the present specification, the "aryloxy group" means an oxy group bonded to the "aryl group" defined above. The aryloxy group is preferably a $C_6$ to $C_{10}$ aryloxy group. Examples of the aryloxy group specifically include a phenoxy group, a 1-naphthyloxy group, and a 2-naphthyloxy group.

In the present specification, the "amino group" means $-NH_2$ in the narrow sense and means -NRR' in the broad sense. In this context, R and R' are each independently selected from a hydrogen atom, an alkyl group, an alkenyl group, an alkynyl group, a cycloalkyl group, a heterocyclyl group, an aryl group, and a heteroaryl group, or R and R' form a ring together with the nitrogen atom bonded thereto. Examples of the amino group preferably include $-NH_2$, a mono-$C_1$ to $C_6$ alkylamino group, a di-$C_1$ to $C_6$ alkylamino group, and a 4- to 8-membered cyclic amino group.

In the present specification, the "monoalkylamino group" means a group of the "amino group" defined above in which R is a hydrogen atom, and R' is the "alkyl group" defined above. The monoalkylamino group is preferably a mono-$C_1$ to $C_6$ alkylamino group, and more preferably a mono-$C_1$ to $C_3$ alkylamino group. Examples of the monoalkylamino group specifically include a methylamino group, an ethylamino group, a n-propylamino group, an i-propylamino group, a n-butylamino group, a s-butylamino group, and a t-butylamino group.

In the present specification, the "dialkylamino group" means a group of the "amino group" defined above in which R and R' are each independently the "alkyl group" defined above. The dialkylamino group is preferably a di-$C_1$ to $C_6$ alkylamino group, more preferably a di-$C_1$ to $C_3$ alkylamino group. Specific examples of the dialkylamino group include a dimethylamino group and a diethylamino group. In the present specification, a monoalkylamino group and a dialkylamino group are also collectively referred to as an "alkylamino group". For example, a "methylamino group" may include a monomethylamino group and a dimethylamino group.

In the present specification, the "cyclic amino group" means a group of the "amino group" defined above in which R and R' form a ring together with the nitrogen atom bonded thereto. The cyclic amino group is preferably a 4- to 8-membered cyclic amino group. Examples of the cyclic amino group specifically include a 1-azetidyl group, a 1-pyrrolidyl group, a 1-piperidyl group, a 1-piperazyl group, a 4-morpholinyl group, a 3-oxazolidyl group, a 1,1-dioxidothiomorpholinyl-4-yl group, and a 3-oxa-8-azabicyclo[3.2.1]octan-8-yl group.

In the present specification, the "protected amino group" means an amino group protected with an arbitrary protective group. Examples of the protected amino specifically include an amino group protected with a protective group such as Boc, Fmoc, Cbz, Troc, or Alloc.

In the present specification, the "aminocarbonyl group" means a carbonyl group bonded to the "amino group" defined above. The aminocarbonyl group is preferably $-CONH_2$, a mono-$C_1$ to $C_6$ alkylaminocarbonyl group, a di-$C_1$ to $C_6$ alkylaminocarbonyl group, or a 4- to 8-membered cyclic aminocarbonyl group. Examples of the aminocarbonyl group specifically include $-CONH_2$, a dimethylaminocarbonyl group, a 1-azetidinylcarbonyl group, a 1-pyrrolidinylcarbonyl group, a 1-piperidinylcarbonyl group, a 1-piperazinylcarbonyl group, a 4-morpholinylcarbonyl group, a 3-oxazolidinylcarbonyl group, a 1,1-dioxidothiomorpholinyl-4-ylcarbonyl group, and a 3-oxa-8-azabicyclo[3.2.1]octane-8-ylcarbonyl group.

In the present specification, the "alkenyloxycarbonyl group" means a carbonyl group bonded to the "alkenyloxy group" defined above. The alkenyloxycarbonyl is preferably a $C_2$ to $C_6$ alkenyloxycarbonyl group. Examples of the alkenyloxycarbonyl group specifically include a vinyloxycarbonyl group, an allyloxycarbonyl group, a 1-propenyloxycarbonyl group, a 2-propenyloxycarbonyl group, a 1-butenyloxycarbonyl group, a 2-butenyloxycarbonyl group (which includes cis and trans), a 3-butenyloxycarbonyl group, a pentenyloxycarbonyl group, and a hexenyloxycarbonyl group.

In the present specification, the "alkylsulfonyl group" means a sulfonyl group bonded to the "alkyl group" defined above. The alkylsulfonyl group is preferably a $C_1$ to $C_6$ alkylsulfonyl group. Examples of the alkylsulfonyl group specifically include a methylsulfonyl group.

In the present specification, the "hydroxyalkyl group" means a group in which one or more hydrogens of the "alkyl group" defined above are replaced with a hydroxy group. The hydroxyalkyl group is preferably a hydroxy $C_1$ to $C_6$ alkyl group. Examples of the hydroxyalkyl group specifically include a hydroxymethyl group, a 1-hydroxyethyl group, a 2-hydroxyethyl group, a 2-hydroxy-2-methylpropyl group, and a 5-hydroxypentyl group.

In the present specification, the "haloalkyl group" means a group in which one or more hydrogen atoms of the "alkyl group" defined above are replaced with a halogen. The haloalkyl group is preferably a halo $C_1$ to $C_6$ alkyl group, more preferably a $C_1$ to $C_6$ fluoroalkyl group. Examples of the haloalkyl group specifically include a difluoromethyl group, a trifluoromethyl group, a 2,2-difluoroethyl group, a 2,2,2-trifluoroethyl group, a 3,3-difluoropropyl group, a 4,4-difluorobutyl group, and a 5,5-difluoropentyl group.

In the present specification, the "cyanoalkyl group" means a group in which one or more hydrogen atoms of the "alkyl group" defined above are replaced with a cyano group. The cyanoalkyl group is preferably a cyano $C_1$ to $C_6$ alkyl group. Examples of the cyanoalkyl group specifically include a cyanomethyl group and a 2-cyanoethyl group.

In the present specification, the "aminoalkyl group" means a group in which one or more hydrogens of the "alkyl group" defined above are replaced with the "amino group" defined above. The aminoalkyl group is preferably an amino $C_1$

to $C_6$ alkyl group. Examples of the aminoalkyl group specifically include a 1-pyridylmethyl group, a 2-(1-piperidyl)ethyl group, a 3-(1-piperidyl)propyl group, and a 4-aminobutyl group.

In the present specification, the "carboxyalkyl group" means a group in which one or more hydrogen atoms of the "alkyl group" defined above are replaced with a carboxy group. The carboxyalkyl group is preferably a $C_2$ to $C_6$ carboxyalkyl group. Examples of the carboxyalkyl group specifically include a carboxymethyl group.

In the present specification, the "alkenyloxycarbonylalkyl group" means a group in which one or more hydrogen atoms of the "alkyl group" defined above are replaced with the "alkenyloxycarbonyl group" defined above. The alkenyloxycarbonylalkyl group is preferably a $C_2$ to $C_6$ alkenyloxycarbonyl-$C_1$ to $C_6$ alkyl group, more preferably a $C_2$ to $C_6$ alkenyloxycarbonyl-$C_1$ or $C_2$ alkyl group. Examples of the alkenyloxycarbonylalkyl group specifically include an allyloxycarbonylmethyl group and a 2-(allyloxycarbonyl)ethyl group.

In the present specification, the "alkoxyalkyl group" means a group in which one or more hydrogen atoms of the "alkyl group" defined above are replaced with the "alkoxy group" defined above. The alkoxyalkyl group is preferably a $C_1$ to $C_6$ alkoxy-$C_1$ to $C_6$ alkyl group, more preferably a $C_1$ to $C_6$ alkoxy-$C_1$ or $C_2$ alkyl group. Examples of the alkoxyalkyl group specifically include a methoxymethyl group, an ethoxymethyl group, a 1-propoxymethyl group, a 2-propoxymethyl group, a n-butoxymethyl group, an i-butoxymethyl group, a s-butoxymethyl group, a t-butoxymethyl group, a pentyloxymethyl group, a 3-methylbutoxymethyl group, a 1-methoxyethyl group, a 2-methoxyethyl group, and a 2-ethoxyethyl group.

In the present specification, the "alkylthioalkyl group" means a group in which one or more hydrogen atoms of the "alkyl group" defined above are replaced with the "alkylthio group" defined above. The alkylthioalkyl group is preferably a $C_1$ to $C_6$ alkylthio-$C_1$ to $C_6$ alkyl group, more preferably a $C_1$ to $C_6$ alkylthio-$C_1$ or $C_2$ alkyl group. Examples of the alkylthioalkyl group specifically include a methylthiomethyl group, an ethylthiomethyl group, a 1-propylthiomethyl group, a 2-propylthiomethyl group, a n-butylthiomethyl group, an i-butylthiomethyl group, a s-butylthiomethyl group, and a t-butylthiomethyl group.

In the present specification, the "alkenyloxyalkyl group" means a group in which one or more hydrogen atoms of the "alkyl group" defined above are replaced with the "alkenyloxy group" defined above. The alkenyloxyalkyl group is preferably a $C_2$ to $C_6$ alkenyloxy-$C_1$ to $C_6$ alkyl group, more preferably a $C_1$ to $C_6$ alkenyloxy-$C_1$ or $C_2$ alkyl group. Examples of the alkenyloxyalkyl group specifically include a vinyloxymethyl group and an allyloxymethyl group.

In the present specification, the "cycloalkylalkyl group" means a group in which one or more hydrogen atoms of the "alkyl group" defined above are replaced with the "cycloalkyl group" defined above. The cycloalkylalkyl group is preferably a $C_3$ to $C_8$ cycloalkyl-$C_1$ to $C_6$ alkyl group, more preferably a $C_3$ to $C_6$ cycloalkyl-$C_1$ or $C_2$ alkyl group. Examples of the cycloalkylalkyl group specifically include a cyclopropylmethyl group, a cyclobutylmethyl group, a cyclopentylmethyl group, and a cyclohexylmethyl group.

In the present specification, the "cycloalkoxyalkyl group" means a group in which one or more hydrogen atoms of the "alkyl group" defined above are replaced with the "cycloalkoxy group" defined above. The cycloalkoxyalkyl group is preferably a $C_3$ to $C_8$ cycloalkoxy-$C_1$ to $C_6$ alkyl group, more preferably a $C_3$ to $C_6$ cycloalkoxy-$C_1$ or $C_2$ alkyl group. Examples of the cycloalkoxyalkyl group specifically include a cyclopropoxymethyl group and a cyclobutoxymethyl group.

In the present specification, the "heterocyclylalkyl group" means a group in which one or more hydrogen atoms of the "alkyl group" defined above are replaced with the "heterocyclyl group" defined above. The heterocyclylalkyl group is preferably a 4- to 7-membered heterocyclyl-$C_1$ to $C_6$ alkyl group, more preferably a 4- to 7-membered heterocyclyl-$C_1$ or $C_2$ alkyl group. Examples of the heterocyclylalkyl group specifically include a 2-(tetrahydro-2H-pyran-4-yl)ethyl group and a 2-(azetidin-3-yl)ethyl group.

In the present specification, the "alkylsulfonylalkyl group" means a group in which one or more hydrogen atoms of the "alkyl group" defined above are replaced with the "alkylsulfonyl group" defined above. The alkylsulfonylalkyl group is preferably a $C_1$ to $C_6$ alkylsulfonyl-$C_1$ to $C_6$ alkyl group, more preferably a $C_1$ to $C_6$ alkylsulfonyl-$C_1$ or $C_2$ alkyl group. Examples of the alkylsulfonylalkyl group specifically include a methylsulfonylmethyl group and a 2-(methylsulfonyl)ethyl group.

In the present specification, the "aminocarbonylalkyl group" means a group in which one or more hydrogen atoms of the "alkyl" defined above are replaced with the "aminocarbonyl group" defined above. The aminocarbonylalkyl group is preferably an aminocarbonyl-$C_1$ to $C_6$ alkyl group, more preferably an aminocarbonyl-$C_1$ to $C_4$ alkyl group. Examples of the aminocarbonylalkyl group specifically include a methylaminocarbonylmethyl group, a dimethylaminocarbonylmethyl group, a t-butylaminocarbonylmethyl group, a 1-azetidinylcarbonylmethyl group, a 1-pyrrolidinylcarbonylmethyl group, a 1-piperidinylcarbonylmethyl group, a 4-morpholinylcarbonylmethyl group, a 2-(methylaminocarbonyl)ethyl group, a 2-(dimethylaminocarbonyl)ethyl group, a 2-(1-azetidinylcarbonyl)ethyl group, a 2-(1-pyrrolidinylcarbonyl)ethyl group, a 2-(4-morpholinylcarbonyl)ethyl group, a 3-(dimethylaminocarbonyl)propyl group, and a 4-(dimethylaminocarbonyl)butyl group.

In the present specification, the "aryloxyalkyl group" means a group in which one or more hydrogen atoms of the "alkyl group" defined above are replaced with the "aryloxy group" defined above. The aryloxyalkyl group is preferably a $C_6$ to $C_{10}$ aryloxy-$C_1$ to $C_6$ alkyl group, more preferably a $C_6$ to $C_{10}$ aryloxy-$C_1$ or $C_2$ alkyl group. Examples of the aryloxyalkyl group specifically include a phenoxymethyl group and a 2-phenoxyethyl group.

In the present specification, the "aralkyl (arylalkyl) group" means a group in which at least one hydrogen atom of the "alkyl group" defined above is replaced with the "aryl group" defined above. The aralkyl group is preferably a $C_7$ to $C_{14}$ aralkyl group, more preferably a $C_7$ to $C_{10}$ aralkyl group. Examples of the aralkyl group specifically include a benzyl group, a phenethyl group, and a 3-phenylpropyl group.

In the present specification, the "aralkoxy group" means an oxy group bonded to the "aralkyl group" defined above. The aralkoxy group is preferably a $C_7$ to $C_{14}$ aralkoxy group, more preferably a $C_7$ to $C_{10}$ aralkoxy group. Examples of the aralkoxy group specifically include a benzyloxy group, a phenethyloxy group, and a 3-phenylpropoxy group.

In the present specification, the "aralkoxyalkyl group" means a group in which one or more hydrogen atoms of the "alkyl group" defined above are replaced with the "aralkoxy group" defined above. The aralkoxyalkyl group is preferably a $C_7$ to $C_{14}$ aralkoxy-$C_1$ to $C_6$ alkyl group, more preferably a $C_7$ to $C_{14}$ aralkoxy-$C_1$ or $C_2$ alkyl group. Examples of the aralkoxyalkyl group specifically include a benzyloxymethyl group and a 1-(benzyloxy)ethyl group.

In the present specification, the "heteroarylalkyl group" means a group in which at least one hydrogen atom of the "alkyl group" defined above is replaced with the "heteroaryl group" defined above. The heteroarylalkyl group is preferably a 5- to 10-membered heteroaryl-$C_1$ to $C_6$ alkyl group, more preferably a 5- to 10-membered heteroaryl-$C_1$ or $C_2$ alkyl group. Examples of the heteroarylalkyl group specifically include a 3-thienylmethyl group, a 4-thiazolylmethyl group, a 2-pyridylmethyl group, a 3-pyridylmethyl group, a 4-pyridylmethyl group, a 2-(2-pyridyl)ethyl group, a 2-(3-pyridyl)ethyl group, a 2-(4-pyridyl)ethyl group, a 2-(6-quinolyl)ethyl group, a 2-(7-quinolyl)ethyl group, a 2-(6-indolyl)ethyl group, a 2-(5-indolyl)ethyl group, and a 2-(5-benzofuranyl)ethyl group.

In the present specification, the "heteroarylalkoxy group" means an oxy group bonded to the "heteroarylalkyl group" defined above. The heteroarylalkoxy group is preferably a 5- to 10-membered heteroaryl-$C_1$ to $C_6$ alkoxy group, more preferably a 5- to 10-membered heteroaryl-$C_1$ or $C_2$ alkoxy group. Examples of the heteroarylalkoxy group specifically include a 3-thienylmethoxy group and a 3-pyridylmethoxy group.

In the present specification, the "heteroarylalkoxyalkyl group" means a group in which one or more hydrogen atoms of the "alkyl group" defined above are replaced with the "heteroarylalkoxy group" defined above. The heteroarylalkoxyalkyl group is preferably a 5- to 10-membered heteroaryl-$C_1$ to $C_6$ alkoxy-$C_1$ to $C_6$ alkyl group, more preferably a 5- to 10-membered heteroaryl-$C_1$ or $C_2$ alkoxy-$C_1$ or $C_2$ alkyl group. Examples of the heteroarylalkoxyalkyl group specifically include a 3-pyridylmethoxymethyl group.

In the present specification, the "heterocyclidenealkyl group" means a group in which one or more hydrogen atoms of the "alkyl group" defined above are replaced with the "heterocyclidene group" defined above. The heterocyclidenealkyl group is preferably a 4- to 7-membered heterocyclidene $C_1$ to $C_6$ alkyl group, more preferably a 4- to 7-membered heterocyclidene $C_1$ to $C_2$ alkyl group. Examples of the heterocyclidenealkyl group specifically include a tetrahydro-4H-pyran-4-ylidenemethyl and azetidin-3-ylidenemethyl group.

In the present specification, the "alkoxyalkenyl group" means a group in which one or more hydrogen atoms of the "alkenyl group" defined above are replaced with the "alkoxy group" defined above. The alkoxyalkenyl group is preferably a $C_1$ to $C_6$ alkoxy-$C_2$ to $C_6$ alkenyl group. Examples of the alkoxyalkenyl group specifically include a (E)-4-methoxybut-2-en-1-yl group.

In the present specification, the "aminocarbonylalkenyl group" means a group in which one or more hydrogen atoms of the "alkenyl group" defined above are replaced with the "aminocarbonyl group" defined above. The aminocarbonylalkenyl group is preferably an aminocarbonyl-$C_2$ to $C_6$ alkenyl group. Examples of the aminocarbonylalkenyl group specifically include a (E)-3-(dimethylaminocarbonylcarbonyl)-prop-2-en-1-yl group.

In the present specification, the "haloalkoxy group" means a group in which one or more hydrogen atoms of the "alkoxy group" defined above are replaced with a halogen atom. The haloalkoxy group is preferably a $C_1$ to $C_6$ haloalkoxy group. Examples of the haloalkoxy group specifically include a difluoromethoxy group, a trifluoromethoxy group, a 2,2-difluoroethoxy group, and a 2,2,2-trifluoroethoxy group.

In the present specification, the "alkylene group" means a divalent group induced by the further removal of one arbitrary hydrogen atom from the "alkyl group" described above. The alkylene group is preferably a $C_4$ to $C_8$ alkylene group. Examples of the alkylene group specifically include $-CH_2-$, $-(CH_2)_2-$, $-(CH_2)_3-$, $-CH(CH_3)CH_2-$, $-C(CH_3)_2-$, $-(CH_2)_4-$, $-CH(CH_3)CH_2CH_2-$, $-C(CH_3)_2CH_2-$, $-CH_2CH(CH_3)CH_2-$, $-CH_2C(CH_3)_2-$, $-CH_2CH_2CH(CH_3)-$, $-(CH_2)_5-$, $-(CH_2)_6-$, $-(CH_2)_7-$, and $-(CH_2)_8-$.

In the present specification, the "cycloalkylene group" means a divalent group induced by the further removal of one arbitrary hydrogen atom from the "cycloalkyl group" described above. The cycloalkylene group is preferably a $C_3$ to $C_8$ cycloalkylene group. Examples of the cycloalkylene group specifically include a cyclopropane-1,2-diyl group, a cyclobutane-1,2-diyl group, a cyclopentane-1,2-diyl group, and a cyclohexane-1,2-diyl group.

In the present specification, the "alkenylene group" means a divalent group induced by the further removal of one arbitrary hydrogen atom from the "alkenyl group" described above. Depending on the conformation of the double bond and a substituent (if present), the geometric morphology of the double bond can assume entgegen (E) or zusammen (Z) and cis or trans conformations. The alkenylene group includes a linear or branched form and is preferably a $C_2$ to $C_{10}$ alkenylene group, more preferably a $C_2$ to $C_6$ alkenylene group.

In the present specification, the "alkynylene group" means a divalent group induced by the further removal of one arbitrary hydrogen atom from the "alkynyl group" described above. The alkynylene group includes a linear or branched form and is preferably a $C_2$ to $C_{10}$ alkynylene group, more preferably a $C_2$ to $C_6$ alkynylene group.

In the present specification, the "arylene group" means a divalent group induced by the further removal of one arbitrary hydrogen atom from the "aryl group" described above. The arylene group may be a monocyclic ring or a condensed ring. The number of atoms constituting the ring is not particularly limited and is preferably 6 to 10 (C6 to 10 arylene). Examples of the arylene group specifically include a 1,2-phenylene group, a 1,3-phenylene group, a 1,4-phenylene group, a 1,2-naphthylene group, a 1,3-naphthylene group, and a 1,4-naphthylene group.

In the present specification, the "heterocyclylene group" means a divalent group induced by the further removal of one arbitrary hydrogen atom from the "heterocyclyl group" described above.

In the present specification, the "heteroarylene group" means a divalent group induced by the further removal of one arbitrary hydrogen atom from the "heteroaryl group" described above.

In the present specification, the "spirocycloalkyl group" means a group formed such that one carbon atom constituting a cycloalkane ring is shared by a carbon atom in a group to be bonded thereto. The spirocycloalkyl group is preferably a $C_3$ to $C_8$ spirocycloalkyl group. Examples thereof specifically include a spirocyclopropyl group, a spirocyclobutyl group, a spirocyclopentyl group, a spirocyclohexyl group, a spirocycloheptyl group, and a spirocyclooctyl group.

In the present specification, the "spiroheterocyclyl group" means a group in which one or more carbon atoms in the "spirocycloalkyl group" are replaced with a heteroatom. Preferred examples of the heterospirocycloalkyl group include a 4- to 10-membered spiroheterocyclyl group.

In the present specification, the "alicyclic ring" means a nonaromatic hydrocarbon ring. The alicyclic ring may have an unsaturated bond in the ring and may be a polycyclic ring having two or more rings. A carbon atom constituting the ring may form a carbonyl through oxidation. The alicyclic ring is preferably a 3- to 8-membered alicyclic ring. Examples thereof specifically include a cyclopropane ring, a cyclobutane ring, a cyclopentane ring, a cyclohexane ring, a cycloheptane ring, a cyclooctane ring, and a bicyclo[2.2.1]heptane ring.

In the present specification, the "heterocyclic ring" means a nonaromatic heterocyclic ring containing preferably 1 to 5, more preferably 1 to 3 heteroatoms among the atoms constituting the ring. The heterocyclic ring may have a double and/or triple bond in the ring. A carbon atom in the ring may form carbonyl through oxidation, and the ring may be a monocyclic ring, a condensed ring, or a spiro ring. The number of atoms constituting the ring is preferably 3 to 12 (3- to 12-membered heterocyclic ring), more preferably 4 to 8 (4- to 8-membered heterocyclic ring). Examples of the heterocyclic ring specifically include an azetidine ring, an oxetane ring, a tetrahydrofuran ring, a tetrahydropyran ring, a morpholine ring, a thiomorpholine ring, a pyrrolidine ring, a 4-oxopyrrolidine ring, a piperidine ring, a 4-oxopiperidine ring, a piperazine ring, a pyrazolidine ring, an imidazolidine ring, an oxazolidine ring, an isoxazolidine ring, a thiazolidine ring, an isothiazolidine ring, a thiadiazolidine ring, an oxazolidone ring, a dioxolane ring, a dioxane ring, a thietane ring, an octahydroindole ring, and an azocane ring, and rings in which one or more single bonds in any of these saturated heterocyclic rings are replaced with a double bond or a triple bond.

In the present specification, the "saturated heterocyclic ring" means a nonaromatic heterocyclic ring containing a carbon atom as well as 1 to 5 heteroatoms without containing a double bond and/or a triple bond in the ring. The saturated heterocyclic ring may be a monocyclic ring or may form a condensed ring with another ring, for example, an aromatic ring such as a benzene ring. When the saturated heterocyclic ring forms a condensed ring, the saturated heterocyclic ring is preferably a 4- to 7-membered saturated heterocyclic ring. Examples thereof specifically include an azetidine ring, an oxetane ring, a tetrahydrofuran ring, a tetrahydropyran ring, a morpholine ring, a thiomorpholine ring, a pyrrolidine ring, a 4-oxopyrrolidine ring, a piperidine ring, a 4-oxopiperidine ring, a piperazine ring, a pyrazolidine ring, an imidazolidine ring, an oxazolidine ring, an isoxazolidine ring, a thiazolidine ring, an isothiazolidine ring, a thiadiazolidine ring, an oxazolidone ring, a dioxolane ring, a dioxane ring, a thietane ring, an octahydroindole ring, an indoline ring, and an azepane ring.

[Peptide Compound]

In the present specification, the "peptide compound" is not particularly limited as long as it is a peptide compound in which amino acid residues are linked by an amide bond or an ester bond. The number of amino acid residues of the peptide compound may be, without particular limitation, 5 or more, 7 or more, 8 or more, or 9 or more. The number of amino acid residues of the peptide compound may also be 30 or less, 25 or less, 15 or less, or 13 or less. The number of amino acid residues in the peptide compound may be, for example, 5 or more and 30 or less, 7 or more and 25 or less, 8 or more and 15 or less, and 9 or more and 13 or less, and may be 11. The peptide compound may have a branched structure.

In the present specification, the "amino acid" includes a natural amino acid and a non-natural amino acid. In the present specification, the "natural amino acid" refers to Gly, Ala, Ser, Thr, Val, Leu, Ile, Phe, Tyr, Trp, His, Glu, Asp, Gln, Asn, Cys, Met, Lys, Arg, or Pro. Examples of the non-natural amino acid include, but are not particularly limited to, $\beta$-amino acids, $\gamma$-amino acids, D-amino acids, N-substituted amino acids, $\alpha,\alpha$-disubstituted amino acids, and amino acids having a side chain different from the natural one. In the present specification, the non-natural N-substituted amino acid means a N-substituted amino acid other than Pro. In the present specification, the amino acid accepts an arbitrary conformation. The side chain of the amino acid can be selected without particular limitations and is freely selected from a hydrogen atom as well as, for example, an alkyl group, an alkenyl group, an alkynyl group, an aryl group, a heteroaryl group, an aralkyl group, and a cycloalkyl group. One or two non-adjacent methylene groups in any of these groups may be substituted by an oxygen atom, a carbonyl group (-CO-), or a sulfonyl group ($-SO_2-$). A substituent may be added to each of the groups. Such a substituent is not limited and can be one or two or more substituents each independently freely selected from arbitrary substituents including a halogen atom, an O atom, a S atom, a N atom, a B atom, a Si atom, and a P atom. Specifically, examples thereof include an optionally substituted alkyl group, alkenyl group, alkynyl group, aryl group, heteroaryl group, aralkyl group, and cycloalkyl group. In a non-limiting aspect, the amino acid in the present specification may be a compound having a carboxy group and an amino group in the same molecule (even in this case, the amino acid also includes imino acids such as proline and hydroxyproline).

The backbone amino group of the amino acid may be unsubstituted ($NH_2$ group) or may be substituted (i.e., a -NHR group wherein R represents an alkyl group, an alkenyl group, an alkynyl group, an aryl group, a heteroaryl group, an aralkyl group, or a cycloalkyl group optionally having a substituent, and one or two non-adjacent methylene groups in any of these groups may be substituted by an oxygen atom, a carbonyl group (-CO-), or a sulfonyl group ($-SO_2-$); and a carbon chain bonded to a N atom and a carbon atom at position $\alpha$ may form a ring, as in proline). The substituent of R is selected in the same manner as in the substituent for the amino acid side chain mentioned above. In the case of a substituted backbone amino group, the R is contained in the "side chain of the amino acid" in the present specification. In the present specification, such an amino acid having the substituted backbone amino group is referred to as the "N-substituted amino acid". In the present specification, examples of the "N-substituted amino acid" preferably include, but are not limited to, N-alkyl amino acids, N-$C_1$ to $C_6$ alkyl amino acids, N-$C_1$ to $C_4$ alkyl amino acids, N-methyl amino acids, and N-ethyl amino acids.

In the present specification, the "amino acid" includes all isotopes corresponding to each amino acid. The isotope of the "amino acid" is a form in which at least one atom is replaced with an atom having the same atomic number (proton number) therewith and a different mass number (total number of protons and neutrons) therefrom at an abundance ratio different from the natural abundance ratio. In the present specification, examples of the isotope included in the "amino acid" include a hydrogen atom, a carbon atom, a nitrogen atom, an oxygen atom, a phosphorus atom, a sulfur atom, a fluorine atom, and a chlorine atom which respectively include $^{2}H$, $^{3}H$, $^{13}C$, $^{14}C$, $^{15}N$, $^{17}O$, $^{18}O$, $^{31}P$, $^{32}P$, $^{35}S$, $^{18}F$, and $^{36}Cl$.

In the present specification, examples of the substituent containing a halogen atom include an alkyl group, a cycloalkyl group, an alkenyl group, an alkynyl group, an aryl group, a heteroaryl group, and an aralkyl group having halogen atom as a substituent, and more specifically include fluoroalkyl, difluoroalkyl, and trifluoroalkyl.

In the present specification, examples of the substituent containing an oxygen atom include groups such as a hydroxy group (-OH), an oxy group (-OR), a carbonyl group (-C(=O)-R), a carboxy group ($-CO_2H$), an oxycarbonyl group (-C(=O)-OR), a carbonyloxy group (-O-C(=O)-R), a thiocarbonyl group (-C(=O)-SR), a carbonylthio group (-S-C(=O)-R), an aminocarbonyl group (-C(=O)-NHR), a carbonylamino group (-NH-C(=O)-R), an oxycarbonylamino group (-NH-C(=O)-OR), a sulfonylamino group ($-NH-SO_2-R$), an aminosulfonyl group ($-SO_2-NHR$), a sulfamoylamino group ($-NH-SO_2-NHR$), a thiocarboxy group (-C(=O)-SH), and a carboxycarbonyl group ($-C(=O)-CO_2H$).

Examples of the oxy (-OR) include an alkoxy group, a cycloalkoxy group, an alkenyloxy group, an alkynyloxy group, an aryloxy group, a heteroaryloxy group, and an aralkyloxy group. The alkoxy group is preferably a $C_1$ to $C_4$ alkoxy group or a $C_1$ or $C_2$ alkoxy group, particularly preferably a methoxy group or an ethoxy group.

Examples of the carbonyl group (-C(=O)-R) include a formyl group (-C(=O)-H), an alkylcarbonyl group, a cycloalkylcarbonyl group, an alkenylcarbonyl group, an alkynylcarbonyl group, an arylcarbonyl group, a heteroarylcarbonyl group, and an aralkylcarbonyl group.

Examples of the oxycarbonyl group (-C(=O)-OR) include an alkyloxycarbonyl group, a cycloalkyloxycarbonyl group, an alkenyloxycarbonyl group, an alkynyloxycarbonyl group, an aryloxycarbonyl group, a heteroaryloxycarbonyl group, and an aralkyloxycarbonyl group.

Examples of the carbonyloxy group (-O-C(=O)-R) include an alkylcarbonyloxy group, a cycloalkylcarbonyloxy group, an alkenylcarbonyloxy group, an alkynylcarbonyloxy group, an arylcarbonyloxy group, a heteroarylcarbonyloxy group, and an aralkylcarbonyloxy group.

Examples of the thiocarbonyl group (-C(=O)-SR) include an alkylthiocarbonyl group, a cycloalkylthiocarbonyl group, an alkenylthiocarbonyl group, an alkynylthiocarbonyl group, an arylthiocarbonyl group, a heteroarylthiocarbonyl group, and an aralkylthiocarbonyl group.

Examples of the carbonylthio group (-S-C(=O)-R) include an alkylcarbonylthio group, a cycloalkylcarbonylthio group, an alkenylcarbonylthio group, an alkynylcarbonylthio group, an arylcarbonylthio group, a heteroarylcarbonylthio group, and an aralkylcarbonylthio group.

Examples of the aminocarbonyl group (-C(=O)-NHR) include an alkylaminocarbonyl group (e.g., a $C_1$ to $C_6$ or $C_1$ to $C_4$ alkylaminocarbonyl group, particularly, an ethylaminocarbonyl group and a methylaminocarbonyl group), a cycloalkylaminocarbonyl group, an alkenylaminocarbonyl group, an alkynylaminocarbonyl group, an arylaminocarbonyl

group, a heteroarylaminocarbonyl group, and an aralkylaminocarbonyl group. Examples thereof additionally include compounds in which the H atom bonded to the N atom in -C(=O)-NHR is further replaced with an alkyl group, a cycloalkyl group, an alkenyl group, an alkynyl group, an aryl group, a heteroaryl group, or an aralkyl group.

Examples of the carbonylamino group (-NH-C(=O)-R) include an alkylcarbonylamino group, a cycloalkylcarbonylamino group, an alkenylcarbonylamino group, an alkynylcarbonylamino group, an arylcarbonylamino group, a heteroarylcarbonylamino group, and an aralkylcarbonylamino group. Examples thereof additionally include compounds in which the H atom bonded to the N atom in -NH-C(=O)-R is further replaced with an alkyl group, a cycloalkyl group, an alkenyl group, an alkynyl group, an aryl group, a heteroaryl group, or an aralkyl group.

Examples of the oxycarbonylamino group (-NH-C(=O)-OR) include an alkoxycarbonylamino group, a cycloalkoxycarbonylamino group, an alkenyloxycarbonylamino group, an alkynyloxycarbonylamino group, an aryloxycarbonylamino group, a heteroaryloxycarbonylamino group, and an aralkyloxycarbonylamino group. Examples thereof additionally include compounds in which the H atom bonded to the N atom in -NH-C(=O)-OR is further replaced with an alkyl group, a cycloalkyl group, an alkenyl group, an alkynyl group, an aryl group, a heteroaryl group, or an aralkyl group.

Examples of the sulfonylamino group (-NH-$SO_2$-R) include an alkylsulfonylamino group, a cycloalkylsulfonylamino group, an alkenylsulfonylamino group, an alkynylsulfonylamino group, an arylsulfonylamino group, a heteroarylsulfonylamino group, and an aralkylsulfonylamino group. Examples thereof additionally include compounds in which the H atom bonded to the N atom in -NH-$SO_2$-R is further replaced with an alkyl group, a cycloalkyl group, an alkenyl group, an alkynyl group, an aryl group, a heteroaryl group, or an aralkyl group.

Examples of the aminosulfonyl group (-$SO_2$-NHR) include an alkylaminosulfonyl group, a cycloalkylaminosulfonyl group, an alkenylaminosulfonyl group, an alkynylaminosulfonyl group, an arylaminosulfonyl group, a heteroarylaminosulfonyl group, and an aralkylaminosulfonyl group. Examples thereof additionally include compounds in which the H atom bonded to the N atom in -$SO_2$-NHR is further replaced with an alkyl group, a cycloalkyl group, an alkenyl group, an alkynyl group, an aryl group, a heteroaryl group, or an aralkyl group.

Examples of the sulfamoylamino group (-NH-$SO_2$-NHR) include an alkylsulfamoylamino group, a cycloalkylsulfamoylamino group, an alkenylsulfamoylamino group, an alkynylsulfamoylamino group, an arylsulfamoylamino group, a heteroarylsulfamoylamino group, and an aralkylsulfamoylamino group. The two H atoms bonded to the N atoms in -NH-$SO_2$-NHR may be substituted by substituents each independently selected from the group consisting of an alkyl group, a cycloalkyl group, an alkenyl group, an alkynyl group, an aryl group, a heteroaryl group, and an aralkyl group, and these two substituents may form a ring.

Examples of the substituent containing a S atom include groups such as a thiol group (-SH), a thio group (-S-R), a sulfinyl group (-S(=O)-R), a sulfonyl group (-$SO_2$-R), and a sulfo group (-$SO_3$H).

Examples of the thio group (-S-R) that can be selected include an alkylthio group, a cycloalkylthio group, an alkenylthio group, an alkynylthio group, an arylthio group, a heteroarylthio group, and an aralkylthio group.

Examples of the sulfonyl group (-$SO_2$-R) include an alkylsulfonyl group, a cycloalkylsulfonyl group, an alkenylsulfonyl group, an alkynylsulfonyl group, an arylsulfonyl group, a heteroarylsulfonyl group, and an aralkylsulfonyl group.

In the present specification, examples of the substituent containing a nitrogen atom include groups such as an azide group (-$N_3$), a cyano group (-CN), a primary amino group (-$NH_2$), a secondary amino group (-NH-R; also referred to as mono-substituted amino group), a tertiary amino group (-NR(R'); also referred to as a di-substituted amino group), an amidino group (-C(=NH)-$NH_2$), a substituted amidino group (-C(=NR)-NR'R"), a guanidino group (-NH-C(=NH)-$NH_2$), a substituted guanidino group (-NR-C(=NR‴)-NR'R"), an aminocarbonylamino group (-NR-CO-NR'R"), a pyridyl group, a piperidino group, a morpholino group, and an azetidinyl group.

Examples of the secondary amino group (-NH-R: mono-substituted amino group) include an alkylamino group, a cycloalkylamino group, an alkenylamino group, an alkynylamino group, an arylamino group, a heteroarylamino group, and an aralkylamino group. The alkylamino group is preferably a $C_1$ to $C_6$ alkyl group, more preferably a $C_1$ to $C_4$ alkyl group, even more preferably a methyl group and an ethyl group.

Examples of the tertiary amino group (-NR(R'): di-substituted amino group) include an amino group having arbitrary two substituents each independently selected from an alkyl group, a cycloalkyl group, an alkenyl group, an alkynyl group, an aryl group, a heteroaryl group, and an aralkyl group, for example, an alkyl(aralkyl)amino group. These arbitrary two substituents together with the nitrogen atoms to which they are attached may form a ring. Examples thereof specifically include a dialkylamino group, particularly, a $C_1$-$C_6$ dialkylamino group, a $C_1$-$C_4$ dialkylamino group, a dimethylamino group, and a diethylamino group. In the present specification, the "$C_p$-$C_q$ dialkylamino group" refers to a group in which an amino group is substituted by two $C_p$-$C_q$ alkyl groups. The $C_p$-$C_q$ alkyl groups may be the same or different.

Examples of the substituted amidino group (-C(=NR)-NR'R") include groups in which three substituents R, R', and R" on the N atoms are each independently selected from an alkyl group, a cycloalkyl group, an alkenyl group, an alkynyl group, an aryl group, a heteroaryl group, and an aralkyl group, for example, an alkyl(aralkyl)(aryl)amidino group.

Examples of the substituted guanidino group (-NR-C(=NR‴)-NR'R") include groups in which R, R', R", and R‴ are each independently selected from an alkyl group, a cycloalkyl group, an alkenyl group, an alkynyl group, an aryl group, a heteroaryl group, and an aralkyl group, and groups in which these substituents form a ring.

Examples of the aminocarbonylamino group (-NR-CO-NR'R") include groups in which R, R', and R" are each independently selected from a hydrogen atom, an alkyl group, a cycloalkyl group, an alkenyl group, an alkynyl group, an aryl group, a heteroaryl group, and an aralkyl group, and groups in which these substituents form a ring.

In the present specification, the "peptide residue" and the "amino acid residue" are also simply referred to as a "peptide" and an "amino acid", respectively.

In the present specification, the phrase "site corresponding to" may be used for characterizing an amino acid residue in the amino acid sequence of the peptide according to the present embodiment with reference to the amino acid sequence represented by SEQ ID NO: 1. The alignment for determining the corresponding site can be achieved by use of various methods within the scope of techniques in the technical field, for example, publicly available computer software such as BLAST, BLAST-2, ALIGN, Megalign (DNASTAR) software, or GENETYX(R) (Genetyx Corp.). Those skilled in the art can determine appropriate parameters for adopting sequence alignment, including an arbitrary algorithm necessary for achieving the maximum alignment over the full lengths of sequences to be compared.

The salt of the compound used herein may be preferably a chemically or pharmaceutically acceptable salt. The salt may also be a solvate thereof, preferably a chemically or pharmaceutically acceptable solvate thereof. Examples of the salt of the compound include: hydrochloride; hydrobromide; hydroiodide; phosphate; phosphonate; sulfate; sulfonate such as methanesulfonate and p-toluenesulfonate; carboxylate such as acetate, citrate, malate, tartrate, succinate, and salicylate; alkali metal salts such as lithium salt, sodium salt, and potassium salt; alkaline earth metal salts such as magnesium salt and calcium salt; and ammonium salts such as ammonium salt, alkylammonium salt, dialkylammonium salt, trialkylammonium salt, and tetraalkylammonium salt.

In the present specification, the solvate refers to those in which a compound forms a molecular population with a solvent, and when the solvent is water, it is referred to as a hydrate. The solvate of the salt of the compound of the present disclosure is preferably a hydrate, and specific examples of the hydrate include mono- to deca-hydrates, preferably mono- to penta-hydrates, and still more preferably mono- to tri-hydrates. The solvate of the compound of the present disclosure include not only a solvate with a single solvent such as water, an alcohol (e.g., methanol, ethanol, 1-propanol, 2-propanol, or the like), dimethylformamide, but also a solvate with a plurality of solvents.

[Production method of amino acid]

The method for producing an amino acid according to one embodiment comprises a step (reaction step) of performing the following reaction (i) or (ii) in the presence of a polypeptide comprising an amino acid sequence having 90% or higher identity to an amino acid sequence represented by SEQ ID NO: 1, a reducing agent, and compound D represented by the following formula (1):

(i) an intermolecular reductive amination reaction between compound A selected from the group consisting of a compound having an amino group and a salt thereof and compound B selected from the group consisting of a compound having a carbonyl group and a salt thereof;
(ii) an intramolecular reductive amination reaction of compound C selected from the group consisting of a compound having an amino group and a carbonyl group and a salt thereof.

Here, an amino acid having a substituted or unsubstituted amino group is formed by an intermolecular reductive amination reaction between an amino group of compound A and a carbonyl group of compound B, or by an intramolecular reductive amination reaction between an amino group and an carbonyl group of compound C.

The method for producing an amino acid according to another embodiment comprises a step (reaction step) of performing the following reaction (i) or (ii) in the presence of a polypeptide having catalytic activity for an intermolecular reductive amination reaction between compound A selected from the group consisting of a compound having an amino group and a salt thereof and compound B selected from the group consisting of a compound having a carbonyl group and a salt thereof, or an intramolecular reductive amination reaction of compound C selected from the group consisting of a compound having an amino group and a carbonyl group and a salt thereof under at least one reaction condition, a reducing agent, and compound D':

(i) an intermolecular reductive amination reaction between compound A selected from the group consisting of a compound having an amino group and a salt thereof and compound B selected from the group consisting of a compound having a carbonyl group and a salt thereof;
(ii) an intramolecular reductive amination reaction of compound C selected from the group consisting of a compound having an amino group and a carbonyl group and a salt thereof.

In the production method, the compound D' is at least one compound selected from the group consisting of dimethyl sulfoxide, dimethylsulfone, trimethylphosphine oxide, dimethoxymethane, N,N-dimethylformamide, N,N-dimethylacetamide, tetramethylene sulfoxide, diethyl sulfoxide, methanol, and methylformamide.

<Compound D and compound D'>

The reaction step of performing a reductive amination reaction is performed in the presence of compound D represented by the following formula (1):

[Formula 10]

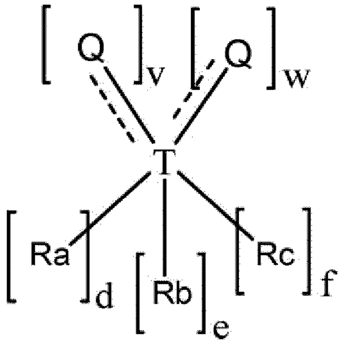

(1)

In the formula (1), v and w each independently represent 0 or 1, at least one of v and w represents 1, and T represents a carbon atom, a phosphorus atom, or a sulfur atom.

In the formula (1), the functional group represented by the following formula (1a):

[Formula 11]

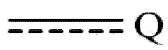

(1a)

represents =O, -ORd, or a hydroxy group (-OH). In this context, =O which is the functional group represented by the formula (1a) means that T is bonded to the oxygen atom through a double bond. Specifically, the compound D wherein the functional group represented by the formula (1a) is =O has a structure represented by T=O. -ORd which is the functional group represented by the formula (1a) means that T is bonded to ORd through a single bond. Specifically, the compound D wherein the functional group represented by the formula (1a) is - ORd has a structure represented by T-ORd. The hydroxy group which is the functional group represented by the formula (1a) means that T is bonded directly to a hydroxy group. Specifically, the compound D wherein the functional group represented by the formula (1a) is - OH has a structure represented by T-OH.

When each of v and w is 1, two functional groups represented by a plurality of formulas (1a) may be the same or different; Ra, Rb, and Rc each independently represent a hydrogen atom, a $C_1$ to $C_3$ alkyl group, an alkylamino group, or $-CH_2$-ORd; at least any two of Ra, Rb, and Rc are optionally linked to each other to form a ring structure together with T; Rd represents a $C_1$ to $C_3$ alkyl group; d, e, and f each independently represent 0 or 1; and at least one of d, e, and f represents 1.

When each of v and w is 1, at least one of Ra, Rb, and Rc is a methyl group, and none of Ra, Rb, and Rc are linked to each other to form a ring structure together with T. For example, when each of v and w is 1, T is a sulfur atom, each functional group represented by the formula (1a) is =O, f is 0, and at least one of Ra and Rb may be a methyl group.

When at least one of Ra, Rb, and Rc is a methylamino group, none of Ra, Rb, and Rc are linked to each other to form a ring structure together with T. When the formula (1a) is a hydroxy group and T is a carbon atom, each of d, e, and f is 1, and each of Ra, Rb, and Rc is a hydrogen atom.

The compound D may be a compound represented by the following formula (1-1) wherein T in the formula (1) is a carbon atom:

[Formula 12]

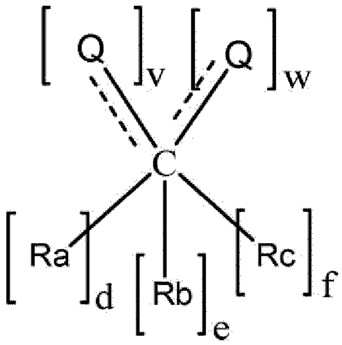

(1 − 1)

The compound represented by the formula (1-1) may be a compound represented by the following formula (1-1a) wherein v is 1, and w is 0:

[Formula 13]

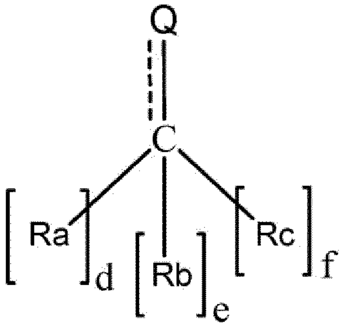

(1 − 1 a)

In the formula (1-la), when the functional group represented by the formula (1a) is =O, two of d, e, and f are 1 and the remaining one is 0. When the functional group represented by the formula (1a) is =O, the compound represented by the formula (1-la) may be a compound represented by the following formula (1-1b) wherein each of d and e is 1, and f is 0:

[Formula 14]

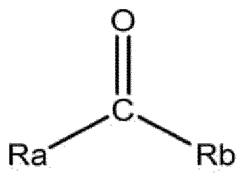

(1 − 1 b)

The compound represented by the formula (1-1b) may be a compound wherein one of Ra and Rb is a hydrogen atom or a $C_1$ to $C_3$ alkyl group, and the other moiety is an alkylamino group. The alkylamino group may be a monomethylamino group or a dimethylamino group. The compound represented by the formula (1-1b) may be, for example, N,N-dimethylformamide, N,N-dimethylacetamide, or N-methylformamide.

In the formula (1-la), when the functional group represented by the formula (1a) is a hydroxy group, each of d, e, and f is 1. When the functional group represented by the formula (1a) is a hydroxy group, the compound represented by the formula (1-1a) may be a compound represented by the following formula (1-1c) wherein each of d, e, and f is 1:

[Formula 15]

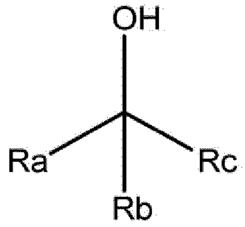

(1-1c)

Ra, Rb, and Rc may each independently be a hydrogen atom or a $C_1$ to $C_3$ alkyl group. The compound represented by the formula (1-1c) may be methanol wherein each of Ra, Rb, and Rc is a hydrogen atom.

In the formula (1-1a), when the functional group represented by the formula (1a) is -ORd, each of d, e, and f may be 1 and each of Rb and Rc may be a hydrogen atom. When the functional group represented by the formula (1a) is -ORd, the compound represented by the formula (1-1a) may be a compound represented by the following formula (1-1d) wherein each of d, e, and f is 1, and each of Rb and Rc is a hydrogen atom. In the compound represented by the following formula (1-1d), Ra may be $-CH_2-ORd$. The compound represented by the following formula (1-1d) may be dimethoxyethane wherein Ra is $-CH_2-ORd$, and Rd is $-CH_3$:

[Formula 16]

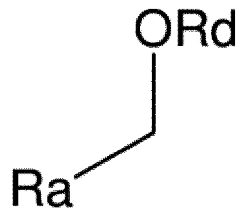

(1-1d)

The compound D may be a compound represented by the following formula (1-2) wherein T in the formula (1) is a phosphorus atom:

[Formula 17]

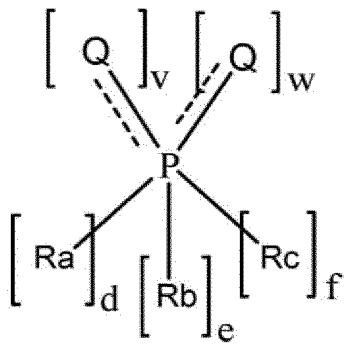

(1-2)

The compound represented by the formula (1-2) may be a compound represented by the following formula (1-2a) wherein v is 1, w is 0, and the functional group represented by the formula (1a) is =O:

[Formula 18]

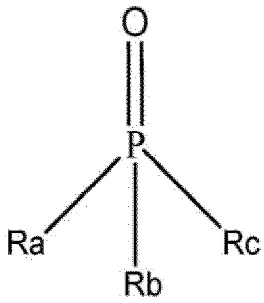

(1-2a)

In the formula (1-2a), each of Ra, Rb, and Rc may be a $C_1$ to $C_3$ alkyl group. The compound represented by the formula (1-2a) may be trimethylphosphine oxide wherein each of Ra, Rb, and Rc is a methyl group.

The compound D may be a compound represented by the following formula (1-3) wherein T in the formula (1) is a sulfur atom:

[Formula 19]

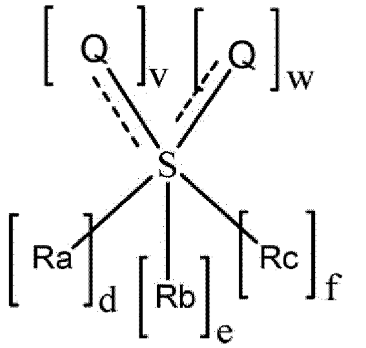

(1-3)

The compound represented by the formula (1-3) may be a compound represented by the following formula (1-3a) wherein v is 1, w is 0, the functional group represented by the formula (1a) is =O, each of d and e is 1, and f is 0:

[Formula 20]

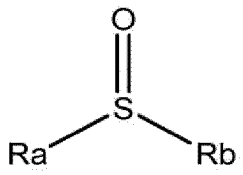

(1-3a)

In the formula (1-3a), each of Ra and Rb may be a $C_1$ to $C_3$ alkyl group, and Ra and Rb may be linked to each other to form a ring structure together with the sulfur atom (S).

In the compound represented by the formula (1-3a), Ra and Rb may each independently be a methyl group or an ethyl group. The compound represented by the formula (1-3a) may be dimethyl sulfoxide wherein each of Ra and Rb is a methyl group, or diethyl sulfoxide wherein each of Ra and Rb is an ethyl group. The compound represented by the formula (1-3a) may be tetramethylene sulfoxide wherein each of Ra and Rb is a $C_1$ to $C_3$ alkyl group, and these alkyl groups are linked to each other to form a ring structure together with S.

The compound represented by the formula (1-3) may be a compound represented by the following formula (1-3b) wherein each of v and w is 1, the functional group represented by the formula (1a) is =O, each of d and e is 1, and f is 0:

[Formula 21]

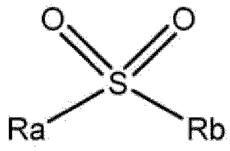

(1-3b)

In the compound represented by the formula (1-3b), at least one of Ra and Rb is a methyl group. The compound represented by the formula (1-3b) may be dimethylsulfone wherein each of Ra and Rb is a methyl group.

The compound D may be compound D' that is at least one compound selected from the group consisting of dimethyl sulfoxide, dimethylsulfone, trimethylphosphine oxide, dimethoxyethane, N,N-dimethylformamide, N,N-dimethylacetamide, tetramethylene sulfoxide, diethyl sulfoxide, methanol, and methylformamide, because the amino acid can be produced with a better yield. The compound D or compound D' may be at least one selected from the group consisting of dimethyl sulfoxide, dimethylsulfone, and trimethylphosphine oxide, and may be dimethyl sulfoxide, because the amino acid can be produced with a better yield.

The compound D or compound D' may be a liquid or a solid under conditions of 25°C and 1 atm, and may be a liquid under conditions of 25°C and 1 atm because the production efficiency of the amino acid is more improved. Examples of the compound D or compound D' which is a liquid under conditions of 25°C and 1 atm include dimethyl sulfoxide, dimethoxyethane, N,N-dimethylformamide, N,N-dimethylacetamide, methanol, and N-methylformamide.

<Compound A>

Compound A selected from the group consisting of a compound having an amino group and a salt thereof may be one or more selected from the group consisting of a compound represented by the following formula (2) and a salt thereof:

[Formula 22]

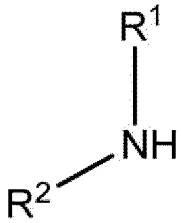

(2)

In the formula (2), $R^1$ and $R^2$ each independently represent a hydrogen atom, an alkyl group, an alkenyl group, an alkynyl group, a cycloalkyl group, an aryl group, a heterocyclyl group, or a heteroaryl group, these groups are optionally substituted, and at least one of $R^1$ and $R^2$ is a hydrogen atom.

In the formula (2), $R^1$ may be a hydrogen atom, $R^2$ may be a hydrogen atom or a $C_1$ to $C_6$ alkyl group, may be a hydrogen atom or a $C_1$ to $C_3$ alkyl group, or may be a hydrogen atom, a methyl group, or an ethyl group.

The compound A may be alkylamine of the formula (2) wherein one of $R^1$ and $R^2$ is an alkyl group, and the other moiety is a hydrogen atom. The alkylamine or a salt thereof may be one or more selected from the group consisting of methylamine, ethylamine and salts thereof. Compound A may be ammonia, wherein each of $R^1$ and $R^2$ in the formula (2) is a hydrogen atom. The compound A may be one or more selected from the group consisting of ammonia, methylamine, ethylamine and salts thereof.

<Compound B>

Compound B selected from the group consisting of a compound having a carbonyl group and a salt thereof may be one or more selected from the group consisting of a compound represented by the following formula (3) and a salt thereof:

[Formula 23]

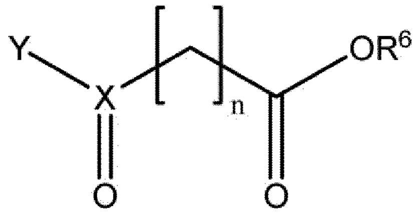

(3)

[Formula 24]

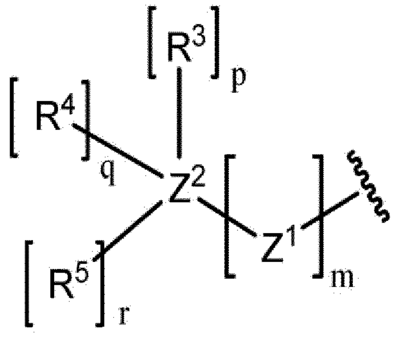

(4)

In the formula (3), X represents a carbon atom, and Y represents a hydrogen atom, or a group represented by the formula (4).

n represents an integer of between 0 and 2. n may be 0 or 1 and may be 0.

$R^6$ represents a hydrogen atom, an optionally substituted $C_1$ to $C_6$ alkyl group, an optionally substituted $C_5$ to $C_{12}$ aryl group, an optionally substituted heteroaryl group having 5 or more and 12 or less ring-constituting atoms, a group containing a nitrogen atom, or a group containing an oxygen atom.

In the formula (4),

[Formula 25]

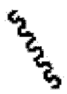

represents a binding point to X.

m represents an integer of between 0 and 6. The lower limit of m is 0 or larger and may be 1 or larger, 2 or larger, 3 or larger, 4 or larger, or 5 or larger. The upper limit of m is 6 or smaller and may be 5 or smaller, 4 or smaller, 3 or smaller, 2 or smaller, or 1 or smaller.

p represents 0 or 1, q represents 0 or 1, and r represents 0 or 1.

$Z^1$ represents an optionally substituted alkylene group, or an ether bond-containing group having 1 or more and 6 or less carbon atoms. When m is an integer of 2 or more, a plurality of $Z^1$ are the same or different. Examples of the "ether bond-containing group" include an alkyl group substituted by an alkoxy group, an aryloxy group, or the like. Specific examples of the ether bond-containing group having between 1 and 6 carbon atoms include a methoxymethyl group, an ethoxymethyl group, an allyloxymethyl group, an allyloxyethyl group, and an allyloxypropyl group.

$Z^2$ represents a carbon atom.

$R^3$, $R^4$, and $R^5$ each independently represent a hydrogen atom, an optionally substituted $C_1$ to $C_6$ alkyl group, an optionally substituted $C_5$ to $C_{12}$ aryl group, an optionally substituted heteroaryl group having 5 or more and 12 or less ring-constituting atoms, a group containing a nitrogen atom, or a group containing an oxygen atom.

Any two or more of $R^3$, $R^4$, and $R^5$ are optionally linked to each other to form a ring structure together with $Z^2$, the ring structure is optionally a cycloalkyl group, an aryl group, a heterocyclyl group, or a heteroaryl group, and these groups are optionally substituted.

$R^3$, $R^4$, and $R^5$ each optionally form a double bond or a triple bond with $Z^2$, and when any one of $R^3$, $R^4$, and $R^5$ is bonded to $Z^2$ through a double bond or a triple bond, at least one of p, q and r is 0.

In the formula (4), at least one of $R^3$, $R^4$ and $R^5$ may not be methyl.

The compound B may be a compound represented by the following formula (3'):

[Formula 26]

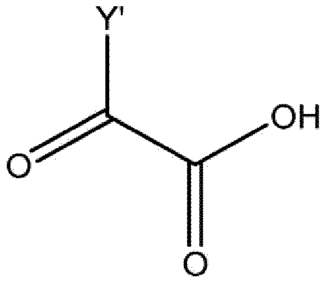

(3')

In the formula (3'), Y' represents a $C_3$ to $C_8$ cycloalkyl group or a $C_7$ to $C_{10}$ aralkyl group, and the aralkyl group is optionally substituted by a $C_1$ to $C_3$ alkyl group or halogen.

The compound represented by the formula (3') or a salt thereof may be one or more selected from the group consisting of 4-(2-chlorophenyl)-2-oxobutanoic acid, phenylpyruvic acid, 2-cyclopentyl-2-oxo-acetic acid, 3-(4,4-difluorocyclohexyl)-2-oxopropanoic acid, 2-oxo-3-phenylbutanoic acid, 3,3-dimethyl-2-oxobutyric acid, and salts thereof. Specific examples of the salt include a sodium salt, a magnesium salt, a potassium salt, and a calcium salt.

The combination of the compound A and the compound B may be a combination of the compound A (ammonia) of the formula (2) wherein each of $R^1$ and $R^2$ is a hydrogen atom, or the compound A (methylamine) of the formula (2) wherein $R^1$ is a hydrogen atom and $R^2$ is a methyl group, and the compound B (4-(2-chlorophenyl)-2-oxobutanoic acid) of the formula (3) wherein each of n and m is 0, Y is a group represented by the formula (4), $R^3$ is a (2-chlorophenyl)methyl group, each of $R^4$, $R^5$ and $R^6$ is a hydrogen atom, and each of p, q and r is 1, or the compound B (phenylpyruvic acid) of the formula (3) wherein each of n and m is 0, Y is a group represented by the formula (4), $R^3$ is a phenyl group, each of $R^4$, $R^5$ and $R^6$ is a hydrogen atom, and each of p, q and r is 1; or may be a combination of the compound A (ethylamine) of the formula (2) wherein $R^1$ is a hydrogen atom and $R^2$ is an ethyl group, and the compound B (phenylpyruvic acid) of the formula (3) wherein each of n and m is 0, Y is a group represented by the formula (4), $R^3$ is a phenyl group, each of $R^4$, $R^5$ and $R^6$ is a hydrogen atom, and each of p, q and r is 1; or may be a combination of the compound A (methylamine) of the formula (2) wherein $R^1$ is a hydrogen atom and $R^2$ is a methyl group, and the compound B (2-cyclopentyl-2-oxo-acetic acid) of the formula (3) wherein each of n and m is 0, Y is a group represented by the formula (4), $R^3$, $R^4$, and $Z^2$ are linked to each other to form a cyclopentane ring, each of $R^5$ and $R^6$ is a hydrogen atom, and each of p, q and r is 1.

The combination of the compound A and the compound B may be a combination of the compound A (methylamine) of the formula (2) wherein $R^1$ is a hydrogen atom and $R^2$ is a methyl group, and the compound B (3-(4,4-difluorocyclohexyl)-2-oxopropanoic acid) of the formula (3) wherein each of n and m is 0, Y is a group represented by the formula (4), $R^3$ is a 4,4-difluorocyclohexyl group, each of $R^4$, $R^5$ and $R^6$ is a hydrogen atom, and each of p, q and r is 1.

The combination of the compound A and the compound B may be a combination of the compound A (ammonia) of the formula (2) wherein each of $R^1$ and $R^2$ is a hydrogen atom, and the compound B (2-oxo-3-phenylbutanoic acid) of the formula (3) wherein each of n and m is 0, Y is a group represented by the formula (4), $R^3$ is a methyl group, $R^4$ is a phenyl group, each of $R^5$ and $R^6$ is a hydrogen atom, and each of p, q and r is 1.

The combination of the compound A and the compound B may be a combination of the compound A (methylamine) of the formula (2) wherein $R^1$ is a hydrogen atom and $R^2$ is a methyl group, and the compound B (3,3-dimethyl-2-oxobutyric acid) of the formula (3) wherein each of n and m is 0, Y is a group represented by the formula (4), each of $R^3$, $R^4$ and $R^5$ is a methyl group, $R^6$ is a hydrogen atom, and each of p, q and r is 1.

<Compound C>

The compound C selected from the group consisting of a compound having an amino group and a carbonyl group and a salt thereof may be a compound represented by the following formula (5):

[Formula 27]

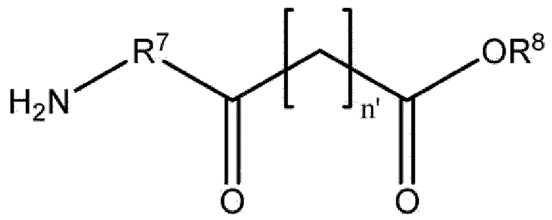

(5)

n' represents an integer of 0 or more and 2 or less. n may be 0 or 1, and may be 0.

$R^7$ represent an alkylene group, an alkenylene group, an alkynylene group, a cycloalkylene group, an arylene group, a heterocyclylene group, or a heteroarylene group, and these groups are optionally substituted.

$R^8$ represents a hydrogen atom, an optionally substituted $C_1$ to $C_6$ alkyl group, an optionally substituted $C_1$ to $C_6$ aryl group, an optionally substituted heteroaryl group having 5 or more and 12 or less ring-constituting atoms, a group containing a nitrogen atom, or a group containing an oxygen atom.

<Polypeptide>

The polypeptide comprises an amino acid sequence having 90% or higher sequence identity to an amino acid sequence represented by SEQ ID NO: 1. The identity to the amino acid sequence represented by SEQ ID NO: 1 may be 90% or higher, 91% or higher, 92% or higher, 93% or higher, 94% or higher, 95% or higher, 96% or higher, 97% or higher, 98% or higher, 99% or higher, or 100%.

The identity to the amino acid sequence represented by SEQ ID NO: 1 can be determined with algorithm BLAST by Karlin and Altschul (Proc. Natl. Acad. Sci. USA (1993) 90: 5873-7). A program called BLASTN or BLASTX has been developed on the basis of this algorithm (Altschul et al., J. Mol. Biol. (1990) 215: 403-10). In the case of analyzing an amino acid sequence with BLASTX on the basis of BLAST, the parameters are set to Score = 50 and Wordlength = 3. In the case of using BLAST and Gapped BLAST programs, the default parameters of each program are used. For specific approaches of these analysis methods, see information on the website of BLAST (basic local alignment search tool) of NCBI (Natianal Center for Biotechnology Information) known in the art.

The polypeptide according to the present embodiment may be a polypeptide having the engineering of one or more amino acid residues in the amino acid sequence represented by SEQ ID NO: 1, and 1 to 20, 1 to 15, 1 to 10, 1 to 7 or 1 to 5 amino acid residues may be engineered. The number of engineered amino acid residues may be 3 or less, may be 2 or less, or may be 1.

The engineering may be one or more selected from the group consisting of substitution, deletion and insertion and can be substitution. The engineering may be conservative engineering. The conservative engineering means the engineering of an amino acid residue so as not to reduce the catalytic activity of interest as compared with the polypeptide before the engineering.

The engineering may be substitution by a natural amino acid different from the amino acid residue before the engineering. The natural amino acid for use in the substitution may be one or more selected from the group consisting of glycine, alanine, serine, threonine, valine, leucine, isoleucine, phenylalanine, tyrosine, tryptophan, histidine, glutamine, asparagine, glutamic acid, aspartic acid, cysteine, methionine, lysine, arginine and proline.

The polypeptide according to the present embodiment may be a polypeptide comprising a sequence having the engineering of an amino acid residue positioned at a site corresponding to at least one amino acid residue selected from the group consisting of a histidine residue at position 44, a phenylalanine residue at position 117, a methionine residue at position 141, a threonine residue at position 156, a histidine residue at position 182, a glutamine residue at position 186, a tryptophan residue at position 253, and a lysine residue at position 260 in the amino acid sequence represented by SEQ ID NO: 1, may be a polypeptide comprising a sequence having the engineering of an amino acid residue positioned at a site corresponding to at least one amino acid residue selected from the group consisting of a methionine residue at position 141, a histidine residue at position 182, a tryptophan residue at position 253, and a lysine residue at position 260 because the catalytic activity for reductive amination reaction is further improved, and may be a polypeptide comprising a sequence having the engineering of an amino acid residue positioned at a site corresponding to at least one amino acid residue selected from the group consisting of a methionine residue at position 141, a tryptophan residue at position 253, and a lysine residue at position 260.

The polypeptide according to the present embodiment may be a polypeptide comprising a sequence having the substitution of an amino acid residue positioned at a site corresponding to a histidine residue at position 44 in the amino acid sequence represented by SEQ ID NO: 1 by an amino acid residue other than histidine residue. The polypeptide may be a polypeptide comprising the amino acid sequence represented by SEQ ID NO: 2. The amino acid residue represented by X in SEQ ID NO: 2 is an alanine residue, an aspartic acid residue, a glutamic acid residue, a phenylalanine residue, a

glycine residue, an isoleucine residue, a lysine residue, a leucine residue, a methionine residue, an asparagine residue, a proline residue, a glutamine residue, an arginine residue, a serine residue, a threonine residue, a valine residue, a tryptophan residue, or a tyrosine residue.

The polypeptide may comprise a sequence having the substitution of an amino acid residue positioned at a site corresponding to a histidine residue at position 44 by a methionine residue because the catalytic activity for reductive amination reaction is further improved. Specifically, the polypeptide according to the present embodiment may comprise an amino acid sequence (H44M) having the substitution of an amino acid residue positioned at a site corresponding to a histidine residue at position 44 in the amino acid sequence represented by SEQ ID NO: 1 by a methionine residue.

The polypeptide according to the present embodiment may be a polypeptide comprising a sequence having the substitution of an amino acid residue positioned at a site corresponding to a phenylalanine residue at position 117 in the amino acid sequence represented by SEQ ID NO: 1 by an amino acid residue other than a phenylalanine residue. The polypeptide may be a polypeptide comprising the amino acid sequence represented by SEQ ID NO: 3. The amino acid residue represented by X in SEQ ID NO: 3 is an alanine residue, an aspartic acid residue, a glutamic acid residue, a glycine residue, a histidine residue, an isoleucine residue, a lysine residue, a leucine residue, a methionine residue, an asparagine residue, a proline residue, a glutamine residue, an arginine residue, a serine residue, a threonine residue, a valine residue, a tryptophan residue, or a tyrosine residue.

The polypeptide may comprise a sequence having the substitution of an amino acid residue positioned at a site corresponding to a phenylalanine residue at position 117 by a leucine residue because the catalytic activity for reductive amination reaction is further improved. Specifically, the polypeptide according to the present embodiment may comprise an amino acid sequence (F117L) having the substitution of an amino acid residue positioned at a site corresponding to a phenylalanine residue at position 117 in the amino acid sequence represented by SEQ ID NO: 1 by a leucine residue.

The polypeptide according to the present embodiment may be a polypeptide comprising a sequence having the substitution of an amino acid residue positioned at a site corresponding to a methionine residue at position 141 in the amino acid sequence represented by SEQ ID NO: 1 by an amino acid residue other than methionine residue. The polypeptide may be a polypeptide comprising the amino acid sequence represented by SEQ ID NO: 4. The amino acid residue represented by X in SEQ ID NO: 4 is an alanine residue, an aspartic acid residue, a glutamic acid residue, a phenylalanine residue, a glycine residue, a histidine residue, an isoleucine residue, a lysine residue, a leucine residue, an asparagine residue, a proline residue, a glutamine residue, an arginine residue, a serine residue, a threonine residue, a valine residue, a tryptophan residue, or a tyrosine residue.

The polypeptide may comprise a sequence having the substitution of an amino acid residue positioned at a site corresponding to a methionine residue at position 141 by at least one amino acid residue selected from the group consisting of a tyrosine residue, a tryptophan residue, a valine residue, a threonine residue, a serine residue, an arginine residue, a leucine residue, a lysine residue, an isoleucine residue, a histidine residue, a phenylalanine residue and an alanine residue because the catalytic activity for reductive amination reaction is further improved. Specifically, the polypeptide according to the present embodiment may comprise an amino acid sequence having the substitution of an amino acid residue positioned at a site corresponding to a methionine residue at position 141 in the amino acid sequence represented by SEQ ID NO: 1 by a tyrosine residue, a tryptophan residue, a valine residue, a threonine residue, a serine residue, an arginine residue, a leucine residue, a lysine residue, an isoleucine residue, a histidine residue, a phenylalanine residue, or an alanine residue (M141Y, M141W, M141V, M141T, M141S, M141R, M141L, M141K, M141I, M141H, M141F, or M141A). The polypeptide may comprise a sequence having the substitution of an amino acid residue positioned at a site corresponding to a methionine residue at position 141 by at least one amino acid residue selected from the group consisting of a tyrosine residue, a tryptophan residue, a valine residue, a lysine residue, an isoleucine residue, a phenylalanine residue and an alanine residue. Specifically, the polypeptide according to the present embodiment may comprise an amino acid sequence having the substitution of an amino acid residue positioned at a site corresponding to a methionine residue at position 141 in the amino acid sequence represented by SEQ ID NO: 1 by a tyrosine residue, a tryptophan residue, a valine residue, a lysine residue, an isoleucine residue, a histidine residue, a phenylalanine residue, or an alanine residue (M141Y, M141W, M141V, M141K, M141I, M141H, M141F, or M141A).

The polypeptide according to the present embodiment may be a polypeptide comprising a sequence having the substitution of an amino acid residue positioned at a site corresponding to a threonine residue at position 156 in the amino acid sequence represented by SEQ ID NO: 1 by an amino acid residue other than threonine residue. The polypeptide may be a polypeptide comprising the amino acid sequence represented by SEQ ID NO: 5. The amino acid residue represented by X in SEQ ID NO: 5 is an alanine residue, an aspartic acid residue, a glutamic acid residue, a phenylalanine residue, a glycine residue, a histidine residue, an isoleucine residue, a lysine residue, a leucine residue, an asparagine residue, a proline residue, a glutamine residue, an arginine residue, a serine residue, a threonine residue, a valine residue, a tryptophan residue, or a tyrosine residue.

The polypeptide can comprise a sequence having the substitution of an amino acid residue positioned at a site corresponding to a threonine residue at position 156 by a serine residue because the catalytic activity for reductive amination reaction is further improved. Specifically, the polypeptide according to the present embodiment can comprise an

amino acid sequence having the substitution of an amino acid residue positioned at a site corresponding to a threonine residue at position 156 in the amino acid sequence represented by SEQ ID NO: 1 by a serine residue (T156S).

The polypeptide according to the present embodiment may be a polypeptide comprising a sequence having the substitution of an amino acid residue positioned at a site corresponding to a histidine residue at position 182 in the amino acid sequence represented by SEQ ID NO: 1 by an amino acid residue other than histidine residue. The polypeptide may be a polypeptide comprising the amino acid sequence represented by SEQ ID NO: 6. The amino acid residue represented by X in SEQ ID NO: 6 is an alanine residue, an aspartic acid residue, a glutamic acid residue, a phenylalanine residue, a glycine residue, an isoleucine residue, a lysine residue, a leucine residue, a methionine residue, an asparagine residue, a proline residue, a glutamine residue, an arginine residue, a serine residue, a threonine residue, a valine residue, a tryptophan residue, or a tyrosine residue.

The polypeptide may comprise a sequence having the substitution of an amino acid residue positioned at a site corresponding to a histidine residue at position 182 by at least one amino acid residue selected from the group consisting of a tyrosine residue, a glutamine residue, a methionine residue, a leucine residue, a glycine residue, a phenylalanine residue and an alanine residue because the catalytic activity for reductive amination reaction is further improved. Specifically, the polypeptide according to the present embodiment may comprise an amino acid sequence having the substitution of an amino acid residue positioned at a site corresponding to a histidine residue at position 182 in the amino acid sequence represented by SEQ ID NO: 1 by a tyrosine residue, a glutamine residue, a methionine residue, a leucine residue, a glycine residue, a phenylalanine residue, or an alanine residue (H182Y, H182Q, H182M, H182L, H182G, H182F, or H182A). The polypeptide may comprise a sequence having the substitution of an amino acid residue positioned at a site corresponding to a histidine residue at position 182 by at least one amino acid residue selected from the group consisting of a methionine residue, a leucine residue, and a phenylalanine residue. Specifically, the polypeptide according to the present embodiment may comprise an amino acid sequence having the substitution of an amino acid residue positioned at a site corresponding to a histidine residue at position 182 in the amino acid sequence represented by SEQ ID NO: 1 by a methionine residue, a leucine residue, or a phenylalanine residue (H182M, H182L, or H182F).

The polypeptide according to the present embodiment may be a polypeptide comprising a sequence having the substitution of an amino acid residue positioned at a site corresponding to a glutamine residue at position 186 in the amino acid sequence represented by SEQ ID NO: 1 by an amino acid residue other than a glutamine residue. The polypeptide may be a polypeptide comprising the amino acid sequence represented by SEQ ID NO: 7. The amino acid residue represented by X in SEQ ID NO: 7 is an alanine residue, an aspartic acid residue, a glutamic acid residue, a phenylalanine residue, a glycine residue, a histidine residue, an isoleucine residue, a lysine residue, a leucine residue, a methionine residue, an asparagine residue, a proline residue, an arginine residue, a serine residue, a threonine residue, a valine residue, a tryptophan residue, or a tyrosine residue.

The polypeptide may comprise a sequence having the substitution of an amino acid residue positioned at a site corresponding to a glutamine residue at position 186 in the amino acid sequence represented by SEQ ID NO: 1 by at least one amino acid residue selected from the group consisting of a methionine residue and a glutamic acid residue because the catalytic activity for reductive amination reaction is further improved. Specifically, the polypeptide according to the present embodiment may comprise an amino acid sequence having the substitution of an amino acid residue positioned at a site corresponding to a glutamine residue at position 186 in the amino acid sequence represented by SEQ ID NO: 1 by a methionine residue or a glutamic acid residue (Q186M, or Q186E).

The polypeptide according to the present embodiment may be a polypeptide comprising a sequence having the substitution of an amino acid residue positioned at a site corresponding to a tryptophan residue at position 253 in the amino acid sequence represented by SEQ ID NO: 1 by an amino acid residue other than a tryptophan residue. The polypeptide may be a polypeptide comprising the amino acid sequence represented by SEQ ID NO: 8. The amino acid residue represented by X in SEQ ID NO: 8 is an alanine residue, an aspartic acid residue, a glutamic acid residue, a phenylalanine residue, a glycine residue, a histidine residue, an isoleucine residue, a lysine residue, a leucine residue, a methionine residue, an asparagine residue, a proline residue, a glutamine residue, an arginine residue, a serine residue, a threonine residue, a valine residue, or a tyrosine residue.

The polypeptide may comprise a sequence having the substitution of an amino acid residue positioned at a site corresponding to a tryptophan residue at position 253 by at least one amino acid residue selected from the group consisting of a tyrosine residue, a valine residue, a threonine residue, a serine residue, an arginine residue, a glutamine residue, a proline residue, an asparagine residue, a methionine residue, a leucine residue, a lysine residue, an isoleucine residue, a histidine residue, a phenylalanine residue and an alanine residue because the catalytic activity for reductive amination reaction is further improved. Specifically, the polypeptide according to the present embodiment can comprise an amino acid sequence having the substitution of an amino acid residue positioned at a site corresponding to a tryptophan residue at position 253 in the amino acid sequence represented by SEQ ID NO: 1 by a tyrosine residue, a valine residue, a threonine residue, a serine residue, an arginine residue, a glutamine residue, a proline residue, an asparagine residue, a methionine residue, a leucine residue, a lysine residue, an isoleucine residue, a histidine residue, a phenylalanine residue, or an alanine residue (W253Y, W253V, W253T, W253S, W253R, W253Q, W253P, W253N, W253M, W253L, W253K,

W253I, W253H, W253F, or W253A). The polypeptide may comprise a sequence having the substitution of an amino acid residue positioned at a site corresponding to a tryptophan residue at position 253 by at least one amino acid residue selected from the group consisting of a leucine residue, an isoleucine residue and a histidine residue. Specifically, the polypeptide according to the present embodiment may comprise an amino acid sequence having the substitution of an amino acid residue positioned at a site corresponding to a tryptophan residue at position 253 in the amino acid sequence represented by SEQ ID NO: 1 by a leucine residue, an isoleucine residue or a histidine residue (W253L, W253I, or W253H).

The polypeptide according to the present embodiment may be a polypeptide comprising a sequence having the substitution of an amino acid residue positioned at a site corresponding to a lysine residue at position 260 in the amino acid sequence represented by SEQ ID NO: 1 by an amino acid residue other than a lysine residue. The polypeptide may be a polypeptide comprising the amino acid sequence represented by SEQ ID NO: 9. The amino acid residue represented by X in SEQ ID NO: 9 is an alanine residue, an aspartic acid residue, a glutamic acid residue, a phenylalanine residue, a glycine residue, a histidine residue, an isoleucine residue, a leucine residue, a methionine residue, an asparagine residue, a proline residue, a glutamine residue, an arginine residue, a serine residue, a threonine residue, a valine residue, a tryptophan residue, or a tyrosine residue.

The polypeptide may comprise a sequence having the substitution of an amino acid residue positioned at a site corresponding to a lysine residue at position 260 by at least one amino acid residue selected from the group consisting of a tyrosine residue, a tryptophan residue, a threonine residue, a serine residue, an arginine residue, a glutamine residue, an asparagine residue, a methionine residue, a leucine residue, a histidine residue, a glycine residue, a phenylalanine residue, a glutamic acid residue and an alanine residue because the catalytic activity for reductive amino reaction is further improved. Specifically, the polypeptide according to the present embodiment may comprise an amino acid sequence having the substitution of an amino acid residue positioned at a site corresponding to a lysine residue at position 260 in the amino acid sequence represented by SEQ ID NO: 1 by a tyrosine residue, a tryptophan residue, a threonine residue, a serine residue, an arginine residue, a glutamine residue, a asparagine residue, a methionine residue, a leucine residue, a histidine residue, a glycine residue, a phenylalanine residue, a glutamic acid residue or an alanine residue (K260Y, K260W, K260T, K260S, K260R, K260Q, K260N, K260M, K260L, K260H, K260G, K260F, K260E, or K260A). The polypeptide may further comprise a sequence having the substitution of an amino acid residue positioned at a site corresponding to a lysine residue at position 260 by at least one amino acid residue selected from the group consisting of a glutamine residue, a methionine residue, a glutamic acid residue and an asparagine residue. Specifically, the polypeptide according to the present embodiment may comprise an amino acid sequence having the substitution of an amino acid residue positioned at a site corresponding to a lysine residue at position 260 in the amino acid sequence represented by SEQ ID NO: 1 by a glutamine residue, a methionine residue, a glutamic acid residue or an asparagine residue (K260Q, K260M, K260E, or K260N).

The polypeptide according to the present embodiment may comprise amino acid sequence a1 represented by SEQ ID NO: 4 wherein the amino acid residue represented by X is a valine residue (mutation: M141V), amino acid sequence a2 represented by SEQ ID NO: 4 wherein the amino acid residue represented by X is a tyrosine residue (mutation: M141Y), amino acid sequence a3 represented by SEQ ID NO: 6 wherein the amino acid residue represented by X is a leucine residue (mutation: H182L), amino acid sequence a4 represented by SEQ ID NO: 8 wherein the amino acid residue represented by X is a histidine residue (mutation: W253H), or amino acid sequence a5 represented by SEQ ID NO: 9 wherein the amino acid residue represented by X is a glutamic acid residue (mutation: K260E).

The polypeptide according to the present embodiment may comprise a sequence having 90% or higher sequence identity to the amino acid sequence a1, a2, a3, a4, or a5. The sequence identity may be 91% or higher, 92% or higher, 93% or higher, 94% or higher, 95% or higher, 96% or higher, 97% or higher, 98% or higher, or 99% or higher, or may be 100%.

The polypeptide according to the present embodiment may be a polypeptide comprising an amino acid sequence a1, a2, a3, a4, or a5 with one or more amino acid residue thereof engineered, and 1 to 20, 1 to 15, 1 to 10, 1 to 7 or 1 to 5 amino acid residues may be engineered. The number of engineered amino acid residues may be 3 or less, may be 2 or less, or may be 1.

In the polypeptide according to the present embodiment, the amino acid sequence represented by SEQ ID NO: 1 may have two or more engineering sites, or two engineering sites. Hereinafter, a preferred embodiment of the polypeptide having two or more modified portions will be described.

The polypeptide may preferably comprise a sequence comprising the engineering of an amino acid residue positioned at a site corresponding to at least one amino acid residue selected from the group consisting of a methionine residue at position 141, a histidine residue at position 182, and a tryptophan residue at position 253 in the amino acid sequence represented by SEQ ID NO: 1 as a first engineering site, and the engineering of an amino acid residue positioned at a site corresponding to at least one amino acid residue selected from the group consisting of a histidine residue at position 182, a tryptophan residue at position 253, and a lysine residue at position 260 in the amino acid sequence represented by SEQ ID NO: 1 as a second engineering site, wherein the amino acid reside engineered at the second

engineering site is positioned at a site corresponding to one amino acid residue different from the amino acid residue engineered at the first engineering site.

The polypeptide may preferably comprise a sequence having the engineering of an amino acid residue positioned at a site corresponding to a methionine residue at position 141 and a histidine residue at position 182, the engineering of an amino acid residue positioned at a site corresponding to a methionine residue at position 141 and a tryptophan residue at position 253, the engineering of an amino acid residue positioned at a site corresponding to a methionine residue at position 141 and a lysine residue at position 260, the engineering of an amino acid residue positioned at a site corresponding to a histidine residue at position 182 and a tryptophan residue at position 253, the engineering of an amino acid residue positioned at a site corresponding to a histidine residue at position 182 and a lysine residue at position 260, or the engineering of an amino acid residue positioned at a site corresponding to a tryptophan residue at position 253 and a lysine residue at position 260 in the amino acid sequence represented by SEQ ID NO: 1. The polypeptide may more preferably comprise a sequence having the engineering of an amino acid residue positioned at a site corresponding to a methionine residue at position 141 and a lysine residue at position 260, the engineering of an amino acid residue positioned at a site corresponding to a histidine residue at position 182 and a lysine residue at position 260, or the engineering of an amino acid residue positioned at a site corresponding to a tryptophan residue at position 253 and a lysine residue at position 260 in the amino acid sequence represented by SEQ ID NO: 1.

The polypeptide may preferably comprise a sequence having the substitution of an amino acid residue positioned at a site corresponding to a methionine residue at position 141 in the amino acid sequence represented by SEQ ID NO: 1 by one amino acid residue selected from the group consisting of a tyrosine residue, a tryptophan residue, a valine residue, a threonine residue, an arginine residue, a lysine residue, an isoleucine residue and an alanine residue, or more preferably comprise a sequence having the substitution of an amino acid residue positioned at a site corresponding to a methionine residue at position 141 in the amino acid sequence represented by SEQ ID NO: 1 by one amino acid residue selected from the group consisting of a tyrosine residue, a valine residue and an alanine residue.

The polypeptide may preferably comprise a sequence having the substitution of an amino acid residue positioned at a site corresponding to a histidine residue at position 182 in the amino acid sequence represented by SEQ ID NO: 1 by one amino acid residue selected from the group consisting of a tyrosine residue, a methionine residue, a leucine residue and a phenylalanine residue, or more preferably comprise a sequence having the substitution of an amino acid residue positioned at a site corresponding to a histidine residue at position 182 in the amino acid sequence represented by SEQ ID NO: 1 by one amino acid residue selected from the group consisting of a methionine residue and a leucine residue.

The polypeptide may preferably comprise a sequence having the substitution of an amino acid residue positioned at a site corresponding to a tryptophan residue at position 253 in the amino acid sequence represented by SEQ ID NO: 1 by one amino acid residue selected from the group consisting of a tyrosine residue, a threonine residue, a serine residue, an asparagine residue, a methionine residue, a histidine residue and an alanine residue, or more preferably comprise a sequence having the substitution of an amino acid residue positioned at a site corresponding to a tryptophan residue at position 253 in the amino acid sequence represented by SEQ ID NO: 1 by a histidine residue.

The polypeptide may preferably comprise a sequence having the substitution of an amino acid residue positioned at a site corresponding to a lysine residue at position 260 in the amino acid sequence represented by SEQ ID NO: 1 by one amino acid residue selected from the group consisting of a serine residue, a glutamine residue, an asparagine residue, a leucine residue, an isoleucine residue, a histidine residue, a glycine residue, a phenylalanine residue, a glutamic acid residue and an alanine residue, or more preferably comprise a sequence having the substitution of an amino acid residue positioned at a site corresponding to a lysine residue at position 260 in the amino acid sequence represented by SEQ ID NO: 1 by one amino acid residue selected from the group consisting of a glutamine residue and a glutamic acid residue.

Hereinafter, in the amino acid sequence represented by SEQ ID NO: 1, a sequence in which an amino acid residue located at a site corresponding to an amino acid residue $X^1$ at position $m^1$ is substituted by another amino acid residue $x^1$ other than $X^1$, and an amino acid residue located at a site corresponding to amino acid residue $X^2$ at a site $m^2$ is substituted by another amino acid residue $x^2$ other than $X^2$ is referred to as $X^1m^1x^1_X^2m^2x^2$.

The amino acid sequence represented by SEQ ID NO : 1 with two amino acid residues engineered thereof may be, for example, an amino acid sequence selected from the group a1 to group a4 consisting of the following amino acid sequences, may be preferably an amino acid sequence selected from the group a1 to group a3, may be more preferably an amino acid sequence selected from the group a1 and group a2, and may be most preferably an amino acid sequence selected from group a1.

Group a1:
M141Y_K260E, H182L_K260Q, M141A _K260E, H182M_K260Q, M141A_K260Q, H182M_K260E, M141V_K260E, W253H_K260E, and H182L_K260E.
Group a2:
M141V_K260Q, W253H_K260Q, W253M _K260E, M141Y_H182M, M141Y_K260Q, H182F_K260Q, M141V_K260F, M141V_H182L, M141V_K260L, M141Y_K260G, H182L_W253H, H182F_K260E, and

M141T_K260Q
Group a3:
M141Y_H182L, W253M_K260Q, M141V_W253M, M141V_K260S, M141V_K260A, M141A_K260F, M141A_K260H, W253A_K260E, M141V_W253A, M141V_W253H, M141K_K260L, M141V_K260G, and M141A_K260L.
Group a4:
M141W_K260A, M141V_W253Y, M141K_K260G, M141A_K260G, M141A_K260S, M141A_K260N, M141Y_W253H, M141A_K260A, M141K_K260S, W253A_K260Q, M141V_H182M, M141A_K260I, M141T_K260G, W253M_K260L, M141I_W253A, W253T _K260L, M141R_K260G, W253A _K260L, M141K_W253S, M141A_H182L, W253N_K260L, M141K_K260A, M141K_W253M, H182Y_W253A, H182M_W253H, M141R_K260A, H182M_W253A and M141K_W253A.

The polypeptide may comprise, for example, a sequence having 90% or more sequence identity to an amino acid sequence selected from the group consisting of group a1 to group a4, preferably comprise a sequence having 90% or more sequence identity to an amino acid sequence selected from the group consisting of group a1 to group a3, more preferably comprises a sequence having 90% or more sequence identity to an amino acid sequence selected from the group consisting of group a1 and group a2, and most preferably comprises a sequence having 90% or more sequence identity to an amino acid sequence selected from group a1.

The polypeptide may comprise an amino acid sequence selected from the group consisting of groups a1 to a4, preferably comprises an amino acid sequence selected from the group consisting of groups a1 to a3, more preferably comprises an amino acid sequence selected from the group consisting of groups a1 and a2, and most preferably comprises an amino acid sequence selected from group a1.

The polypeptide may have three or more engineering sites or three engineering sites in the amino acid sequence represented by SEQ ID NO: 1. Hereinafter, a preferred embodiment of the polypeptide having three or more engineering sites is described.

The polypeptide may preferable comprise a sequence comprising the engineering of an amino acid residue positioned at a site corresponding to at least one amino acid residue selected from the group consisting of a methionine residue at position 141, and a histidine residue at position 182 in the amino acid sequence represented by SEQ ID NO: 1 as a first engineering site, the engineering of an amino acid residue positioned at a site corresponding to at least one amino acid residue selected from the group consisting of a histidine residue at position 182, and a tryptophan residue at position 253 in the amino acid sequence represented by SEQ ID NO: 1 as a second engineering site, wherein the amino acid reside engineered at the second engineering site is positioned at a site corresponding to one amino acid residue different from the amino acid residue engineered at the first engineering site, and the engineering of an amino acid residue positioned at a site corresponding to at least one amino acid residue selected from the group consisting of a tryptophan residue at position 253, and a lysine residue at position 260 in the amino acid sequence represented by SEQ ID NO: 1 as a third engineering site, wherein the amino acid reside engineered at the third engineering site is positioned at a site corresponding to one amino acid residue different from the amino acid residue engineered at the first engineering site and the second engineering site.

The polypeptide may preferably comprise a sequence having the engineering of an amino acid residue positioned at a site corresponding to a methionine residue at position 141, a histidine residue at position 182, and a tryptophan residue at position 253, the engineering of an amino acid residue positioned at a site corresponding to a methionine residue at position 141, a histidine residue at position 182 and a lysine residue at position 260, the engineering of an amino acid residue positioned at a site corresponding to a methionine residue at position 141, a tryptophan residue at position 253 and a lysine residue at position 260, or the engineering of an amino acid residue positioned at a site corresponding to a histidine residue at position 182, a tryptophan residue at position 253 and a lysine residue at position 260 in the amino acid sequence represented by SEQ ID NO: 1. The polypeptide may more preferably comprise a sequence having the engineering of an amino acid residue positioned at a site corresponding to a methionine residue at position 141, a histidine residue at position 182 and a lysine residue at position 260, the engineering of an amino acid residue positioned at a site corresponding to a methionine residue at position 141, a tryptophan residue at position 253 and a lysine residue at position 260, or the engineering of an amino acid residue positioned at a site corresponding to a histidine residue at position 182, a tryptophan residue at position 253 and a lysine residue at position 260 in the amino acid sequence represented by SEQ ID NO: 1.

The polypeptide may preferably have three engineering sites in the amino acid sequence represented by SEQ ID NO: 1 and comprise a sequence having the substitution of an amino acid residue positioned at a site corresponding to a methionine residue at position 141 in the amino acid sequence represented by SEQ ID NO: 1 by one amino acid residue selected from the group consisting of a tyrosine residue, a valine residue, a threonine residue, a serine residue, a leucine residue, a lysine residue, an isoleucine residue and an alanine residue. The polypeptide may more preferably have three engineering sites in the amino acid sequence represented by SEQ ID NO: 1 and comprise sequence having the

substitution of an amino acid residue positioned at a site corresponding to a methionine residue at position 141 in the amino acid sequence represented by SEQ ID NO: 1 by one amino acid residue selected from the group consisting of a tyrosine residue, a valine residue and an isoleucine residue.

The polypeptide may preferably have three engineering sites in the amino acid sequence represented by SEQ ID NO: 1 and comprise a sequence having the substitution of an amino acid residue positioned at a site corresponding to a histidine residue at position 182 in the amino acid sequence represented by SEQ ID NO: 1 by one amino acid residue selected from the group consisting of a methionine residue, a leucine residue and a phenylalanine residue. The polypeptide may more preferably have three engineering sites in the amino acid sequence represented by SEQ ID NO: 1 and comprise a sequence having the substitution of an amino acid residue positioned at a site corresponding to a histidine residue at position 182 in the amino acid sequence represented by SEQ ID NO: 1 by one amino acid residue selected from the group consisting of a methionine residue and a leucine residue.

The polypeptide may preferably have three engineering sites in the amino acid sequence represented by SEQ ID NO: 1 and comprise a sequence having the substitution of an amino acid residue positioned at a site corresponding to a tryptophan residue at position 253 in the amino acid sequence represented by SEQ ID NO: 1 by one amino acid residue selected from the group consisting of a valine residue, a threonine residue, a serine residue, a glutamine residue, a methionine residue, a leucine residue, a histidine residue, a phenylalanine residue and a glutamic acid residue. The polypeptide may more preferably have three engineering sites in the amino acid sequence represented by SEQ ID NO: 1 and comprise a sequence having the substitution of an amino acid residue positioned at a site corresponding to a tryptophan residue at position 253 in the amino acid sequence represented by SEQ ID NO: 1 by one amino acid residue selected from the group consisting of a glutamine residue, a methionine residue, a leucine residue and a histidine residue.

The polypeptide may preferably have three engineering sites in the amino acid sequence represented by SEQ ID NO: 1 and comprise a sequence having the substitution of an amino acid residue positioned at a site corresponding to a lysine residue at position 260 in the amino acid sequence represented by SEQ ID NO: 1 by one amino acid residue selected from the group consisting of a glutamine residue and a glutamic acid residue.

Hereinafter, in the amino acid sequence represented by SEQ ID NO: 1, an amino acid sequence at a site corresponding to an amino acid residue $X^1$ at position $m^1$ is substituted by another amino acid residue $x^1$ other than $X^1$, an amino acid residue at a site corresponding to an amino acid residue $X^2$ at position $m^2$ is substituted by another amino acid residue $x^2$ other than $X^2$, and an amino acid residue at a site corresponding to an amino acid residue $X^3$ at position $m^3$ is substituted by another amino acid residue $x^3$ other than $X^3$ is referred to as $X^1m^1x^1$_$X^2m^2x^2$_$X^3m^3x^3$.

The amino acid sequence by SEQ ID NO: 1 with three amino acid residues engineered thereof may be, for example, an amino acid sequence selected from the group b1 to group b4 consisting of the following amino acid sequences, may be preferably an amino acid sequence selected from the group b1 to group b3, may be more preferably an amino acid sequence selected from the group b1 and group b2, and may be most preferably an amino acid sequence selected from group b1.

Group b1:
M141Y_H182M_K260E, M141V_W253M _K260E, H182L_W253H_K260E, M141Y_H182M_K260Q, M141V_H182M_K260E, M141V_H182L_K260Q, M141Y_H182L_K260Q, M141V_W253Q_K260E, H182L_W253Q_K260E, M141I_W253H_K260E, M141V_H182L_K260E, M141Y_H182L_K260E, H182L _W253M_K260E, M141V_W253Q_K260Q, M141V_W253H_K260Q, M141V _W253L_K260Q, M1411_W253H_K260Q, and M141V_W253M_K260Q.

Group b2:
H182M_W253H_K260E, M141V_W253V_K260Q, H182L_W253H_K260Q, M141V_W253H_K260E, H182L_W253Q_K260Q, M141L_W253H_K260E, H182M_W253Q_K260E, M141V_W253F _K260Q, M141V_W253L_K260E, M141V _W253T_K260Q, M141Y_H182F_K260E, H182M_W253M_K260E, H182L_W253M_K260Q, M141Y_W253H_K260E, M141Y_W253Q_K260E, M141V_H182M_K260Q, H182M_W253L_K260E, H182F_W253H_K260E, M141A_H182F_K260Q, M141Y_W253M_K260E, H182M_W253Q_K260Q, and H182M_W253M_K260Q.

Group b3:
H182L_W253T_K260Q, M141Y_W253S_K260E, M141V_H182F _K260E, M141A_H182M_K260E, M141T_H182L_K260Q, M141Y_W253T _K260E, H182M_W253L_K260Q, M141V_H182F_K260Q, M141S_W253H_K260E, M141T_W253H_K260E, H182M_W253H_K260Q, M141A _H182L_K260Q, H182M_W253F_K260Q, M141A_H182M _K260Q, M141A_W253Q_K260E, M141A_H182L_K260E, M141A_W253H_K260E, M141V_W253E_K260Q, M141A_W253M_K260E, M141A_W253H_K260Q, M141K_H182L_W253H, and M141T_H182M_K260Q.

Group b4:
M141A_W253M_K260Q and M141A_H182F_K260E

The polypeptide may comprise, for example, an amino acid sequence having 90% or more sequence identity to an amino acid sequence selected from the group consisting of groups b1 to b4, preferably comprises a sequence having 90% or more sequence identity to an amino acid sequence selected from the group consisting of groups b1 to b3, more preferably comprises a sequence having 90% or more sequence identity to an amino acid sequence selected from the group consisting of groups b1 and b2, and most preferably comprises a sequence having 90% or more sequence identity to an amino acid sequence selected from the group consisting of group b1.

The polypeptide may comprise an amino acid sequence selected from the group consisting of groups b1 to b4, preferably comprises an amino acid sequence selected from the group consisting of groups b1 to b3, more preferably comprises an amino acid sequence selected from the group consisting of groups b1 and b2, and most preferably comprises an amino acid sequence selected from group b1.

The polypeptide according to the present embodiment may be fused with another polypeptide or a protein. Specifically, the polypeptide according to the present embodiment may comprise an amino acid sequence (additional amino acid sequence) other than the sequence having 90% or higher sequence identity to an amino acid sequence represented by SEQ ID NO: 1 with one amino acid residue thereof engineered at any one or both of the N terminus and the C terminus. The additional amino acid sequence may be, for example, a tag sequence.

Examples of a polypeptide or a protein having the additional amino acid sequence include His tag (6 × His, 10 × His, etc.) which is a tag consisting of several (e.g., 6 and 10) His (histidine) residues, streptavidin-bound peptide tag (SBP tag) comprising an amino acid sequence having the ability to bind to a biotin binding site of streptavidin, GST (glutathione S-transferase), HA (influenza hemagglutinin), immunoglobulin constant regions, B-galactosidase, MBP (maltose binding protein), FLAG (Hopp, T.P. et al., BioTechnology (1988) 6, 1204-1210), influenza hemagglutinin (HA), human c-myc fragments, VSV-GP fragments, p18 HIV fragments, T7-tag, HSV-tag, E-tag, SV40T antigen fragments, Ick tag, α-tubulin fragments, B-tag, protein C fragments, Stag, StrepTag, and HaloTag. The His tag may be, for example, the amino acid sequence represented by SEQ ID NO: 12. The SBP tag may be, for example, the amino acid sequence represented by SEQ ID NO: 13.

The polypeptide according to the present embodiment may comprise one or more selected from the group consisting of a streptavidin-bound peptide tag sequence and a His tag sequence at any one or both of the N terminus and the C terminus, and may comprise a streptavidin-bound peptide tag sequence (SBP tag) and a His tag sequence at the C terminus.

The polypeptide according to the present embodiment may comprise amino acid sequence a1 represented by SEQ ID NO: 4 wherein the amino acid residue represented by X is a valine residue (mutation: M141V), amino acid sequence a2 represented by SEQ ID NO: 4 wherein the amino acid residue represented by X is a tyrosine residue (mutation: M141Y), amino acid sequence a3 represented by SEQ ID NO: 6 wherein the amino acid residue represented by X is a leucine residue (mutation: H182L), amino acid sequence a4 represented by SEQ ID NO: 8 wherein the amino acid residue represented by X is a histidine residue (mutation: W253H), or amino acid sequence a5 represented by SEQ ID NO: 9 wherein the amino acid residue represented by X is a glutamic acid residue (mutation: K260E), and a tag sequence. The polypeptide according to the present embodiment may comprise, in addition to the amino acid sequence a1, a2, a3, a4, or a5 and a tag sequence, a linker sequence that links the amino acid sequence a1, a2, a3, a4, or a5 to the tag sequence. The linker sequence may comprise, for example, the amino acid sequence represented by GGSS or GGS.

The polypeptide according to one embodiment may be a polypeptide X1 constituted by the amino acid sequence a1, a2, a3, a4, or a5, a linker sequence that binds to the C terminus of the amino acid sequence a1, a2, a3, a4, or a5 and is represented by the amino acid sequence: GGSS, and a His tag that binds to the linker sequence and represented by SEQ ID NO: 12. One or more amino acid residues in polypeptide X1 may be engineered, and 1 to 20, 1 to 15, 1 to 10, 1 to 7 or 1 to 5 amino acid residues may be engineered. The number of engineered amino acid residues in polypeptide X1 may be 3 or less, may be 2 or less, or may be 1.

The polypeptide according to one embodiment may be a polypeptide X2 constituted by the amino acid sequence a1, a2, a3, a4, or a5, an SBP tag that binds to the C terminus of the amino acid sequence a1, a2, a3, a4, or a5 and is represented by SEQ ID NO: 13, a His tag represented by SEQ ID NO: 12, and a linker sequence that links the His tag and the SBP tag and is represented by the amino acid sequence: GGS. One or more amino acid residues in polypeptide X2 may be engineered, and 1 to 20, 1 to 15, 1 to 10, 1 to 7 or 1 to 5 amino acid residues may be engineered. The number of engineered amino acid residues in polypeptide X2 may be 3 or less, may be 2 or less, or may be 1.

The polypeptide according to the present embodiment may be used as a mixture with another polypeptide and may be used in an isolated or purified state. In the case of using the polypeptide as a mixture with another polypeptide, the amino acid of interest may be obtained through the step of isolating or purifying the target product.

The number of amino acid residues in the polypeptide according to the present embodiment in the form of a monomer may be 300 or more, 310 or more, 320 or more, or 325 or more, or may be 330. The number may be 400 or less, 390 or less, 380 or less, or 375 or less.

When the additional amino acid sequence is added to neither the N terminus nor the C terminus, the number of amino acid residues in the polypeptide according to the present embodiment may be 300 or more, 310 or more, 320 or

more, or 325 or more, or may be 330. The number may be 360 or less, 350 or less, 340 or less, or 335 or less.

When the additional amino acid sequence is added to any one or both of the N terminus and the C terminus, the number of amino acid residues in the polypeptide according to the present embodiment may be 340 or more, 350 or more, 360 or more, or 370 or more, or may be 374. The number may be 400 or less, 390 or less, 380 or less, or 375 or less.

The polypeptide according to the present embodiment may be used as a monomer or may be used in an associated form of two or more monomers. The polypeptide according to the present embodiment may be a homodimer.

When the polypeptide according to the present embodiment is a homodimer, the homodimer has two times the number of amino acid residues in the form of a monomer.

In one aspect, the polypeptide has catalytic activity for an intermolecular reductive amination reaction between compound A and compound B or an intramolecular reductive amination reaction of compound C under at least one reaction condition.

For the measurement of the catalytic activity, methylamine as the compound A and sodium 4-(2-chlorophenyl)-2-oxobutanoate as the compound B can be used in combination. In this case, the catalytic activity of the polypeptide can be evaluated with MeHph(2-Cl) synthetic activity as an index.

In another aspect, for the measurement of the catalytic activity, ethylamine as the compound A and sodium phenylpyruvate as the compound B can be used in combination. In this case, the catalytic activity of the polypeptide can be evaluated with EtPhe synthetic activity as an index.

In another aspect, for the measurement of the catalytic activity, methylamine as the compound A and sodium 2-cyclopentyl-2-oxoacetate as the compound B can be used in combination. In this case, the catalytic activity of the polypeptide can be evaluated with MeGly(cPent) synthetic activity as an index.

In another aspect, for the measurement of the catalytic activity, ammonia as the compound A and phenylpyruvic acid as the compound B can be used in combination. In this case, the catalytic activity of the polypeptide can be evaluated with Phe synthetic activity as an index.

In one aspect, the catalytic activity of a polypeptide can be evaluated using reaction conditions described in Examples. For example, the concentration of each compound in the reaction solution at the start of reaction can be 50 mM for the compound B or a salt thereof, 100 mM for D(+)-glucose, 500 mM for the compound A or a salt thereof, 100 mM for a phosphate buffer solution, 1 mM for NADPH, 0.002 unit/$\mu$L for a GDH solution, 2.5 $\mu$M for the polypeptide to be evaluated, and 20 v/v% or 20 w/v% for the compound D or compound D'.

In one embodiment, the catalytic activity can be evaluated by starting the reaction with 50 mM sodium 4-(2-chlorophenyl)-2-oxobutanoate, sodium phenylpyruvate, or sodium 2-cyclopentyl-2-oxoacetate, 100 mM D(+)-glucose, 500 mM methylamine or ethylamine, a 100 mM phosphate buffer solution, 1 mM NADPH, a 0.002 unit/$\mu$L GDH solution, the polypeptide to be evaluated at 2.5 $\mu$M, and the compound D or compound D' at 20 v/v% or 20 w/v% in the reaction solution under conditions of 25°C or 37°C and pH 8 to 9, and determining a yield of an amino acid formed through reductive amination reaction after a lapse of 3 or 23 hours.

The catalytic activity is calculated on the basis of the amount of an amino acid (hereinafter, also referred to as a "target product") formed through reductive amination reaction. The amount of the target product formed can be measured using a liquid chromatograph mass spectrometer (LCMS). Specifically, the amount can be measured under conditions described in Examples mentioned later.

The polypeptide according to the present embodiment can be produced by, for example, a method comprising: culturing a recombinant cell which is a transformant in accordance with a routine method; and obtaining the polypeptide of interest by isolation or purification from the cultures thus obtained by culture. The produced polypeptide can also be used without isolation and purification or with only partial purification from the culture thus obtained by culture.

An isolation or purification method usually used can be used as a method for obtaining the polypeptide of interest from the cultures. Examples of the isolation or purification method include: methods exploiting solubility, such as salting out and solvent precipitation; methods exploiting difference in molecular weight, such as dialysis, ultrafiltration, gel filtration, and sodium dodecyl sulfate-polyacrylamide gel electrophoresis; methods exploiting electric charge, such as ion-exchange chromatography and hydroxylapatite chromatography; methods exploiting specific affinity, such as affinity chromatography; methods exploiting difference in hydrophobicity, such as reverse-phase high-performance liquid chromatography; and methods exploiting difference in isoelectric point, such as isoelectric focusing electrophoresis.

When the polypeptide of interest is present in the periplasm or cytoplasm of the cultured recombinant cell (*E. coli,* etc.), the cultures are subjected to a routine method such as filtration or centrifugation, bacterial cells or cells are collected and suspended in an appropriate buffer solution, and the cell walls and/or cell membranes of the cells, etc. are disrupted by a method, for example, ultrasonic wave, lysozyme, freezing and thawing, followed by a method such as centrifugation or filtration to obtain a fraction containing the polypeptide of interest. The fraction is solubilized using a surfactant such as Triton(TM)-X100 to obtain a crude solution. Then, the polypeptide of interest can be isolated and purified from the crude solution by use of the routine method given above.

The polypeptide according to the present embodiment can be prepared by the gene recombination technique known in the art as mentioned above and, in general, can be prepared as follows: a site-directed mutation is introduced to a

particular position and a particular amino acid by PCR with a plasmid containing the wild-type polypeptide gene as a template using primers therefor, and the template plasmid is digested with a restriction enzyme, followed by the transformation of *E. coli,* etc. to clone a plasmid harboring the mutation of interest.

In the case of introducing an amino acid mutation to the second amino acid, site-directed mutagenesis with the plasmid harboring the mutation at the particular site as a template using primers is subsequently repeated in the same manner as above to construct plasmid DNA encoding a 2-amino acid substitution mutant. The further introduction of an amino acid mutation can also be performed in the same manner as above.

An *E. coli* BL21 strain or the like is cotransformed with the prepared DNA and, for example, a plasmid pREP4 encoding lac repressor (Laci). The obtained transformed strain is isolated and cultured, followed by the induction of expression with IPTG. The obtained bacterial strain is then disrupted, and the polypeptide of interest is purified, for example, by applying a supernatant to an affinity column through the use of His tag.

Alternatively, the polypeptide can also be prepared by the following method: a gene having a nucleotide sequence encoding the polypeptide of interest is synthesized, and the nucleotide sequence is transferred to an expression vector, followed by protein expression and the purification of the polypeptide of interest using an affinity column through the use of various purification tags.

The method for producing the polypeptide according to the present embodiment is not limited to the methods mentioned above. Various gene manipulation techniques, such as point mutation techniques, gene synthesis techniques, and methods for introducing variant fragments using restriction enzymes, known in the art can be used. The expression is not limited by E. *coli,* and animal cells or a cell-free translation system may be used. The purification method is not limited by an affinity column using polyhistidine, and various peptide tags or purification columns can be used.

<Reducing agent>

The reducing agent may be one or more selected from the group consisting of reduced nicotinamide adenine dinucleotide phosphate (NADPH), oxidized nicotinamide adenine dinucleotide phosphate (NADP+), reduced nicotinamide adenine dinucleotide (NADH) and oxidized nicotinamide adenine dinucleotide (NAD+). In one embodiment, the reducing agent can be NADPH.

In addition, additives, such as glucose dehydrogenase (GDH) and glucose, formate dehydrogenase and formic acid, alcohol dehydrogenase and alcohol, amino acid dehydrogenase and amino acid, and organic acid dehydrogenase (such as malate dehydrogenase) and organic acid, which reduce NADP+ and NAD+ into NADPH and NADH, respectively, can be further added together therewith. That is, in the reaction step, various additives may be further present, and glucose and glucose dehydrogenase may be further present.

In the case of using, for example, NADPH, as the reducing agent, a reactant that reduces NADP+ (oxidized nicotinamide adenine dinucleotide phosphate) which may appear in the reaction solution into NADPH may be used because the amount of NADPH used can be reduced. Examples of the reactant include a combination of glucose and GDH (glucose dehydrogenase). In the case of using glucose and GDH together with NADPH, the amount of NADPH used can be reduced to a catalytic amount.

<Reaction step>

The reaction step is performed in a reaction solution comprising a polypeptide, a reducing agent, compound D, compound A and compound B, or compound C. The reaction solution may contain other components such as a solvent (excluding a compound that falls under compound D). The solvent may be water or a buffer solution. Examples of the buffer solution include a phosphate buffer solution, CHES (N-cyclohexyl-2-aminoethanesulfonic acid), Tris (trishydroxymethylaminomethane), and Bicine (N,N-di(2-hydroxyethyl)glycine). The reaction step may be performed under appropriate reaction conditions. "Appropriate reaction conditions" means conditions under which the polypeptide described above may exhibit catalytic activity.

The reaction step may be performed under temperature conditions of 0°C or higher and 50°C or lower. The lower limit of the temperature may be 0°C or higher, 10°C or higher, or 20°C or higher. The upper limit of the temperature may be 50°C or lower, 40°C or lower, or 30°C or lower. The temperature may be, for example, 0°C or higher and 50°C or lower, 10°C or higher and 40°C or lower, or 20°C or higher and 30°C or lower. In one aspect, the temperature may be 25°C or may be 16°C. The lower limit of the temperature may be 30°C or higher. The upper limit of the temperature may be 40°C or lower. The temperature may be, for example, 30°C or higher and 40°C or lower. The temperature may also be 37°C. The reaction temperature may be changed stepwise or continuously.

The reaction step may be performed under pH conditions of 7 or higher and 11 or lower. The lower limit of the pH may be 7 or higher, 7.5 or higher, 8 or higher, or 8.5 or higher. The upper limit of the pH may be 11 or lower, 10 or lower, 9.5 or lower, or 9 or lower. The pH may be, for example, between pH 7 and 11, between 8 and 10, or between 8.5 and 9.5. In one aspect, the pH may be 8 or may be 9. In this context, the pH refers to pH in the reaction solution at the start of reaction, and a

pH engineering during reaction is accepted. The pH engineering during reaction may be within 2, may be within 1.5, or may be within 1.

In the reaction step, the concentration of compound A at the start of reaction may be 10 mM or higher and 3000 mM or lower in the total amount of the reaction solution. The lower limit of the concentration of the compound A in the total amount of the reaction solution may be 10 mM or higher, 100 mM or higher, 300 mM or higher, 500 mM or higher, 700 mM or higher, 900 mM or higher, 1100 mM or higher, 1300 mM or higher, 1500 mM or higher, or 1700 mM or higher. The upper limit of the concentration of the compound A in the total amount of the reaction solution may be 3000 mM or lower, 2500 mM or lower, or 2000 mM or lower. The concentration of the compound A in the total amount of the reaction solution may be 10 mM or higher and 3000 mM or lower, or 500 mM or higher and 2000 mM or lower. In one aspect, the concentration of the compound A in the total amount of the reaction solution may be 500 mM or may be 1750 mM.

In the reaction step, the concentration of the compound B or the compound C at the start of reaction may be 0.001 mM or higher and 1000 mM or lower in the total amount of the reaction solution.

The concentration of the compound B at the start of reaction may be 10 mM or higher and 500 mM or lower in the total amount of the reaction solution. The lower limit of the concentration of the compound B at the start of reaction may be 10 mM or higher, 30 mM or higher, 50 mM or higher, 100 mM or higher, 150 mM or higher, 200 mM or higher, 250 mM or higher, 300 mM or higher, or 330 mM or higher in the total amount of the reaction solution. The upper limit of the concentration of the compound B at the start of reaction may be 500 mM or lower, 450 mM or lower, 400 mM or lower, or 380 mM or lower in the total amount of the reaction solution. The concentration of the compound B at the start of reaction may be 10 mM or higher and 500 mM or lower, or 300 mM or higher and 400 mM or lower in the total amount of the reaction solution. In one aspect, the concentration of the compound B at the start of reaction in the total amount of the reaction solution may be 50 mM or may be 350 mM.

The concentration of the compound C at the start of reaction may be 10 mM or higher and 500 mM or lower in the total amount of the reaction solution. The lower limit of the concentration of the compound C at the start of reaction may be 10 mM or higher, 30 mM or higher, 50 mM or higher, 100 mM or higher, 150 mM or higher, 200 mM or higher, 250 mM or higher, 300 mM or higher, or 330 mM or higher in the total amount of the reaction solution. The upper limit of the concentration of the compound C at the start of reaction may be 500 mM or lower, 450 mM or lower, 400 mM or lower, or 380 mM or lower in the total amount of the reaction solution. The concentration of the compound C at the start of reaction may be 10 mM or higher and 500 mM or lower, or 300 mM or higher and 400 mM or lower in the total amount of the reaction solution. In one aspect, the concentration of the compound C at the start of reaction in the total amount of the reaction solution may be 50 mM or may be 350 mM.

In the reaction step, the ratio of the molar number of the compound A to the molar number of the compound B at the start of reaction (molar number of compound A/ molar number of compound B) in the total amount of the reaction solution may be 1 or more. The lower limit of the ratio of the molar number of the compound A to the molar number of the compound B at the start of reaction in the total amount of the reaction solution may be 1 or more, 3 or more, 5 or more, 8 or more, or 9 or more. The upper limit of the ratio of the molar number of the compound A to the molar number of the compound B at the start of reaction in the total amount of the reaction solution may be 100 or less, 50 or less, 30 or less, 20 or less, or 15 or less. The ratio of the molar number of the compound A to the molar number of the compound B at the start of reaction in the total amount of the reaction solution may be 5 or 10.

If the compound D or compound D' is a liquid under conditions of 25°C and 1 atm, the reaction step may be performed under conditions in which the concentration of the compound D or compound D' in the reaction solution is 1 v/v% or more and 60 v/v% or less. The lower limit of the concentration of the compound D or compound D' in the reaction solution may be 1 v/v% or more, 5 v/v% or more, 10 v/v% or more, 20 v/v% or more, 30 v/v% or more, or 40 v/v% or more because the yield can be more improved. The upper limit of the concentration of the compound D or compound D' in the reaction solution may be 60 v/v% or less, 50 v/v% or less, or 40 v/v% or less because the yield can be more improved. In one aspect, the concentration of the compound D or compound D' in the reaction solution may be 20 v/v% or 10 v/v%. The concentration of the compound D or compound D' in the reaction solution means the concentration of the compound D or compound D' in the reaction solution at the start of reaction.

If the compound D or compound D' is a solid under conditions of 25°C and 1 atm, the reaction step may be performed under conditions in which the concentration of the compound D or compound D' in the reaction solution is 1 w/v% or more and 60 w/v% or less. The lower limit of the concentration of the compound D or compound D' in the reaction solution may be 1 w/v% or more, 5 w/v% or more, 10 w/v% or more, 20 w/v% or more, 30 w/v% or more, or 40 w/v% or more because the yield can be more improved. The upper limit of the concentration of the compound D or compound D' in the reaction solution may be 60 w/v% or less, 50 w/v% or less, or 40 w/v% or less because the yield can be more improved. In one aspect, the concentration of the compound D or compound D' in the reaction solution may be 20 w/v% or 10 w/v%. The concentration of the compound D or compound D' in the reaction solution means the concentration of the compound D or compound D' in the reaction solution at the start of reaction.

The reaction step may be performed under conditions in which the concentration of the polypeptide in the reaction solution is 0.1 μM or more and 10 μM or less. The lower limit of the concentration of the polypeptide in the reaction solution

may be 0.5 μM or more, 1.0 μM or more, 1.5 μM or more, 2.0 μM or more, 2.3 μM or more, or 2.4 μM or more. The upper limit of the concentration of the polypeptide in the reaction solution may be 10 μM or less, 5 μM or less, 4 μM or less, 3 μM or less, 2.7 μM or less, or 2.6 μM or less. In one aspect, the concentration of the polypeptide in the reaction solution may be 2.5 μM.

The reaction step may be performed under conditions in which the concentration of the reducing agent in the reaction solution is 0.1 mM or more and 100 mM or less. The lower limit of the concentration of the reducing agent in the reaction solution may be 0.1 mM or more, 0.2 mM or more, 0.4 mM or more, 0.6 mM or more, 0.8 mM or more, or 1 mM or more. The upper limit of the concentration of the reducing agent in the reaction solution may be 100 mM or less, 80 mM or less, 60 mM or less, 40 mM or less, 20 mM or less, or 10 mM or less. In one aspect, the concentration of the reducing agent in the reaction solution may be 1 mM or may be 2 mM.

The yield of the amino acid formed through the reaction in the presence of the compound D or compound D' may be 1.2 or more times, 1.3 or more times, 1.4 or more times, 1.5 or more times, 1.6 or more times, 1.7 or more times, or 1.8 or more times and 10 or less times, 9 or less times, 8 or less times, 7 or less times, 6 or less times, 5 or less times, 4 or less times, 3 or less times, or 2 or less times the yield of the amino acid formed in the reaction step under conditions of the absence of the compound D or compound D'. How many times the yield of the amino acid formed through the reaction in the presence of the compound D or compound D' is higher than the yield of the amino acid formed in the reaction step under conditions of the absence of the compound D or compound D' can be measured at any reaction temperature and at any reaction time, but can be determined by calculating the ratio of the yields of the target product under both conditions, for example, at 25°C or 37°C, after a lapse of 1 hour, 3 hours or 23 hours from the start of reaction. Specifically, this can be determined by dividing the yield of the amino acid formed through the reaction in the presence of the compound D or compound D' by the yield of the amino acid formed in the reaction step under conditions of the absence of the compound D or compound D'. It should be noted that it is preferably calculated from the yield at 25°C after a lapse of 3 hours from the start of reaction.

The yield of the amino acid formed in the reaction step may be 15% or more, 30% or more, 50% or more, 60% or more, 70% or more, 80% or more, 90% or more, 93% or more, or 95% or more, and may be 100% or less, 99% or less, 98% or less, 97% or less, or 96% or less.

The yield of the amino acid formed in the reaction step can be measured under the following conditions.

The reaction is started with 50 mM phenylpyruvic acid, 2-oxo-3-(p-tolyl)propanoic acid, or 2-cyclopentyl-2-oxo-acetic acid, 100 mM D(+)-glucose, 500 mM ammonia, methylamine or ethylamine, a 100 mM phosphate buffer solution, 1 mM NADPH, a 0.002 unit/μL GDH solution, the polypeptide at a concentration of 2.5 μM, and when the compound D or compound D' is used, the compound D or compound D' at 20 v/v% if the compound D or compound D' is a liquid under conditions of 25°C and 1 atm, or the compound D or compound D' at 20 w/v% if the compound D or compound D' is a solid under conditions of 25°C and 1 atm, in the reaction solution under conditions of 37°C and pH 8 to 9, and a yield of an amino acid formed through reductive amination reaction is determined after a lapse of 19 hours.

In the present specification, the start of reaction means, when the reaction step is a step of performing an intermolecular reductive amination reaction between compound A and compound B, a later point in time among points in time when the compound A, the compound B, a polypeptide disclosed in the present specification, and a reducing agent are added into the system, and means, when the reaction step is a step of performing an intramolecular reductive amination reaction of compound C, a later point in time among points in time when the compound C, a polypeptide disclosed in the present specification, and a reducing agent are added into the system.

The yield of the amino acid formed in the reaction step is calculated by the following method. A reaction solution and a separately prepared calibration curve sample (compound having the same structure or a structure that exhibits the same UV absorption wavelength as that of the target product) are subjected to LCMS analysis to obtain their respective UV charts or extracted ion chromatograms. A UV peak area or a MS peak area derived from the target product is obtained from the UV chart or the extracted ion chromatogram obtained from the reaction solution. Likewise, a UV peak area or a MS peak area of a preparation is obtained from the calibration curve sample. The concentration of the target product contained in the reaction solution is calculated from the UV peak area or the MS peak area on the basis of the correspondence relationship between the concentration of the preparation contained in the calibration curve sample and the UV peak area or the MS peak area. When the compound B or compound C in the reaction solution is completely converted to the target product, it is considered that the concentration of the target product in the reaction solution is equal to the concentration of the compound B or compound C in the reaction solution at the start of reaction. In actuality, the yield is calculated by dividing the concentration of the target product contained in the reaction solution by the concentration of the compound B or compound C in the reaction solution at the start of reaction.

The reaction yield can also be calculated by correcting the ratio of the absorbance coefficient of each compound according to the following expression (here, the reaction yield determined by this method is also referred to as the reaction conversion rate).

$$\text{Expression: Reaction conversion rate (\%)} = S_1/((S_2/8.0) + S_1) \times 100$$

In the expression, $S_1$ indicates the UV peak area of the target product. $S_2$ indicates the UV peak area of the compound B or the compound C.

[Production method of peptide compound]

The method for producing a peptide compound according to the present embodiment comprises the following steps:

(1) producing an amino acid by the production method of amino acid described above; and
(2) linking the amino acid to one or more selected from the group consisting of other amino acids and a peptide to produce a peptide compound.

The step of producing the peptide compound may be performed in an aqueous vehicle or in a mixed solution of an aqueous vehicle and an organic solvent. The aqueous vehicle may be water or a buffer solution. Examples of the buffer solution include phosphate buffer solutions, CHES (N-cyclohexyl-2-aminoethanesulfonic acid), Tris (trishydroxymethylaminomethane), and Bicine (N,N-di(2-hydroxyethyl)glycine). Examples of the organic solvent include dimethyl sulfoxide.

[Reductive amination reaction accelerator]

The reductive amination reaction accelerator according to the present embodiment may be a compound represented by the formula (1) described above, which accelerates the following reaction (i) or (ii):

(i) an intermolecular reductive amination reaction between compound A selected from the group consisting of a compound having an amino group and a salt thereof and compound B selected from the group consisting of a compound having a carbonyl group and a salt thereof;
(ii) an intramolecular reductive amination reaction of compound C selected from the group consisting of a compound having an amino group and a carbonyl group and a salt thereof.

To specific aspects of the reductive amination reaction accelerator, the aspects described in the Production method of amino acid can be applied.
The reductive amination reaction accelerator may be at least one selected from the group consisting of dimethyl sulfoxide, dimethylsulfone, dimethoxyethane, trimethylphosphine oxide, N,N-dimethylformamide, N,N-dimethylacetamide, tetramethylene sulfoxide, diethyl sulfoxide, methanol, and methylformamide, may be at least one selected from the group consisting of dimethyl sulfoxide, dimethylsulfone, and trimethylphosphine oxide, and may be dimethyl sulfoxide.
The present invention described above can be taken as a use of the compound represented by the formula (1) for accelerating the reductive amination reaction of (i) or (ii) described above. The present invention described above can also be taken as a compound represented by the formula (1) for use in accelerating the reductive amination reaction of (i) or (ii) described above. The present invention described above can also be taken as the use (application) of the compound represented by the formula (1) for the production of a reductive amination reaction accelerator for (i) or (ii) described above. To specific aspects of these, the aspects described in the Production method of amino acid can be applied.
The following abbreviations are used in this specification.

[Table 1]

| Abbreviation | Name |
|---|---|
| NMAADH | N-Methylamino acid dehydrogenase |
| FA | Formic acid |
| NADPH | Reduced nicotinamide adenine dinucleotide phosphate |
| 1 × TNG | 50 mM Trishydroxymethylaminomethane, 150 mM sodium chloride, 10 v/v% glycerol, pH 8.0 solution |
| GDH | Glucose dehydrogenase |
| PTFE | Polyvinylidene fluoride |
| DMSO | Dimethyl sulfoxide |

(continued)

| Abbreviation | Name |
|---|---|
| $DMSO_2$ | Dimethylsulfone |
| $Me_3PO$ | Trimethylphosphine oxide |
| DME | Dimethoxyethane |
| DMF | N,N-dimethylformamide |
| DMA | N,N-dimethylacetamide |
| TMSO | Tetramethylene sulfoxide |
| $TMSO_2$ | Tetramethylene sulfone/sulfolane |
| DESO | Diethyl sulfoxide |
| $DESO_2$ | Diethylsulfone |
| MeOH | Methanol |
| MFA | N-Methylformamide |
| THF | Tetrahydrofuran |
| NMP | N-Methyl-2-pyrrolidone |
| MeCN | Acetonitrile |

[Table 2]

| Abbreviation | Structural formula |
|---|---|
| MeHph(2-Cl) | 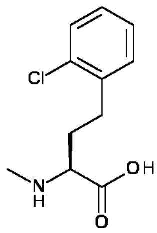 |
| EtPhe | 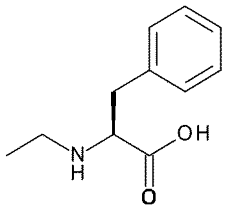 |
| MeGly(cPent) | 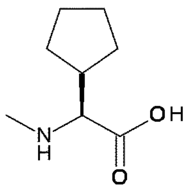 |
| Phe | 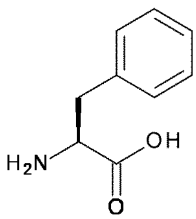 |

[Examples]

The present invention will be further illustrated with reference to Examples given below and however, is not limited by these examples.

The analysis conditions of LCMS are as described below.

LCMS method
Condition name: FA05
Apparatus: Waters Acquity UPLC/SQD
Column (I.D. x length (mm), particle size (μm)): Aldrich Ascentis Express C18 (2.1 x 50, 2.7)
Mobile phase: A) 0.1% FA $H_2O$, B) 0.1% FA MeCN
Gradient (A/B): 95/5 to 0/100 (1.0 min) → 0/100 (0.4 min)
Flow rate (ml/min): 1
Column temperature: 35°C
Wavelength: 210-400 nm PDA total
Condition name: TFA00
Apparatus: Waters H class
Column: (I.D. x length (mm), particle size (μm)): ACQUITY UPLC HSS T3 (2.1 x 50, 1.8)
Mobile phase: A) 0.05% TFA $H_2O$, B) 0.05% TFA MeCN
Gradient (A/B): 100/0 to 2/98 (5.0 min) → 2/98 (1.0 min) → 100/0 (0.01 min) → 100/0 (2.0 min)
Flow rate (ml/min): 0.5
Column temperature: 30°C
Wavelength: 197 nm
Condition name: TFA00-QDa
Apparatus: Waters H class/QDa
Column: (I.D. x length (mm), particle size (μm)): ACQUITY UPLC HSS T3 (2.1 x 50, 1.8)
Mobile phase: A) 0.05% TFA $H_2O$, B) 0.05% TFA MeCN
Gradient (A/B): 100/0 to 2/98 (5.0 min) → 2/98 (1.0 min) → 100/0 (0.01 min) → 100/0 (2.0 min)
Flow rate (ml/min): 0.5
Column temperature: 30°C
Wavelength: 197 nm

Reagents not specifically described were purchased from commercial suppliers.

Preparation Example 1: Preparation of wild-type NMAADH-C-His

A gene of sequence of SEQ ID NO: 1, C-terminally tagged with a linker sequence (GGSS), and a His tag sequence (HHHHHH) was synthesized and cloned into a vector for expression in E. coli. The expression vector was transformed into a BL21(DE3) E. coli strain, and cultured. The protein of interest was purified from ultrasonically disrupted cell supernatants using a nickel column. The protein solution was dialyzed (50 mM tris hydrochloric acid, 10% glycerol, 150 mM sodium chloride, pH 8.0) to give a final preparation (SEQ ID NO: 10, wild-type NMAADH-His).

SEQ ID NO: 1:
MRVPFTELQSLLQAIFQRHGCSEAVARVLAHNCASAQRDGAHSHGVFRMPGYVSTLAS
GWVDGQATPQVSDVAAGYVRVDAAGGFAQPALAAARELLVAKARSAGIAVLAIHNSH
HFAALWPDVEPFAEEGLVALSVVNSMTCVVPHGARKPLFGTNPIAFAAPCAEHDPIVFD
MATSAMAHGDVQIAARAGQQLPEGMGVDADGQPTTDPKAILEGGALLPFGGHKGSAL
SMMVELLAAALTGGHFSWEFDWSGHPGAKTPWTGQLIIVIDPGKAEGQRFAQRSRELV
EHMQAVGLTRMPGERRYREREVAEEEGVAVTEQELKGLKELLG

SEQ ID NO: 10:

MRVPFTELQSLLQAIFQRHGCSEAVARVLAHNCASAQRDGAHSHGVFRMPGYVSTLAS GWVDGQATPQVSDVAAGYVRVDAAGGFAQPALAAARELLVAKARSAGIAVLAIHNSH HFAALWPDVEPFAEEGLVALSVVNSMTCVVPHGARKPLFGTNPIAFAAPCAEHDPIVFD

MATSAMAHGDVQIAARAGQQLPEGMGVDADGQPTTDPKAILEGGALLPFGGHKGSAL SMMVELLAAALTGGHFSWEFDWSGHPGAKTPWTGQLIIVIDPGKAEGQRFAQRSRELV EHMQAVGLTRMPGERRYREREVAEEEGVAVTEQELKGLKELLGGGSSHHHHHH

Preparation Example 2: Preparation of W253H-SBP-His

A gene of amino acid sequence represented by SEQ ID NO: 8, in which X is His, C-terminally tagged with a streptavidin-binding peptide tag sequence (GTDEKTTGWRGGHVVEGLAGELEQLRARLEHHPQ), a linker sequence (GGS), and a His tag sequence (HHHHHH) was synthesized and cloned into a vector for expression in E. coli. This expression vector was transferred to a BL21 (DE3) *E. coli* strain (Novagen), which was then cultured at 18°C for 2 days using Overnight Express Instant TB Medium (Novagen) to express the protein of interest.

The obtained bacterial cells were recovered by centrifugation, and the bacterial cells were ultrasonically disrupted. The lysate was fractionated by centrifugation, and a supernatant fraction was purified by affinity chromatography using cOmplete His-Tag Purification Resin (Roche). After recovering a fraction containing the protein of interest, the sample was dialyzed against 50 mM Tris-hydrochloric acid (pH 8.0)/150 mM sodium chloride/10% glycerol and used as a final preparation (SEQ ID NO: 11, W253H).

Amino acid sequence represented by SEQ ID NO: 8, in which X is His:

MRVPFTELQSLLQAIFQRHGCSEAVARVLAHNCASAQRDGAHSHGVFRMPGYVSTLAS GWVDGQATPQVSDVAAGYVRVDAAGGFAQPALAAARELLVAKARSAGIAVLAIHNSH HFAALWPDVEPFAEEGLVALSVVNSMTCVVPHGARKPLFGTNPIAFAAPCAEHDPIVFD MATSAMAHGDVQIAARAGQQLPEGMGVDADGQPTTDPKAILEGGALLPFGGHKGSAL SMMVELLAAALTGGHFSWEFDHSGHPGAKTPWTGQLIIVIDPGKAEGQRFAQRSRELVE HMQAVGLTRMPGERRYREREVAEEEGVAVTEQELKGLKELLG

| SEQ ID NO: 11:

MRVPFTELQSLLQAIFQRHGCSEAVARVLAHNCASAQRDGAHSHGVFRMPGYVSTLAS GWVDGQATPQVSDVAAGYVRVDAAGGFAQPALAAARELLVAKARSAGIAVLAIHNSH HFAALWPDVEPFAEEGLVALSVVNSMTCVVPHGARKPLFGTNPIAFAAPCAEHDPIVFD MATSAMAHGDVQIAARAGQQLPEGMGVDADGQPTTDPKAILEGGALLPFGGHKGSAL SMMVELLAAALTGGHFSWEFDHSGHPGAKTPWTGQLIIVIDPGKAEGQRFAQRSRELVE HMQAVGLTRMPGERRYREREVAEEEGVAVTEQELKGLKELLGGTDEKTTGWRGGHV VEGLAGELEQLRARLEHHPQGGSHHHHHH

Preparation Example 3: Preparation of variant NMAADH-SBP-His

A gene of each variant enzyme sequence (SEQ ID Nos: 14 to 17) C-terminally tagged with a streptavidin-binding peptide tag sequence (GTDEKTTGWRGGHVVEGLAGELEQLRARLEHHPQ), a linker sequence (GGS), and a His tag sequence (HHHHHH) was synthesized and cloned into a vector for expression in E. coli. This expression vector was transferred to a BL21 (DE3) E. coli strain (Novagen), which was then cultured at 18°C for 2 days using Overnight Express Instant TB Medium

(Novagen) to express the protein of interest.

The obtained bacterial cells were recovered by centrifugation, and the bacterial cells were ultrasonically disrupted. The lysate was fractionated by centrifugation, and a supernatant fraction was purified by affinity chromatography using Ni Sepharose 6 Fast Flow (Cytiva). A fraction containing the protein of interest was recovered and used as a final preparation (SEQ ID NO: 18, M141V_W253Q_K260E-SBP-His; SEQ ID NO: 19, M141I_W253H_K260E-SBP-His; SEQ ID NO: 20, M141V_W253L_K260Q-SBP-His; SEQ ID NO: 21, M141V_W253L_K260E-SBP-His).

SEQ ID NO: 14:

MRVPFTELQSLLQAIFQRHGCSEAVARVLAHNCASAQRDGAHSHGVFRMPGYVSTLAS
GWVDGQATPQVSDVAAGYVRVDAAGGFAQPALAAARELLVAKARSAGIAVLAIHNSH
HFAALWPDVEPFAEEGLVALSVVNSVTCVVPHGARKPLFGTNPIAFAAPCAEHDPIVFD
MATSAMAHGDVQIAARAGQQLPEGMGVDADGQPTTDPKAILEGGALLPFGGHKGSAL
SMMVELLAAALTGGHFSWEFDQSGHPGAETPWTGQLIIVIDPGKAEGQRFAQRSRELVE
HMQAVGLTRMPGERRYREREVAEEEGVAVTEQELKGLKELLG

SEQ ID NO: 15:

MRVPFTELQSLLQAIFQRHGCSEAVARVLAHNCASAQRDGAHSHGVFRMPGYVSTLAS
GWVDGQATPQVSDVAAGYVRVDAAGGFAQPALAAARELLVAKARSAGIAVLAIHNSH
HFAALWPDVEPFAEEGLVALSVVNSITCVVPHGARKPLFGTNPIAFAAPCAEHDPIVFDM
ATSAMAHGDVQIAARAGQQLPEGMGVDADGQPTTDPKAILEGGALLPFGGHKGSALS
MMVELLAAALTGGHFSWEFDHSGHPGAETPWTGQLIIVIDPGKAEGQRFAQRSRELVE
HMQAVGLTRMPGERRYREREVAEEEGVAVTEQELKGLKELLG

SEQ ID NO: 16:

MRVPFTELQSLLQAIFQRHGCSEAVARVLAHNCASAQRDGAHSHGVFRMPGYVSTLAS
GWVDGQATPQVSDVAAGYVRVDAAGGFAQPALAAARELLVAKARSAGIAVLAIHNSH
HFAALWPDVEPFAEEGLVALSVVNSVTCVVPHGARKPLFGTNPIAFAAPCAEHDPIVFD
MATSAMAHGDVQIAARAGQQLPEGMGVDADGQPTTDPKAILEGGALLPFGGHKGSAL
SMMVELLAAALTGGHFSWEFDLSGHPGAQTPWTGQLIIVIDPGKAEGQRFAQRSRELVE
HMQAVGLTRMPGERRYREREVAEEEGVAVTEQELKGLKELLG

SEQ ID NO: 17:

MRVPFTELQSLLQAIFQRHGCSEAVARVLAHNCASAQRDGAHSHGVFRMPGYVSTLAS
GWVDGQATPQVSDVAAGYVRVDAAGGFAQPALAAARELLVAKARSAGIAVLAIHNSH
HFAALWPDVEPFAEEGLVALSVVNSVTCVVPHGARKPLFGTNPIAFAAPCAEHDPIVFD
MATSAMAHGDVQIAARAGQQLPEGMGVDADGQPTTDPKAILEGGALLPFGGHKGSAL
SMMVELLAAALTGGHFSWEFDLSGHPGAETPWTGQLIIVIDPGKAEGQRFAQRSRELVE
HMQAVGLTRMPGERRYREREVAEEEGVAVTEQELKGLKELLG

SEQ ID NO: 18:

MRVPFTELQSLLQAIFQRHGCSEAVARVLAHNCASAQRDGAHSHGVFRMPGYVSTLAS
GWVDGQATPQVSDVAAGYVRVDAAGGFAQPALAAARELLVAKARSAGIAVLAIHNSH
HFAALWPDVEPFAEEGLVALSVVNSVTCVVPHGARKPLFGTNPIAFAAPCAEHDPIVFD
MATSAMAHGDVQIAARAGQQLPEGMGVDADGQPTTDPKAILEGGALLPFGGHKGSAL
SMMVELLAAALTGGHFSWEFDQSGHPGAETPWTGQLIIVIDPGKAEGQRFAQRSRELVE
HMQAVGLTRMPGERRYREREVAEEEGVAVTEQELKGLKELLGGTDEKTTGWRGGHV
VEGLAGELEQLRARLEHHPQGGSHHHHHH

SEQ ID NO: 19:

MRVPFTELQSLLQAIFQRHGCSEAVARVLAHNCASAQRDGAHSHGVFRMPGYVSTLAS
GWVDGQATPQVSDVAAGYVRVDAAGGFAQPALAAARELLVAKARSAGIAVLAIHNSH
HFAALWPDVEPFAEEGLVALSVVNSITCVVPHGARKPLFGTNPIAFAAPCAEHDPIVFDM
ATSAMAHGDVQIAARAGQQLPEGMGVDADGQPTTDPKAILEGGALLPFGGHKGSALS
MMVELLAAALTGGHFSWEFDHSGHPGAETPWTGQLIIVIDPGKAEGQRFAQRSRELVE
HMQAVGLTRMPGERRYREREVAEEEGVAVTEQELKGLKELLGGTDEKTTGWRGGHV
VEGLAGELEQLRARLEHHPQGGSHHHHHH

SEQ ID NO: 20:

MRVPFTELQSLLQAIFQRHGCSEAVARVLAHNCASAQRDGAHSHGVFRMPGYVSTLAS
GWVDGQATPQVSDVAAGYVRVDAAGGFAQPALAAARELLVAKARSAGIAVLAIHNSH
HFAALWPDVEPFAEEGLVALSVVNSVTCVVPHGARKPLFGTNPIAFAAPCAEHDPIVFD
MATSAMAHGDVQIAARAGQQLPEGMGVDADGQPTTDPKAILEGGALLPFGGHKGSAL
SMMVELLAAALTGGHFSWEFDLSGHPGAQTPWTGQLIIVIDPGKAEGQRFAQRSRELVE
HMQAVGLTRMPGERRYREREVAEEEGVAVTEQELKGLKELLGGTDEKTTGWRGGHV
VEGLAGELEQLRARLEHHPQGGSHHHHHH

SEQ ID NO: 21:

MRVPFTELQSLLQAIFQRHGCSEAVARVLAHNCASAQRDGAHSHGVFRMPGYVSTLAS
GWVDGQATPQVSDVAAGYVRVDAAGGFAQPALAAARELLVAKARSAGIAVLAIHNSH
HFAALWPDVEPFAEEGLVALSVVNSVTCVVPHGARKPLFGTNPIAFAAPCAEHDPIVFD
MATSAMAHGDVQIAARAGQQLPEGMGVDADGQPTTDPKAILEGGALLPFGGHKGSAL
SMMVELLAAALTGGHFSWEFDLSGHPGAETPWTGQLIIVIDPGKAEGQRFAQRSRELVE
HMQAVGLTRMPGERRYREREVAEEEGVAVTEQELKGLKELLGGTDEKTTGWRGGHV
VEGLAGELEQLRARLEHHPQGGSHHHHHH

The following reagents were used in this Example.

[Table 3]

| Reagent name | CAS registration No. | Supplier |
|---|---|---|
| Sodium pyruvate | 113-24-6 | Tokyo Chemical Industry Co., Ltd. |
| 2-Chlorobenzaldehyde | 89-98-5 | Tokyo Chemical Industry Co., Ltd. |
| Palladium/carbon PE type | 7440-05-3 | NE Chemcat |
| Diphenyl sulfide | 139-66-2 | FUJIFILM Wako Pure Chemical Corp. |
| D(+)-Glucose | 50-99-7 | FUJIFILM Wako Pure Chemical Corp. |
| 40% aqueous methylamine solution | 74-89-5 | FUJIFILM Wako Pure Chemical Corp. |
| 33% aqueous ethylamine solution | 75-04-7 | Tokyo Chemical Industry Co., Ltd. |
| Phosphate buffer powder (1/15 mol/L, pH 7.4) | - | FUJIFILM Wako Pure Chemical Corp. |
| NADPH | 2646-71-1 | Oriental Yeast Co., Ltd. |
| GDH | 9028-53-9 | FUJIFILM Wako Pure Chemical Corp. |
| Sodium 2-cyclopentyl-2-oxoacetate | 223422-04-6 | Hebei Yaocheng Pharmaceutical Technology |
| N,N-Di(2-hydroxyethyl)glycine | 150-25-4 | Tokyo Chemical Industry Co., Ltd. |
| Ammonium hydroxide solution (28 to 30% $NH_3$) | 1336-21-6 | Sigma-Aldrich Japan G.K. |

The following reagents were pre-prepared and used as follows.

[Table 4]

| Reagent name | Preliminary preparation |
|---|---|
| Methylamine solution | The 40% aqueous methylamine solution was prepared into a 2.5 M solution of pH 8.5 using hydrochloric acid and ultrapure water. |
| Ethylamine solution | The 33% aqueous ethylamine solution was prepared into a 1.75 M solution of pH 8.5 using hydrochloric acid and ultrapure water. |
| Phosphate buffer solution | The phosphate buffer powder was prepared into a 1 M solution of pH 8.5 using an aqueous sodium hydroxide solution and ultrapure water. |
| NADPH solution | A 100 mM solution was prepared using ultrapure water. |
| GDH solution | A 0.02 unit/$\mu$L solution was prepared using 1 $\times$ TNG. |
| Ammonia solution | The ammonia concentration of the ammonium hydroxide solution (28 to 30% $NH_3$) was adjusted to 28%, and a 2.5 M solution of pH 8.5 was prepared using hydrochloric acid and ultrapure water. |
| N,N-Di(2- hydroxyethyl)glycine buffer solution | N,N-Di(2-hydroxyethyl)glycine was prepared into a 1 M solution of pH 9 using an aqueous sodium hydroxide solution and ultrapure water. |

Synthesis Example 1: Synthesis of starting material intermediate (E)-4-(2-chlorophenyl)-2-oxobut-3-enoic acid

[Formula 28]

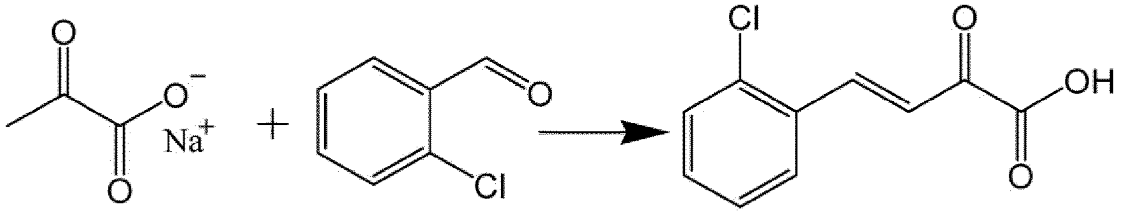

Sodium pyruvate (2.42 g, 0.022 mol) was added to a mixed solution of a 1 N aqueous sodium hydroxide solution (60 ml) and ethanol (3 ml) at 0°C, then 2-chlorobenzaldehyde (2.25 ml, 0.02 mol) was added dropwise thereto over 20 minutes or longer, and the reaction solution was stirred at 0°C for 2 hours. Then, water (160 ml) was added to the reaction solution, and the mixture was washed with toluene (100 ml). 5 N hydrochloric acid was added dropwise to the obtained aqueous layer at 0°C until pH became 1. The solution was stirred for 30 minutes. The resulting precipitate in the solution was recovered by filtration, and the precipitate was washed with cold water. The obtained wet solid was heated and dried under reduced pressure to give (E)-4-(2-chlorophenyl)-2-oxobut-2-enoic acid (2.7 g, 0.013 mol, 64% yield).

LCMS (ESI) m/z = 209.0 $(M - H)^-$
Retention time: 0.52 min (analysis condition FA05)

Synthesis Example 2: Synthesis of starting material sodium 4-(2-chlorophenyl)-2-oxobutanoate

[Formula 29]

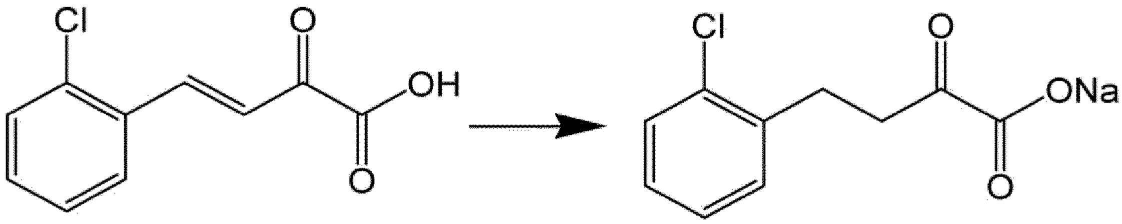

A suspension of (E)-4-(2-chlorophenyl)-2-oxobut-2-enoic acid (1.00 g, 4.75 mol) and palladium/carbon (0.20 g, 1.88 mol) in methanol (20 ml) and diphenyl sulfide (7.97 μl, 0.047 mmol) was stirred at room temperature for 1 hour in a hydrogen atmosphere. The reaction solution was filtered, and the obtained filtrate was then concentrated under a reduced pressure condition. A 1 N aqueous sodium hydroxide solution (15 ml) was added to the obtained residue, and the mixture was washed (twice) with toluene (15 ml). The aqueous layer was stirred at 0°C for 30 minutes. Then, the resulting precipitate in the solution was recovered by filtration, and the precipitate was washed with cold water. The obtained wet solid was heated and dried under reduced pressure to give sodium 4-(2-chlorophenyl)-2-oxobutanoate (847 mg, 3.61 mmol, 75% yield).

LCMS (ESI) m/z = 211.0 $(M - H)^-$
Retention time: 0.59 min (analysis condition FA05)

Evaluation Example 1-1: Evaluation 1-1 of additive in MeHph(2-Cl) synthesis

A mixed solution (pH 8 to 9) of sodium 4-(2-chlorophenyl)-2-oxobutanoate (final concentration: 50 mM), D(+)-glucose (final concentration: 100 mM), a methylamine solution (final concentration: 500 mM), a phosphate buffer solution (final concentration: 0.1 M), a NADPH solution (final concentration: 1 mM), a GDH solution (final concentration: 0.002 unit/μL), any of the additives described in Table 5 (20 v/v% based on the reaction solution for DMSO, NMP, formamide, 3-methyl-2-oxazolidinone, oxazolidinone, ethanol, dioxane, NMP, 2-propanol, MeCN, THF, $TMSO_2$, and 20 w/v% based on the reaction solution for $Me_3PO$, $DMSO_2$, $DESO_2$), wild-type NMAADH-C-His (final concentration: 2.5 μM), and ultrapure water was prepared and incubated at 25°C or 37°C for 3 hours. For an additive-free (0 v/v%) condition as a comparative condition, ultrapure water was added instead of the additives.

To the reaction solution thus incubated, a hydrochloric acid-containing DMSO solution was added in 19 times the amount of the reaction solution such that the final concentration of added hydrogen chloride was 300 mM. The obtained solution was allowed to pass through a 0.45 μm PTFE membrane filter (Cosmospin Filter H, Nacalai Tesque, Inc.) to

prepare a sample for LCMS analysis, which was then subjected to LCMS measurement (analysis condition: FA05). The UV peak area at 254 nm of the title compound MeHph(2-Cl) was calculated to determine a reaction yield. The results are shown in Table 5.

LCMS (ESI) m/z = 228.1 $(M + H)^+$
Retention time: 0.38 min (analysis condition FA05)

[Table 5]

| Additive | | Yield (%) at reaction temperature of 25°C | Yield (%) at reaction temperature of 37°C |
|---|---|---|---|
| Comparative Example 1 | Additive-free | 49 | 17 |
| Example 1 | DMSO | 95 | 97 |
| Example 2 | $DMSO_2$ | 98 | 89 |
| Example 3 | $Me_3PO$ | 93 | 99 |
| Comparative Example 2 | Ethanol | 46 | 4 |
| Comparative Example 3 | Dioxane | 44 | 9 |
| Comparative Example 4 | NMP | 11 | 3 |
| Comparative Example 5 | Formamide | 3 | 0.4 |
| Comparative Example 6 | 3-Methyl-2-oxazolidinone | 2 | - |
| Comparative Example 7 | $DESO_2$ | 1 | 1 |
| Comparative Example 8 | Oxazolidinone | 1 | - |
| Comparative Example 9 | 2-Propanol | 0.7 | 0.9 |
| Comparative Example 10 | MeCN | 0.1 | 1 |
| Comparative Example 11 | THF | 0.1 | 0.2 |
| Comparative Example 12 | $TMSO_2$ | 0.1 | 0.3 |

Evaluation Example 1-2: Evaluation 1-2 of additive in MeHph(2-Cl) synthesis

The same evaluation as in Evaluation Example 1-2 was performed except that any of additives (20 v/v%) described in Table 6 was used, and the temperature (reaction temperature) at the time of incubation for 3 hours was set to 25°C. The results are shown in Table 6.

[Table 6]

| Additive | | Yield (%) |
|---|---|---|
| Example 4 | DME | 93 |
| Example 5 | DMF | 83 |
| Example 6 | TMSO | 80 |
| Example 7 | DMA | 78 |
| Example 8 | DESO | 76 |
| Example 9 | MeOH | 71 |
| Example 10 | MFA | 62 |

Evaluation Example 2: Evaluation of added amount of additive

The effect of added amount on the reaction was evaluated by changing only the content of DMSO as an additive

from 0 v/v% to 40 v/v% for the reaction solution under the same reaction conditions as in Evaluation Example 1 at 25°C or 37°C. For 0 v/v% condition, ultrapure water was added instead of the additive. Yield after 3 hours of the reaction was evaluated. Samples for LCMS analysis were prepared in the same manner as in Evaluation Example 1, and LCMS measurements were performed (analysis condition: FA05). The UV peak area at 254 nm of the title compound MeHph(2-Cl) was calculated to determine a reaction yield. The results are shown in Table 7.

LCMS (ESI) m/z = 228.1 $(M + H)^+$
Retention time: 0.38 min (analysis condition FA05)

[Table 7]

| **DMSO added amount (v/v%)** | Yield (%) at reaction temperature of 25°C | Yield (%) at reaction temperature of 37°C |
|---|---|---|
| 0 | 49 | 17 |
| 10 | 97 | 97 |
| 20 | 95 | 97 |
| 30 | 98 | 97 |
| 40 | 98 | 98 |

Evaluation Example 3: Evaluation of adding effect of additive in EtPhe synthesis

Evaluation of the reaction was performed under the same conditions as the reaction in Evaluation Example 1, using phenylpyruvic acid (final concentration 50 mM) instead of sodium 4-(2-chlorophenyl)-2-oxobutanoate (final concentration 50 mM), using an ethylamine solution (final concentration 500 mM) instead of the methylamine solution (final concentration 500 mM), and adding DMSO as an additive to 20 v/v% of the total reaction solution volume. A sample for LCMS analysis was prepared in the same manner as in Evaluation Example 1 and then subjected to LCMS measurement (analysis condition: FA05). The UV peak area at 254 nm of the title compound EtPhe was calculated to determine a reaction yield. In this respect, the reaction yield was determined after 3 and 23 hours under two conditions of reaction temperatures of 25°C and 37°C. The results are shown in Table 8.

LCMS (ESI) m/z = 194.1 $(M + H)^+$
Retention Time : 0.30 min (analysis condition FA05)

[Table 8]

| **Additive** | Yield (%) at reaction temperature of 25°C | | Yield (%) at reaction temperature of 37°C | |
|---|---|---|---|---|
| | **3 hours** | **23 hours** | **3 hours** | **23 hours** |
| Additive-free | 10 | 48 | 12 | 47 |
| DMSO | 27 | 72 | 31 | 72 |

Evaluation Example 4: Evaluation of adding effect of additive in MeGly(cPent) synthesis

Evaluation of the reaction was performed under the same conditions as the reaction in Evaluation Example 1, using sodium 2-cyclopentyl-2-oxoacetate (final concentration 50 mM) instead of sodium 4-(2-chlorophenyl)-2-oxobutanoate (final concentration 50 mM), and adding DMSO as an additive to 20 v/v% of the total reaction solution volume. The evaluation of the reaction was determined by the reaction yield after 24 hours under two conditions of reaction temperature of 25°C and 37°C. To the reaction solution thus incubated, a hydrochloric acid solution was added in 7 times the amount of the reaction solution such that the final concentration of added hydrogen chloride was 200 mM. The obtained solution was allowed to pass through a 0.45 μm PTFE membrane filter (Cosmospin Filter H, Nacalai Tesque, Inc.) to prepare a sample for LCMS analysis, which was then subjected to LCMS measurement (analysis condition: FA05). The mass peak area at 158.1 $(M + H)^+$ of the title compound MeGly(cPent) was calculated and the reaction yield was determined. The results are shown in Table 9.

LCMS (ESI) m/z = 158.1 $(M + H)^+$
Retention Time : 0.23 min (analysis condition FA05)

[Table 9]

| Additive | Yield (%) at reaction temperature of 25°C | Yield (%) at reaction temperature of 37°C |
|---|---|---|
| Additive-free | 17 | 29 |
| DMSO | 67 | 68 |

Evaluation Example 5: Reaction example using variant enzyme NMAADH (W253H)

Evaluation of the reaction was performed under the same conditions as the reaction in Evaluation Example 1, using a W253H-SBP-His solution prepared in Preparation Example 2 instead of wild-type NMAADH-C-His. The evaluation of the reaction was determined after 3 hours under two conditions of reaction temperature of 25°C and 37°C. A sample for LCMS analysis was prepared in the same manner as in Evaluation Example 1 and then subjected to LCMS measurement (analysis condition: FA05). The UV peak area at 254 nm of the title compound MeHph(2-Cl) was calculated to determine a reaction yield. The results are shown in Table 10.

LCMS (ESI) m/z = 228.1 $(M + H)^+$
Retention time: 0.38 min (analysis condition FA05)

[Table 10]

| Additive | Yield (%) at reaction temperature of 25°C | Yield (%) at reaction temperature of 37°C |
|---|---|---|
| Additive-free | 38 | 14 |
| DMSO | 98 | 99 |

Evaluation Example 6: Evaluation 2 of additive in MeHph(2-Cl) synthesis

A mixed solution of sodium 4-(2-chlorophenyl)-2-oxobutanoate (final concentration: 10 mM), D(+)-glucose (final concentration: 20 mM), a methylamine solution (final concentration: 100 mM), a phosphate buffer solution (final concentration: 0.1 M), a NADPH solution (final concentration: 1 mM), a GDH solution (final concentration: 0.002 unit/μL), additive DMSO (20 v/v%), wild-type NMAADH-C-His (final concentration 2.5 μM), and ultrapure water was prepared and incubated at 25°C for 1 hour. For an additive-free (0 v/v%) condition as a comparative condition, ultrapure water was added instead of the additive.

To the reaction solution thus incubated, a hydrochloric acid-containing DMSO solution was added in 7 times the amount of the reaction solution such that the final concentration of added hydrogen chloride was 300 mM. The obtained solution was allowed to pass through a 0.45 μm PTFE membrane filter (Cosmospin Filter H, Nacalai Tesque, Inc.) to prepare a sample for LCMS analysis, which was then subjected to LCMS measurement (analysis condition: FA05). The UV peak area at 254 nm of the title compound MeHph(2-Cl) was calculated to determine a reaction yield. The results are shown in Table 11.

LCMS (ESI) m/z = 228.1 $(M + H)^+$
Retention time: 0.38 min (analysis condition FA05)

[Table 11]

| Additive | Yield (%) |
|---|---|
| Additive-free | 48 |
| DMSO | 62 |

Evaluation Example 7: Evaluation of additive in MeGly(cPent) synthesis using variant enzyme NMAADH (W253H)

Sodium 2-cyclopentyl-2-oxoacetate (final concentration: 100 mM), D(+)-glucose (final concentration: 200 mM), methylamine hydrochloride (final concentration: 500 mM), NADPH (final concentration: 2.0 mM), and N,N-di(2-hydroxyethyl)glycine (final concentration: 100 mM) were dissolved in distilled water. To the solution, the additive DMSO (0 to 40 v/v%) was added, followed by pH adjustment to 8.8 to 8.9 with a 5 M aqueous sodium hydroxide solution. A GDH solution (0.10 units/μL, 1 × TNG solution, final concentration: 0.0040 units/μL) and a variant NMAADH W253H-SBP-His solution (0.67 mM, final concentration: 5.0 μM) were added, and the mixture was incubated at 25°C for 24 hours.

During the incubation, 50 μL of the reaction solution was sampled, and 750 μL of methanol and 200 μL of 1 M hydrochloric acid were added to the sampled solution. The obtained solution was filtered through a 0.5 μm PTFE membrane filter (DISMIC, ADVANTEC) to prepare a sample for LC analysis, which was then subjected to LC measurement (analysis condition: TFA00). A UV peak area at 197 nm was measured by LC, and the ratio of the absorbance coefficient of each compound was corrected according to the expression X given below to calculate a reaction conversion rate. Changes in the reaction conversion rate are shown in Table 12.
Retention time: 1.1 min (analysis condition :TFA00)

The obtained solution was measured by LCMS (analysis condition: TFA00-QDa) to confirm a MS peak of MeGly(cPent).

Retention time: 1.5 min (analysis condition :TFA00-QDa)
LCMS (ESI) m/z = 158.1 $(M + H)^+$

$$\text{Expression X: Reaction conversion rate (\%)} = S_{1a} / ((S_{2a} / 8.0) + S_{1a}) \times 100$$

In the expression X, $S_{1a}$ represents the UV peak area of MeGly(cPent). $S_{2a}$ represents the UV peak area of sodium 2-cyclopentyl-2-oxoacetate.

[Table 12]

| DMSO (v/v%) | Reaction conversion rate (%) | | |
|---|---|---|---|
| | 3h | 9h | 24h |
| 0 | 26 | 64 | 97 |
| 10 | 37 | 79 | 99 |
| 20 | 47 | 86 | 99 |
| 30 | 47 | 85 | 95 |
| 40 | 47 | 84 | 97 |

Evaluation Example 8: Evaluation of additive in Phe synthesis

Evaluation of the reaction was performed under the same conditions as the reaction in Evaluation Example 1, using phenylpyruvic acid (final concentration 50 mM) instead of sodium 4-(2-chlorophenyl)-2-oxobutanoate (final concentration 50 mM), using an ammonia solution (final concentration 500 mM) instead of the methylamine solution (final concentration 500 mM), and adding DMSO as an additive to 20 v/v% of the total reaction solution volume. For an additive-free (0 v/v%) condition as a comparative condition, ultrapure water was added instead of the additive. A sample for LCMS analysis was prepared in the same manner as in Evaluation Example 1 and then subjected to LCMS measurement (analysis condition: FA05). The UV peak area at 254 nm of the title compound Phe was calculated to determine a reaction yield. In this respect, the reaction yield was determined after 3 and 5 hours under two conditions of reaction temperatures of 25°C and 37°C. The results are shown in Table 13.

LCMS (ESI) m/z = 166.1 $(M + H)^+$
Retention Time : 0.27 min (analysis condition FA05)

[Table 13]

| Additive | Yield (%) at reaction temperature of 25°C | | Yield (%) at reaction temperature of 37°C | |
|---|---|---|---|---|
| | **3 hours** | **5 hours** | **3 hours** | **5 hours** |
| Additive-free | 67 | 71 | 56 | 77 |
| DMSO | 90 | 91 | 78 | 89 |

Evaluation Example 9: Evaluation of additive in Phe synthesis using variant enzyme NMAADH (W253H)

A mixed solution (pH 8 to 9) of phenylpyruvic acid (final concentration: 50 mM), D(+)-glucose (final concentration: 100 mM), an ammonia solution (final concentration: 500 mM), a N,N-di(2-hydroxyethyl)glycine buffer solution (final concentration: 0.1 M), a NADPH solution (final concentration: 1 mM), a GDH solution (final concentration: 0.002 unit/μL), the additive DMSO (20 v/v% based on the reaction solution), variant NMAADH W253H-SBP-His (final concentration: 2.5 μM), and ultrapure water was prepared and incubated at 25°C, followed by the evaluation of the reaction. For an additive-free (0 v/v%) condition as a comparative condition, ultrapure water was added instead of the additive. A sample for LCMS analysis was prepared in the same manner as in Evaluation Example 1 and then subjected to LCMS measurement (analysis condition: FA05). The UV peak area at 254 nm of the title compound Phe was calculated to determine a reaction yield. In this respect, the reaction yield was determined after 3 and 5 hours at the reaction temperature of 25°C. The results are shown in Table 14.

LCMS (ESI) m/z = 166.1 $(M + H)^+$
Retention Time : 0.27 min (analysis condition FA05)

[Table 14]

| Additive | Yield (%) at reaction temperature of 25°C | |
|---|---|---|
| | **3 hours** | **5 hours** |
| Additive-free | 32 | 68 |
| DMSO | 50 | 86 |

Evaluation Example 10: Evaluation 3 of additive in MeHph(2-Cl) synthesis

A mixed solution of sodium 4-(2-chlorophenyl)-2-oxobutanoate (final concentration: 50 mM), D(+)-glucose (final concentration: 100 mM), a methylamine solution (final concentration: 500 mM), a N,N-di(2-hydroxyethyl)glycine buffer solution (final concentration 0.1 M), a NADPH solution (final concentration: 1 mM), a GDH solution (final concentration: 0.002 unit/μL), the additive DMSO (20 v/v%), variant NMAADH W253H-SBP-His solution (final concentration: 2.5 μM), and ultrapure water was prepared and incubated at 16°C for 1 hour. For an additive-free (0 v/v%) condition as a comparative condition, ultrapure water was added instead of the additive.

To the reaction solution thus incubated, a hydrochloric acid-containing DMSO solution was added in 19 times the amount of the reaction solution such that the final concentration of added hydrogen chloride was 300 mM. The obtained solution was allowed to pass through a 0.45 μm PTFE membrane filter (Cosmospin Filter H, Nacalai Tesque, Inc.) to prepare a sample for LCMS analysis, which was then subjected to LCMS measurement (analysis condition: FA05). The UV peak area at 254 nm of the title compound MeHph(2-Cl) was calculated to determine a reaction yield. The results are shown in Table 15.

LCMS (ESI) m/z = 228.1 $(M + H)^+$
Retention time: 0.38 min (analysis condition FA05)

[Table 15]

| **Additive** | Yield (%) at reaction temperature of 16°C |
|---|---|
| Additive-free | 57 |

(continued)

| Additive | Yield (%) at reaction temperature of 16°C |
|---|---|
| DMSO | 83 |

Evaluation Example 11: Evaluation of additive in EtPhe synthesis using variant enzyme NMAADH

A mixed solution (pH 8 to 9) of phenylpyruvic acid (final concentration: 50 mM), D(+)-glucose (final concentration: 100 mM), an ethylamine solution (final concentration: 500 mM), a N,N-di(2-hydroxyethyl)glycine buffer solution (final concentration: 0.1 M), a NADPH solution (final concentration: 1 mM), a GDH solution (final concentration: 0.002 unit/μL), the additive DMSO (20 v/v% based on the reaction solution), each variant NMAADH-SBP-His solution (final concentration: 2.5 μM), and ultrapure water was prepared and incubated at 37°C, followed by the evaluation of the reaction. It should be noted that five types of variant NMAADH-SBP-His solution (M141V_W253Q_K260E-SBP-His solution, M141I_W253H_K260E-SBP-His solution, M141V_W253L_K260Q-SBP-His solution, M141V_ W253L_K260E-SBP-His solution, and W253H-SBP-His solution) prepared in Preparation Examples 2 and 3 were used each for evaluation. For an additive-free (0 v/v%) condition as a comparative condition, ultrapure water was added instead of the additive. A sample for LCMS analysis was prepared in the same manner as in Evaluation Example 1 and then subjected to LCMS measurement (analysis condition: FA05). The UV peak area at 254 nm of the title compound EtPhe was calculated to determine a reaction yield. In this respect, the reaction yield was determined after 2, 4 and 24 hours at the reaction temperature of 37°C. The results are shown in Tables 16 to 20.

LCMS (ESI) m/z = 194.1 $(M + H)^+$
Retention Time : 0.30 min (analysis condition FA05)

[Table 16]

| M141V_W253Q_K260E-SBP-His | | | |
|---|---|---|---|
| Additive | Yield (%) | | |
| | 2 hours | 4 hours | 24 hours |
| Additive-free | 6 | 10 | 43 |
| DMSO | 22 | 36 | 95 |

[Table 17]

| M141I_W253H_K260E-SBP-His | | | |
|---|---|---|---|
| Additive | Yield (%) | | |
| | 2 hours | 4 hours | 24 hours |
| Additive-free | 3 | 5 | 19 |
| DMSO | 10 | 18 | 53 |

[Table 18]

| M141V_W253L_K260Q-SBP-His | | | |
|---|---|---|---|
| Additive | Yield (%) | | |
| | 2 hours | 4 hours | 24 hours |
| Additive-free | 2 | 3 | 17 |
| DMSO | 8 | 12 | 57 |

[Table 19]

| M141V_W253L_K260E-SBP-His | | | |
|---|---|---|---|
| **Additive** | **Yield (%)** | | |
| | **2 hours** | **4 hours** | **24 hours** |
| Additive-free | 3 | 5 | 23 |
| DMSO | 10 | 14 | 65 |

[Table 20]

| W253H-SBP-His | | | |
|---|---|---|---|
| **Additive** | **Yield (%)** | | |
| | **2 hours** | **4 hours** | **24 hours** |
| Additive-free | 20 | 42 | 87 |
| DMSO | 57 | 80 | 95 |

Evaluation Example 12: Evaluation of additive in (S)-3-(4,4-difluorocyclohexyl)-2-(methylamino)propanoic acid synthesis using variant enzyme NMAADH

A mixed solution of 3-(4,4-difluorocyclohexyl)-2-oxopropanoic acid (final concentration: 50 mM), D(+)-glucose (final concentration: 100 mM), a methylamine solution (final concentration: 500 mM), a N,N-di(2-hydroxyethyl)glycine buffer solution (final concentration 0.1 M), a NADPH solution (final concentration: 1 mM), a GDH solution (final concentration: 0.002 unit/μL), the additive DMSO (20 v/v%), a variant NMAADH W253H-SBP-His solution (final concentration: 2.5 μM), and ultrapure water was prepared and incubated at 37°C for 1 hour. For an additive-free (0 v/v%) condition as a comparative condition, ultrapure water was added instead of the additive.

To the reaction solution thus incubated, a hydrochloric acid solution was added in 3 times the amount of the reaction solution such that the final concentration of added hydrogen chloride was 200 mM. The obtained solution was allowed to pass through a 0.45 μm PTFE membrane filter (Cosmospin Filter H, Nacalai Tesque, Inc.) to prepare a sample for LCMS analysis, which was then subjected to LCMS measurement (analysis condition: FA05). The mass peak area at 222.1 $(M + H)^+$ of the title compound (S)-3-(4,4-difluorocyclohexyl)-2-(methylamino)propanoic acid was calculated and the reaction yield was determined. The results are shown in Table 21.

LCMS (ESI) m/z = 222.1 $(M + H)^+$
Retention Time : 0.36 min (analysis condition FA05)

[Table 21]

| **Additive** | Yield (%) at reaction temperature of 37°C |
|---|---|
| Additive-free | 48 |
| DMSO | 64 |

Synthesis Example 3: Synthesis of (2S,3S)-2-amino-3-phenyl-butanoic acid using variant enzyme W253H-SBP-His and additive (dimethyl sulfoxide)

In this example, the following reagents were used. The reagents not listed were those in Table 3.

[Table 22]

| Reagent name | CAS registration No. | Supplier |
|---|---|---|
| Sodium 2-oxo-3-phenylbutanoic acid | 125116-72-5 | Chemieliva Pharmaceutical |
| 5mol/L Hydrochloric acid | 7647-01-0 | FUJIFILM Wako Pure Chemical Corp. |

(continued)

| Reagent name | CAS registration No. | Supplier |
|---|---|---|
| (2S,3R) -2-amino-3-phenylbutanoic acid hydrochloride | 143251-58-5 | J&W Pharmlab |
| (2S,3S) -2-amino-3-phenylbutanoic acid hydrochloride | 53331-55-8 | J&W Pharmlab |
| (2R,3R) -2-amino-3-phenylbutanoic acid hydrochloride | 143251-57-4 | J&W Pharmlab |

$^{1}$H-NMR analysis was performed using the following apparatus and deuterated solvent. Apparatus: AVANCE III HD 400 SMART-BBFO probe (400 MHz, Bruker) Deuterated solvent: Trifluoroacid-$d_1$

The following abbreviations are used in this Example.

[Table 23]

| Abbreviation | Compound |
|---|---|
| (I) | Compound obtained by the reaction: (2S,3S)-2-amino-3-phenylbutanoic acid |
| (II) | Standard compound: (2S,3R)-2-amino-3-phenylbutanoic acid hydrochloride |
| (III) | Standard compound: (2S,3S)-2-amino-3-phenylbutanoic acid hydrochloride |
| (IV) | Standard Compound: (2R,3R)-2-amino-3-phenylbutanoic acid hydrochloride |

[Formula 30]

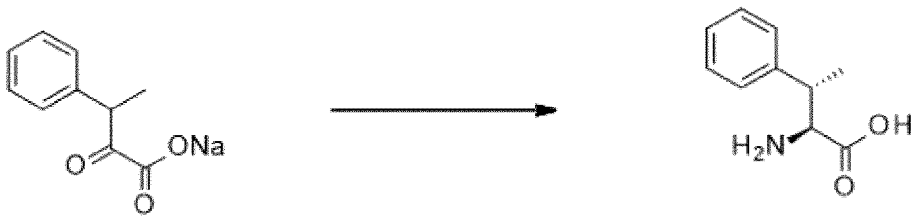

Sodium 2-oxo-3-phenylbutanoic acid

(2S,3S)-2-Amino-3-phenylbutanoic acid (I)

A mixed solution of sodium 2-oxo-3-phenylbutanoate (final concentration 50 mM), D(+)-glucose (final concentration 100 mM), an ammonia solution (final concentration 500 mM), a N,N-di(2-hydroxyethyl)glycine buffer solution (final concentration 0.1 M), a NADPH solution (final concentration 1 mM), a GDH solution (final concentration 0.002 unit/μL), the additive DMSO (20 vol%), the variant enzyme W253H-SBP-His (final concentration 20 μM) prepared in Preparation Example 2, and ultrapure water was prepared and incubated at 37°C for 47 hours.

600 μL of 5 mol/L hydrochloric acid was added to the reaction solution after the incubation, and the obtained solution was purified by reversed-phase silica gel column chromatography (0. 1% formic acid aqueous solution / 0. 1% formic acid acetonitrile solution = 100/0 to 95/5) to obtain (2S, 3S) - 2-amino-3-phenyl-butanoic acid (I) (16.5 mg, 18%).

LCMS(ESI) m/z=180.1$(M+H)^+$
Retention Time : 0.31 min (Analysis Condition FA05)
$^{1}$H-NMR (400 MHz, Trifluoroacid-$d_1$, 298K) δ 7.32-7.21 (3H, m), 7.18-7.16 (2H, m), 4.94 (1H, d, J = 5.6 Hz), 3.55-3.48 (1H, m), 1.45 (1H, d, J = 6.8 Hz)

$^{1}$H-NMR and chiral HPLC analysis data of the compound (2S, 3S) - 2-amino-3-phenyl-butanoic acid (I) obtained by the reaction were compared with those of the standard compound (2S, 3R) - 2-amino-3-phenyl-butanoic acid hydrochloride (II), (2S, 3S) - 2-amino-3-phenyl-butanoic acid hydrochloride (III), and (2R, 3R) - 2-amino-3-phenyl-butanoic acid hydrochloride (IV) which were purchased, to specify the steric structure of the obtained compound.

The structure of the compound (I) obtained in the reaction was specified as (2S,3S)-2-amino-3-phenylbutanoic acid from the comparison result of characteristic proton peaks at the 2-and 3-positions in the $^{1}$H-NMR analysis (FIG. 1) and

the comparison result of chiral HPLC analysis (FIG. 1 and Table 24).

Chiral HPLC analysis condition was as follows.
Apparatus: SHIMADZU Nexera X3
Column (I.D. x Length (mm), Particle Size (μm)) : DAICEL CORPORATION CHIRALPAK ZWIX (+) (3.0 x 150, 3.0)
Mobile phase: MeOH : MeCN : $H_2O$ (49 : 49 : 2, v / v) solution containing 50 mM formic acid and 25 mM diethylamine.
Elution Method: Isocratic
Analysis time: 10 minutes.
Flow rate (ml/min): 0.5
Column temperature: 25°C
Wavelength: 254 nm

(V) in FIG. 2 and Table 24 was prepared by mixing the purchased standard compounds (III) and (IV) at a ratio of (III) : (IV) = 7 : 3.

[Table 24]

| | PDA Ch1 254nm | | | |
|---|---|---|---|---|
| | Peak # | Retention time | Area | Area % |
| (I) | 1 | 4.103 | 96765 | 100.000 |
| | Total | | 96765 | 100.000 |
| | PDA Ch1 254nm | | | |
| | Peak # | Retention time | Area | Area % |
| (V) | 1 | 4.082 | 133994 | 71.853 |
| | 2 | 5.344 | 52490 | 28.147 |
| | Total | | 186484 | 100.000 |

Synthesis Example 4: Synthesis of (S)-3,3-dimethyl-2-(methylamino)butyric acid using variant enzyme W253H-SBP-His and additive (dimethyl sulfoxide)

LC/MS was carried out under the following analysis conditions.

HPLC method
Apparatus: Waters AQUITY UPLC H-Class/QDa
Column: ACQUITY UPLC HSS T3 2.1x50mm, 1.8 μ m
Solvents: A) 0.05% TFA-$H_2O$, B) 0.05% TFA-$CH_3CN$
Gradient: 0% B (0 min) → 98% B (5.0 min) → 98% B (6.0 min) → 0% B (6.01 min) → 0% B (8.0 min)
Flow rate: 0.5mL/min
Injection volume: 0.5 μl
Temperature: 30°C
Wavelength: 197 nm

In this example, reagents described in the following table were used. The reagents not listed were those in Table 3.

[Table 25]

| Reagent name | CAS registration No. | Supplier |
|---|---|---|
| 3,3-Dimethyl-2-oxobutyric acid | 815-17-8 | Tokyo Chemical Industry Co., Ltd. |
| (S)-2-Amino-3,3-dimethylbutyric acid | 20859-02-3 | Tokyo Chemical Industry Co., Ltd. |
| Methylamine hydrochloride | 593-51-1 | Tokyo Chemical Industry Co., Ltd. |
| 50 w/v% Aqueous sodium hydroxide solution | 1310-73-2 | FUJIFILM Wako Pure Chemical Corp. |
| Oxidized nicotinamide adenine dinucleotide phosphate | 1184-16-3 | Oriental Yeast Co., Ltd. |

(continued)

| Reagent name | CAS registration No. | Supplier |
|---|---|---|
| D-Glucose dehydrogenase (CDX-901) | 9028-53-9 | Codexis |
| Concentrated hydrochloric acid | 7647-01-0 | FUJIFILM Wako Pure Chemical Corp. |

[Formula 31]

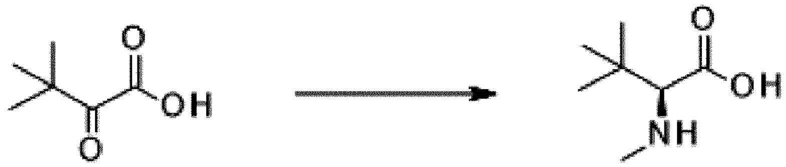

Oxidized nicotinamide adenine dinucleotide phosphate (50 mg) was dissolved in 0.4 mol/L of N, N-di (2-hydroxyethyl) glycine solution (1.0 mL, pH9.0) to prepare an oxidized nicotinamide adenine dinucleotide phosphate solution (solution A).

D-Glucose dehydrogenase (50 mg) was dissolved in 0.4 mol/L of N, N-di (2-hydroxyethyl) glycine solution (1.0 mL, pH9.0) to prepare D-glucose dehydrogenase solution (solution B).
3, 3-Dimethyl-2-oxobutyric acid (50 mg, 0.38 mmol), N, N-di (2-hydroxyethyl) glycine (130 mg, 0.77 mmol), methylamine hydrochloride (130 mg, 1.9 mmol), and D-glucose (140 mg, 0.77 mmol) were dissolved in distilled water (0.80 mL) and dimethyl sulfoxide (0.20 mL). A 50w/v% sodium hydroxide aqueous solution (88 μ L) was added to adjust the pH to 9.0 to prepare a substrate solution (solution C).
Solution A (59 μL, 3.8 μmol) and Solution B (10 μL, 1.0 wt%) were added to Solution C, the modified enzyme solution (1.0 to 100 wt%, Each was added as a 73.3 mg/ml solution) was added thereto, the mixture was stirred at an external temperature of 25 °C. for 17 hours, and then shaken at an external temperature of 37 °C for 24 hours.

The yield of (S) -3, 3-dimethyl-2 - (methylamino) butyric acid in the reaction solution was calculated from the UV absorption peak area at 197 nm in LC/MS using (S) - 2-amino-3, 3-dimethylbutyric acid as a standard. The results are shown in Table 26.

[Table 26]

| Amount of variant enzyme (wt%) | Yield (%) | |
|---|---|---|
| | 25°C, 15 hours | 37°C, 24 hours |
| 1.0 | 0 | 0 |
| 10 | 0 | 1 |
| 100 | 1 | 4 |

## Claims

**1.** A method for producing an amino acid, comprising a step of performing the following reaction (i) or (ii) in the presence of a polypeptide comprising an amino acid sequence having 90% or higher identity to an amino acid sequence represented by SEQ ID NO: 1, a reducing agent, and compound D represented by the following formula (1):

(i) an intermolecular reductive amination reaction between compound A selected from the group consisting of a compound having an amino group and a salt thereof and compound B selected from the group consisting of a compound having a carbonyl group and a salt thereof;
(ii) an intramolecular reductive amination reaction of compound C selected from the group consisting of a compound having an amino group and a carbonyl group and a salt thereof:

[Formula 1]

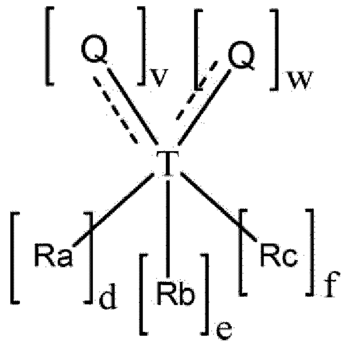

(1)

wherein in the formula (1),

v and w each independently represent 0 or 1,
at least one of v and w represents 1,
T represents a carbon atom, a phosphorus atom, or a sulfur atom,
a functional group represented by the following formula (Ia):

[Formula 2]

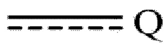

(1 a)

represents =O, -ORd, or a hydroxy group,

when each of v and w is 1, two functional groups represented by a plurality of formulas (1a) are the same or different,
Ra, Rb, and Rc each independently represent a hydrogen atom, a $C_1$ to $C_3$ alkyl group, an alkylamino group, or $-CH_2-ORd$,
at least any two of Ra, Rb, and Rc are optionally linked to each other to form a ring structure together with T,
Rd represents a $C_1$ to $C_3$ alkyl group,
d, e, and f each independently represent 0 or 1,
at least one of d, e, and f represents 1,
when each of v and w is 1, at least one of Ra, Rb, and Rc is a methyl group, and none of Ra, Rb, and Rc are linked to each other to form a ring structure together with T,
when at least one of Ra, Rb, and Rc is a methylamino group, none of Ra, Rb, and Rc are linked to each other to form a ring structure together with T, and
when the functional group represented by the formula (1a) is a hydroxy group and T is a carbon atom, v is 1, w is 0, each of d, e, and f is 1, and each of Ra, Rb, and Rc is a hydrogen atom.

**2.** The production method according to claim 1, wherein in the formula (1),

T represents a phosphorus atom or a sulfur atom,
the functional group represented by the formula (1a) is =O, and
each of Ra, Rb, and Rc is a methyl group.

**3.** The production method according to claim 1 or 2, wherein the compound D is dimethyl sulfoxide.

**4.** A method for producing an amino acid, comprising a step of performing an intermolecular reductive amination reaction between compound A selected from the group consisting of a compound having an amino group and a salt thereof and compound B selected from the group consisting of a compound having a carbonyl group and a salt thereof, or an intramolecular reductive amination reaction of compound C selected from the group consisting of a compound having an amino group and a carbonyl group and a salt thereof, in the presence of a polypeptide having catalytic activity for an intermolecular reductive amination reaction between the compound A and the compound B, or an intramolecular

reductive amination reaction of the compound C under at least one reaction condition, a reducing agent, and compound D',
wherein the compound D' is at least one compound selected from the group consisting of dimethyl sulfoxide, dimethylsulfone, trimethylphosphine oxide, dimethoxyethane, N,N-dimethylformamide, N,N-dimethylacetamide, tetramethylene sulfoxide, diethyl sulfoxide, methanol, and methylformamide.

**5.** The production method according to claim 4, wherein the compound D' is at least one compound selected from the group consisting of dimethyl sulfoxide, dimethylsulfone and trimethylphosphine oxide.

**6.** The production method according to claim 4 or 5, wherein the compound D' is dimethyl sulfoxide.

**7.** The production method according to any one of claims 1 to 6, wherein the compound A is at least one compound selected from the group consisting of a compound represented by the following formula (2) and a salt thereof:

[Formula 3]

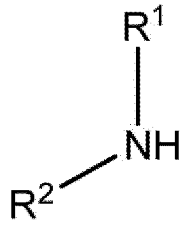

(2)

wherein in the formula (2),
$R^1$ and $R^2$ each independently represent a hydrogen atom, an alkyl group, an alkenyl group, an alkynyl group, a cycloalkyl group, an aryl group, a heterocyclyl group, or a heteroaryl group, these groups are optionally substituted, and at least one of $R^1$ and $R^2$ is a hydrogen atom.

**8.** The production method according to claim 7, wherein in the formula (2), $R^1$ is a hydrogen atom, and $R^2$ is an alkyl group.

**9.** The production method according to claim 7 or 8, wherein in the formula (2), $R^1$ is a hydrogen atom and $R^2$ is a methyl group or an ethyl group.

**10.** The production method according to any one of claims 1 to 9, wherein the compound B is at least one compound selected from the group consisting of a compound represented by the following formula (3) and a salt thereof:

[Formula 4]

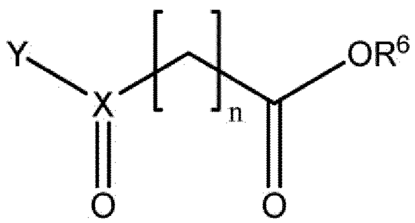

(3)

[Formula 5]

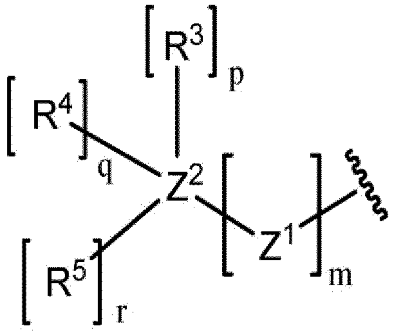

(4)

wherein in the formula (3),

X represents a carbon atom,
Y represents a hydrogen atom, or a group represented by the formula (4),
n represents an integer of 0 or more and 2 or less, and
$R^6$ represents a hydrogen atom, an optionally substituted $C_1$ to $C_6$ alkyl group, an optionally substituted $C_1$ to $C_6$ aryl group, an optionally substituted heteroaryl group having 5 or more and 12 or less ring-constituting atoms, a group containing a nitrogen atom, or a group containing an oxygen atom,
in the formula (4),

[Formula 6]

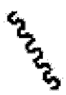

represents a binding point to X,

m represents an integer of 0 or more and 6 or less,
p is 0 or 1,
q is 0 or 1,
r is 0 or 1,
$Z^1$ represents an optionally substituted alkylene group, or an ether bond-containing group having 1 or more and 6 or less carbon atoms, and when m is an integer of 2 or more, a plurality of $Z^1$ are the same or different,
$Z^2$ represents a carbon atom,
$R^3$, $R^4$, and $R^5$ each independently represent a hydrogen atom, an optionally substituted $C_1$ to $C_6$ alkyl group, an optionally substituted $C_5$ to $C_{12}$ aryl group, an optionally substituted heteroaryl group having 5 or more and 12 or less ring-constituting atoms, a group containing a nitrogen atom, or a group containing an oxygen atom,
any two or more of $R^3$, $R^4$, and $R^5$ are optionally linked to each other to form a ring structure together with $Z^2$, the ring structure is optionally a cycloalkyl group, an aryl group, a heterocyclyl group, or a heteroaryl group, and these groups are optionally substituted,
$R^3$, $R^4$, and $R^5$ each optionally form a double bond or a triple bond with $Z^2$, and when any one of $R^3$, $R^4$, and $R^5$ is bonded to $Z^2$ through a double bond or a triple bond, at least one of p, q and r is 0.

**11.** The production method according to claim 10, wherein in the formula (3), n is 0, $R^6$ is a hydrogen atom, Y is a $C_3$ to $C_8$ cycloalkyl group, or a $C_6$ to $C_9$ aralkyl group, and the aralkyl group is optionally substituted by a $C_1$ to $C_3$ alkyl group or halogen.

**12.** The production method according to claim 10 or 11, wherein in the formula (3), n is 0, $R^6$ is a hydrogen atom, and Y is a (2-chlorophenyl)ethyl group, a phenylmethyl group, or a cyclopentyl group.

**13.** The production method according to any one of claims 1 to 12, wherein the step is performed under appropriate reaction conditions.

**14.** A method for producing a peptide compound, comprising the steps of:

(1) producing an amino acid by the production method according to any one of claims 1 to 13; and
(2) linking the amino acid to one or more selected from the group consisting of other amino acids and a peptide to produce a peptide compound.

**15.** A reductive amination reaction accelerator represented by the following formula (1), which accelerates the following reaction (i) or (ii):

(i) an intermolecular reductive amination reaction between compound A selected from the group consisting of a compound having an amino group and a salt thereof and compound B selected from the group consisting of a compound having a carbonyl group and a salt thereof;
(ii) an intramolecular reductive amination reaction of compound C selected from the group consisting of a compound having an amino group and a carbonyl group and a salt thereof:

[Formula 7]

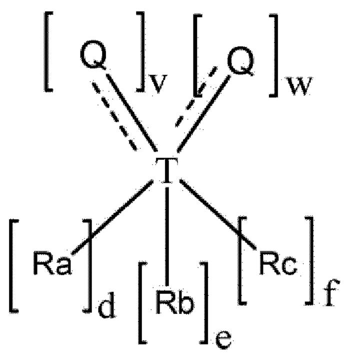

(1)

wherein in the formula (1),

v and w each independently represent 0 or 1,
at least one of v and w represents 1,
T represents a carbon atom, a phosphorus atom, or a sulfur atom,
a functional group represented by the following formula (la):

[Formula 8]

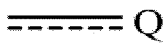

(1 a)

represents =O, -ORd, or a hydroxy group,

when each of v and w is 1, two functional groups represented by a plurality of formulas (1a) are the same or different,
Ra, Rb, and Rc each independently represent a hydrogen atom, a $C_1$ to $C_3$ alkyl group, an alkylamino group, or $-CH_2-ORd$,
at least any two of Ra, Rb, and Rc are optionally linked to each other to form a ring structure together with T,
Rd represents a $C_1$ to $C_3$ alkyl group,
d, e, and f each independently represent 0 or 1,
at least one of d, e, and f represents 1,
when each of v and w is 1, at least one of Ra, Rb, and Rc is a methyl group, and none of Ra, Rb, and Rc are linked to each other to form a ring structure together with T,
when at least one of Ra, Rb, and Rc is a methylamino group, none of Ra, Rb, and Rc are linked to each other to form a ring structure together with T, and

when the functional group represented by the formula (1a) is a hydroxy group and T is a carbon atom, each of d, e, and f is 1, and each of Ra, Rb, and Rc is a hydrogen atom.

Fig.1

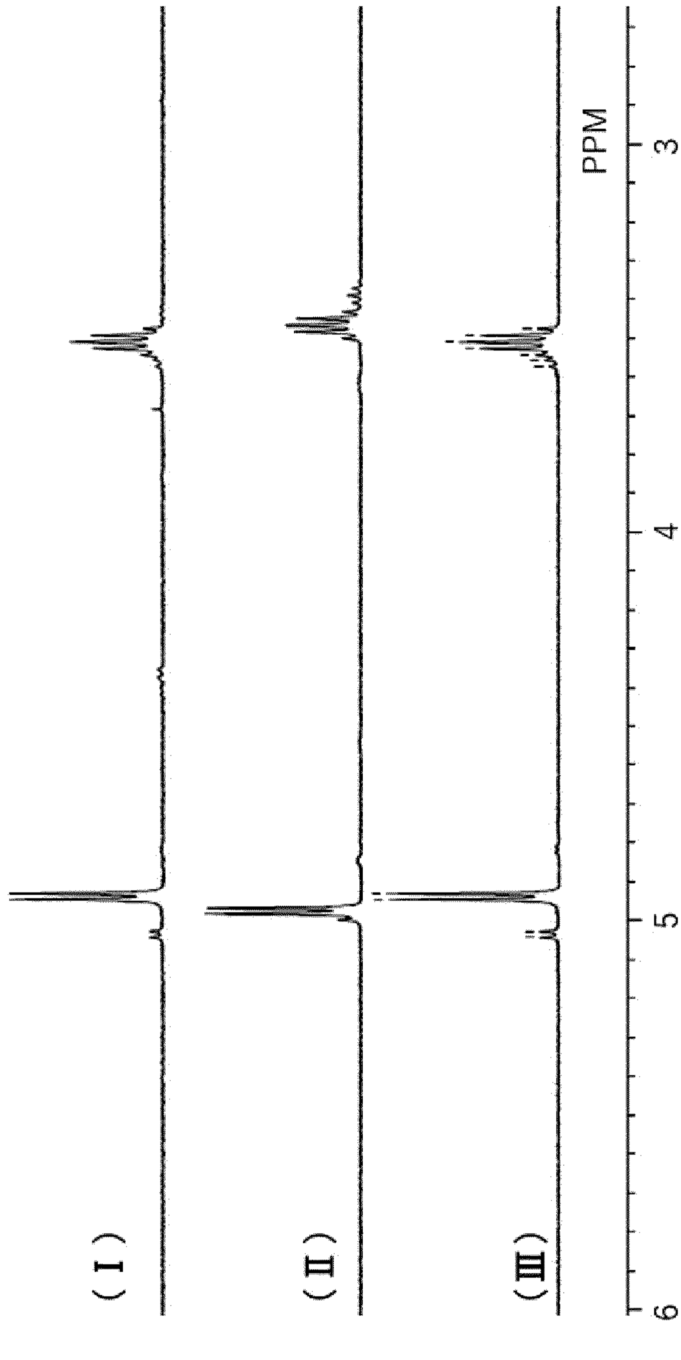

*Fig.2*

(I)

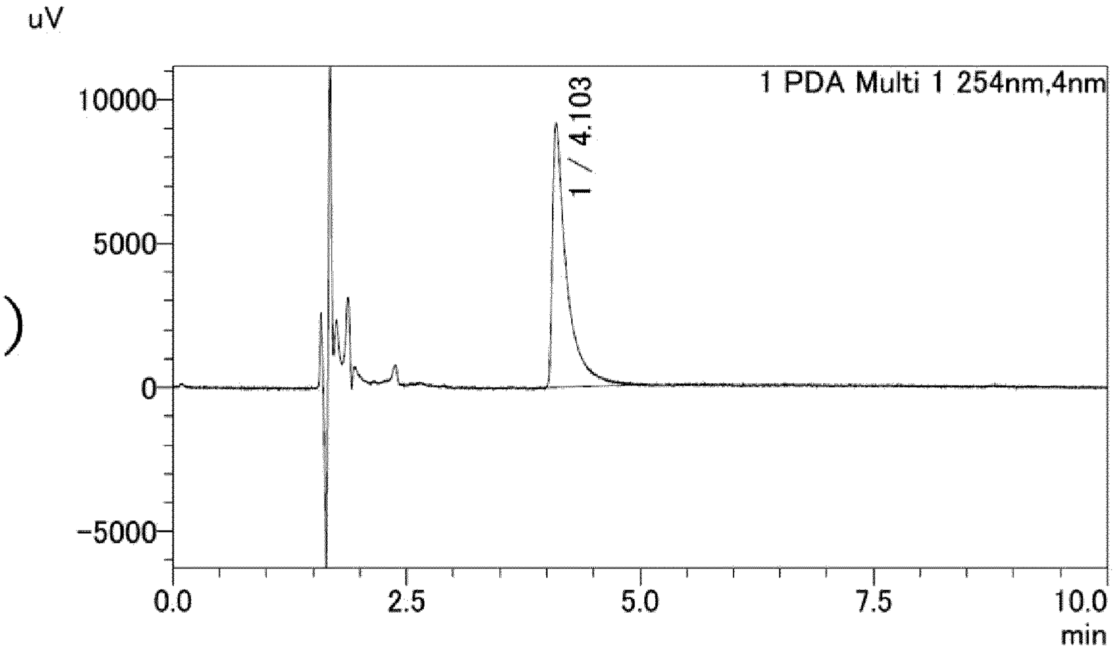
uV
1 PDA Multi 1 254nm,4nm
1 / 4.103
10000
5000
0
-5000
0.0
2.5
5.0
7.5
10.0
min

(V)

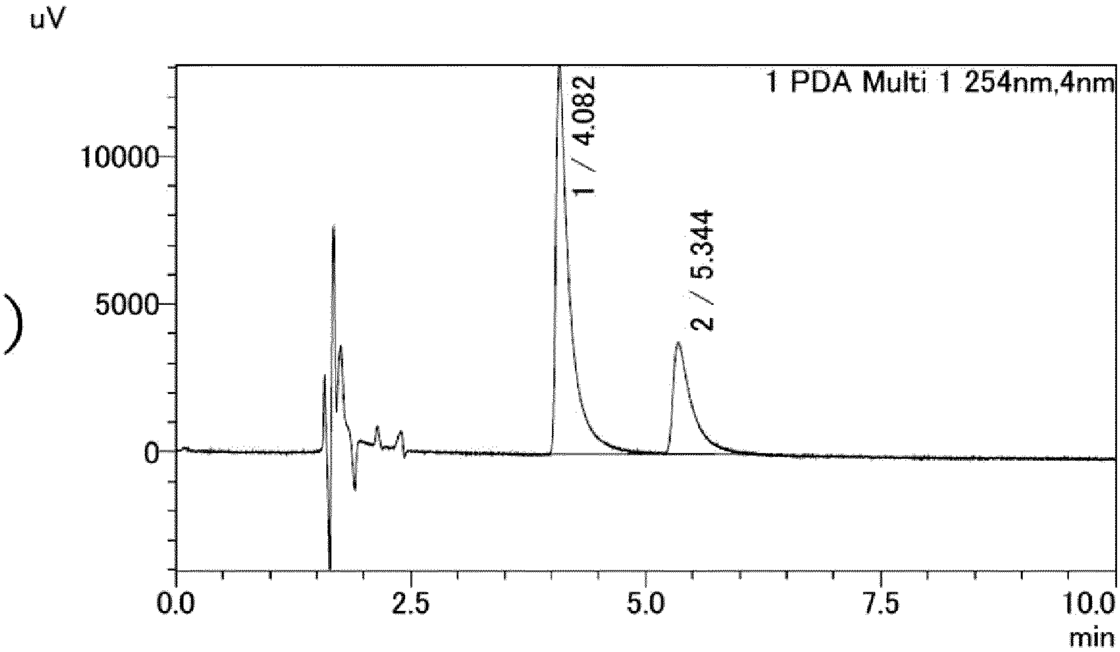
uV
1 PDA Multi 1 254nm,4nm
1 / 4.082
2 / 5.344
10000
5000
0
0.0
2.5
5.0
7.5
10.0
min

## INTERNATIONAL SEARCH REPORT

International application No. **PCT/JP2023/017861**

**A. CLASSIFICATION OF SUBJECT MATTER**

***C12N 15/53***(2006.01)i; ***C07C 227/28***(2006.01)i; ***C07C 229/12***(2006.01)i; ***C07K 1/00***(2006.01)i; ***C12N 9/06***(2006.01)i; ***C12N 15/31***(2006.01)i; ***C12P 13/04***(2006.01)i
FI: C12N15/53; C07C227/28; C07C229/12; C07K1/00; C12N9/06 Z; C12N15/31; C12P13/04 ZNA

According to International Patent Classification (IPC) or to both national classification and IPC

**B. FIELDS SEARCHED**

Minimum documentation searched (classification system followed by classification symbols)

C12N15/53; C07C227/28; C07C229/12; C07K1/00; C12N9/06; C12N15/31; C12P13/04

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/MEDLINE/EMBASE/BIOSIS (STN), JSTPlus/JMEDPlus/JST7580 (JDreamIII), UniProt/GeneSeq, PubMed, Japio-GPG/FX

**C. DOCUMENTS CONSIDERED TO BE RELEVANT**

| Category* | Citation of document, with indication, where appropriate, of the relevant passages | Relevant to claim No. |
|---|---|---|
| X | WO 2003/072770 A1 (MITSUBISHI CHEMICAL CORPORATION) 04 September 2003 (2003-09-04)<br>claims 1-14, p. 20, 4th-5th paragraphs | 1-15 |
| Y | claims 1-14, p. 20, 4th-5th paragraphs | 1-15 |
| Y | MINDT, M. et al. Fermentative Production of N-Alkylated Glycine Derivatives by Recombinant Corynebacterium glutamicum Using a Mutant of Imine Reductase DpkA From Pseudomonas putida. Frontiers in Bioengineering and Biotechnology. 2019, vol. 7, article 232, pp. 1-12<br>abstract, p. 2, right column, 3rd paragraph | 1-15 |
| A | JP 2005-095167 A (MITSUBISHI CHEMICAL CORPORATION) 14 April 2005 (2005-04-14)<br>entire text, all drawings | 1-15 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **20 July 2023** | **01 August 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2023/017861**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      - ☑ in the form of an Annex C/ST.25 text file.
      - ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      - ☐ in the form of an Annex C/ST.25 text file (Rule 13*ter*.1(a)).
      - ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

   The “form of an Annex C/ST.25 text file” is replaced with the “form of ST.26.”

Form PCT/ISA/210 (continuation of first sheet) (January 2015)

**INTERNATIONAL SEARCH REPORT**
**Information on patent family members**

International application No.
**PCT/JP2023/017861**

| Patent document cited in search report | Publication date (day/month/year) | Patent family member(s) | Publication date (day/month/year) |
|---|---|---|---|
| WO 2003/072770 A1 | 04 September 2003 | US 2005/0124040 A1<br>claims 1-28, paragraphs [0201]-[0202]<br>US 2006/0205045 A1<br>EP 1479762 A1 | |
| JP 2005-095167 A | 14 April 2005 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2003319788 A
- JP 2005095167 A
- JP 2012080878 A
- JP 2012080879 A

### Non-patent literature cited in the description

- **KARLIN** ; **ALTSCHUL**. *Proc. Natl. Acad. Sci. USA*, 1993, vol. 90, 5873-7
- **ALTSCHUL et al.** *J. Mol. Biol.*, 1990, vol. 215, 403-10
- **HOPP, T.P. et al.** *BioTechnology*, 1988, vol. 6, 1204-1210